# EUROPEAN PATENT APPLICATION

(11) **EP 2 077 326 A1**
(43) Date of publication of application: **08.07.2009**
(21) Application number: 07828180.5
(22) Date of filing: 04.09.2007
(51) Int. Cl.: C12N 15/09, A61K 31/7088, A61K 48/00, A61P 3/10, A61P 19/08, A61P 35/00, A61P 43/00, C12N 1/15, C12N 1/19, C12N 1/21, C12N 5/10, C12Q 1/68

(54) **NOVEL NUCLEIC ACID**

(30) Priority: 04.09.2006 JP 2006238459
(71) Applicant: Kyowa Hakko Kirin Co., Ltd., Tokyo 100-8185 (JP)
(72) Inventor: YAMADA, Yoji c/o biofrontier Lab., Tokyo 194-8533 (JP); MIYAZAWA, Tatsuya c/o Biofrontier Lab., Tokyo 194-8533 (JP); YOSHIDA, Tetsuo c/o BioFrontier Lab, tOKYO 194-8533 (JP); NAKANO, HaruoC/O bIOfRONTIER lAB, Tokyo 194-8533 (JP); KOSAKA, Kyokoc/o BioFrontier Lab., Tokyo 194-8533 (JP)
(74) Representative: Vossius & Partner
(86) International application number: PCT/JP2007/067187
(87) International publication number: WO 2008/029790

(57) **Abstract**

According to the present invention, it possible to provide a novel nucleic acid, a vector that expresses the nucleic acid, a screening method for a substance that controls the nucleic acid, a method of detecting the expression and a mutation of the nucleic acid, and a diagnostic method or therapeutic method for a disease caused by an abnormality of mesenchymal stem cells, mast cells or the like, or a disease caused by a cell proliferation abnormality; such as cancer.

## Description

### Technical Field

The present invention relates to a novel nucleic acid, a method of expressing or suppressing the nucleic acid, and a diagnostic reagent or therapeutic drug comprising a molecule having the nucleic acid.

### Background Art

A micro-RNA (miRNA) is a small non-coding single-stranded RNA of about 22 nucleotides that is not translated into a protein, and has been confirmed as being present in many types in organisms, including humans (non-patent documents 1 and 2).
A micro-RNA is produced from a gene transcribed to a single or clustered micro-RNA precursor. Specifically, first, a primary-micro-RNA (pri-miRNA), which is a primary transcript, is transcribed from the gene, then, in stepwise processing from the pri-miRNA to a mature type micro-RNA, a precursor-micro-RNA (pre-miRNA) of about 70 nucleotides having a characteristic hairpin structure is produced from the pri-miRNA. Furthermore, the mature type micro-RNA is produced from the pre-miRNA by Dicer-mediated processing (non-patent document 3).

A mature type micro-RNA is thought to be involved in the post-transcriptional control of gene expression by complementarily binding to a mRNA for a target to suppress the translation of the mRNA, or to degrade the mRNA. As of May 2006, in the micro-RNA database miRBase (http://microrna.sanger.ac.uk/), 455 species of micro-RNAs were registered for humans, and 3685 species for all organisms. Of the micro-RNAs expressed in mammals, including humans, only some have their physiological functions elucidated to date, including miR-181, which is involved in hematopoietic lineage differentiation (non-patent document 4), miR-375, which is involved in insulin secretion (non-patent document 5), and the like; many have their bioactivities unclarified. However, studies using nematodes or Drosohila have shown that micro-RNAs play various important roles in the development and differentiation in organisms, and a report of the relation to human diseases has been presented suggesting a profound relation to cancers (non-patent document 6).

For identification of micro-RNAs, there are a method wherein a small RNA is cloned from a cell, a method wherein bioinformatics is applied to genome sequence information, and the like. Registration of any micro-RNA in the miRBase requires both information on the expression and information on the biosynthesis and structure; a structural prediction from genome sequence information only does not suffice approval as a micro-RNA (non-patent document 7).

Mesenchymal stem cells are present in mammalian bone marrow, fat tissue, umbilical blood and the like, and are known as multipotent stem cells that differentiate into adipocytes, chondrocytes, osteocytes and the like. Mesenchymal stem cells, because of the multipotency thereof, are attracting attention as graft sources for regenerative medicine for many tissues, including bones, cartilage, tendons, muscles, fat, and periodontal tissue (non-patent document 8).
Mesenchymal stem cells can be differentiated into particular cells in vitro by the addition of a drug, a cytokine and the like; for example, differentiation into adipocytes can be induced by allowing 1-methyl-3-isobutylxanthine, dexamethasone, insulin and indomethacin to act, and differentiation into osteoblasts can be induced by allowing dexamethasone, β-glycerol phosphate, and ascorbic acid to act (non-patent document 9). However, details of the molecular mechanisms in these differentiation processes are unknown. From the results of knockout mice and gene expression analyses in the differentiation stage, it is known that differentiation into adipocytes is mediated by the PPARγ and C/EBP families, and that during osteoblast differentiation, the expression of genes such as Cbfa1/Runx2 and Osterix is involved (non-patent document 10); however, the mechanisms of differentiation from mesenchymal stem cells cannot be explained solely on the basis of these genes, and artificial control of the differentiation and proliferation has not been realized. Also, no micro-RNA is known to act on the differentiation and proliferation of mesenchymal stem cells.

Mast cells are known to be activated by various stimuli to undergo degranulation and release or produce many inflammatory mediators (non-patent documents 11 to 13). For example, it is known that when an antigen is recognized by a mast cell, histamine and tryptase are quickly released upon degranulation, and chemical mediators such as prostaglandin D2 (PGD2), leukotriene (LT), and platelet activation factor (PAF), various chemokines such as macrophage inflammatory protein (MIP)-1α, and various cytokines such as granulocyte macrophage colony stimulation factor (GM-CSF) are newly synthesized and released. Regarding major basic proteins, which are cytotoxic proteins that have been thought to be produced by eosinophils, it has recently been shown that in the case of humans, they are produced in large amounts by mast cells (non-patent document 14).

Hence, mast cells are thought to play major roles in the pathogenesis of various allergic diseases; therefore, it is thought that by controlling a function of mast cells, treatment of allergic diseases is possible.
However, it is known that rodent mast cells and human mast cells have different reactivities to drugs (non-patent document 12). Specifically, sodium cromoglicate, which is used as a suppressant of inflammatory mediator release, remarkably suppresses the IgE-dependent release of inflammatory mediators in rat abdominal mast cells, but the action thereof on human mast cells is not potent (non-patent document 15). Azelastine hydrochloride, at high concentrations, suppresses the release of histamine, PGD2, and LT and production of GM-CSF and MIP-1α, from human mast cells in culture, but none of these activities are potent. Suplatast tosilate, which is used as an anti-cytokine drug, exhibited inflammatory mediator release suppressive action on rat mast cells, but lacks action on human mast cells (non-patent document 15).

As a result of a comparison of genes expressed in human mast cells and mouse mast cells, it is known that the genes expressed with various stimuli do not always agree (non-patent document 16).
Regarding micro-RNAs, a micro-RNA expressed in mouse bone marrow derived mast cells has been reported (non-patent document 17), but no relationship is known between a micro-RNA and a function of mast cells. No report is available on a micro-RNA expressed in human mast cells; taking into account the above-described interspecific differences between humans and mice, it is difficult to predict information on the expression of micro-RNAs in human mast cells on the basis of information on the expression of micro-RNAs in mouse mast cells.

Because micro-RNAs are involved in the control of the expression of a wide variety of genes, abnormalities of micro-RNAs are supposed to be involved in various human diseases. Particularly in cancers, research has been advanced; it has been reported that in many cancers, the expression of micro-RNAs differs from that in normal tissues, that classification of cancers is enabled by expression profile analyses of micro-RNAs, and the like (non-patent document 18). It is also known that about half of the human micro-RNAs that have been found so far are present in chromosome aberrations or fragile portions of chromosomes known in human cancers (non-patent document 19). Examples of relationships between cancers and micro-RNAs that have been reported so far include the finding that the miR-15a/miR-16 cluster is contained in chromosome 13q14, which is deleted in B cell chronic lymphatic leukemia (B-CLL), the deletion being supposed to be a cause of B-CLL (non-patent document 20), the finding that in lung cancer, the expression of Let-7, which is a micro-RNA, is decreased, one of the targets thereof being Ras, which is known as a carcinogenic gene (non-patent documents 21 and 22), and the like. Many micro-RNAs have their expression decreased in cancer cells; conversely, however, there are some micro-RNAs with gene amplification or overexpression in cancers. For example, in regions where gene amplification is seen in malignant lymphoma, a cluster consisting of six species of micro-RNAs (miR-17-92) is present; it has been reported that when this miRNA cluster gene is forcibly expressed in a mouse model of human B cell lymphoma, the onset of lymphoma is promoted (non-patent document 23). It has also been shown that a gene called BIC, which has been regarded as a cancer gene candidate that does not encode a protein overexpressed in Hodgkin lymphoma, encodes miR-155 (non-patent document 24).

As stated above, relationships between cancers and micro-RNAs have recently been reported in many cases, but most of them show expression abnormalities in cancer cells; there are only a few studies showing a function of a micro-RNA, including a report that the proliferation of a cancer cell line was inhibited by forcibly expressing Let-7 in a lung cancer cell line (non-patent document 25) and the like. Currently, no report is available that cancer growth was suppressed in an animal model by administering a micro-RNA or a precursor thereof, or an antisense oligo thereof, from outside the body to increase or decrease the expression of the micro-RNA.
non-patent document 1: Science, 294, 853-858 (2001)
non-patent document 2: Cell, 113, 673-676 (2003)
non-patent document 3: Nature Reviews Genetics, 5, 522-531 (200 4)
non-patent document 4: Science, 303, 83-86 (2004)
non-patent document 5: Nature, 432, 226-230 (2004)
non-patent document 6: Nature.Reviews Cancer, 6, 259-269 (2006)
non-patent document 7: RNA, 9, 277-279 (2003)
non-patent document 8: Idenshi Igaku, vol. 4, p. 58-61 (2000)
non-patent document 9: Science, 284, 143-147 (1999)
non-patent document 10: Jikken Igaku, vo. 20, p.2459-2464 (2002)
non-patent document 11: Himan Saibo no Rinsho, ed. Motohito Kuro sawa, Sentan Igaku-sha Ltd., p 142 (2001)
non-patent document 12: Himan Saibo no Rinsho, ed. Motohito Kuro sawa, Sentan Igaku-sha Ltd., p 559 (2001)
non-patent document 13: Crit. Rev. Immunol., 22, 115-140 (2002)]
non-patent document 14: Blood, 98, 1127-1134 (2001)
non-patent document 15: Clin. Exp. Allergy, 28, 1228-1236 (1998)
non-patent document 16: Blood, 100, 3861-3868 (2002)
non-patent document 17: Genome Biology, 6, R71 (2005)
non-patent document 18: Nature, 435, 839-843 (2005)
non-patent document 19: Proc.Natl.Acad.Sci.USA, 101, 2999-3004 (2004)
non-patent document 20: Proc.Natl.Acad.Sci.USA, 99, 15524-15529 (2002)
non-patent document 21: Cancer Research, 64, 3753-3756, (2004)
non-patent document 22: Cell, 120, 635-647, (2005)
non-patent document 23: Nature, 435, 823-833 (2005)
non-patent document 24: Proc.Natl.Acad.Sci.USA, 102, 3627-3632 (2005)
non-patent document 25: Cancer Research, 64, 3753-3756, (2004)

### Disclosure of the Invention

### Problems to Be Solved by the Invention

It is expected that by identifying micro-RNAs expressed in various human organs, and analyzing the functions thereof to elucidate their relations to diseases, new therapeutic drugs and diagnostic reagents will be developed.
Finding a micro-RNA that works in mesenchymal stem cells is expected to lead to the functional elucidation of differentiation and proliferation in mesenchymal stem cells, and to lead to the development of a method of controlling the differentiation from mesenchymal stem cells to particular cells and a new therapy based on differentiation control.

Finding a micro-RNA that works in mast cells is expected to lead to the functional elucidation of differentiation, degranulation, inflammatory mediator production, cytokine production, chemokine production and the like in mast cells, and to contribute to the development of a method of control thereof and a new therapy for allergic diseases and the like.
Furthermore, finding a micro-RNA that causes cancer cell proliferation or suppression is expected not only to help to understand the mechanisms of carcinogenesis, but also to lead to the development of diagnostic reagent and therapeutic drugs for human cancers, and new diagnostic methods and therapies for cancers based thereon. Furthermore, regarding diseases other than cancers, the same is expected to contribute to the development of diagnostic reagents and therapeutic drugs for diseases caused by abnormal proliferation of cells, tissue hyperplasia and the like, such as arteriosclerosis, rheumatoid arthritis, prostatic hyperplasia, blood vessel restenosis after percutaneous transvascular coronary angioplasty, fibroid lung, glomerulonephritis, and autoimmune diseases, and diagnostic methods and therapies based thereon.
It is an object of the present invention to acquire a set of micro-RNAs, and to provide nucleic acids that are useful in controlling the differentiation and proliferation of mesenchymal stem cells, controlling mast cell differentiation, degranulation, inflammatory mediator production, cytokine production, chemokine production and the like, treating allergic diseases, controlling cancer cell differentiation and proliferation, and diagnosing and treating diseases such as cancers, and methods of utilizing the same.

### Means of Solving the Problems

The present invention relates to (1) to (40) below.
(1) A nucleic acid consisting of a nucleotide sequence having an identity of 90% or more with the nucleotide sequence of any one of SEQ ID NO:1 to 29 and 78 to 80.
(2) A nucleic acid that hybridizes under stringent conditions with a complementary strand of nucleic acid consisting of the nucleotide sequence of any one of SEQ ID NO:1 to 29 and 78 to 80.
(3) A micro-RNA consisting of the nucleotide sequence of any one of SEQ ID NO:1 to 29 and 78 to 80.
(4) A micro-RNA precursor comprising the nucleic acid described in any one of (1) to (3).
(5) The micro-RNA precursor described in (4), consisting of a nucleotide sequence having an identity of 80% or more with the nucleotide sequence of any one of SEQ ID NO:30 to 73 and 81 to 83.
(6) The micro-RNA precursor described in (4) or (5), which hybridizes under stringent conditions with a complementary strand of nucleic acid consisting of the nucleotide sequence of any one of SEQ ID NO:30 to 73 and 81 to 83.
(7) A micro-RNA precursor consisting of the nucleotide sequence of any one of SEQ ID NO:30 to 73 and 81 to 83.
(8) A nucleic acid consisting of a nucleotide sequence complementary to the nucleic acid, micro-RNA or micro-RNA precursor described in any one of (1) to (7).
(9) A double-stranded nucleic acid consisting of the nucleic acid, micro-RNA or micro-RNA precursor described in any one of (1) to (7) and a nucleic acid consisting of a nucleotide sequence complementary to the nucleotide sequence of the nucleic acid, micro-RNA or micro-RNA precursor.
(10) A method of detecting the expression of a micro-RNA, comprising using the nucleic acid, micro-RNA or micro-RNA precursor described in any one of (1) to (9).
(11) A method of detecting a mutation of a micro-RNA, comprising using the nucleic acid, micro-RNA or micro-RNA precursor described in any one of (1) to (9).
(12) A method of separating a cell, comprising using the nucleic acid, micro-RNA or micro-RNA precursor described in any one of (1) to (9).
(13) A vector that expresses the nucleic acid, micro-RNA or micro-RNA precursor described in any one of (1) to (9).
(14) A method of suppressing the expression of a gene having a target nucleotide sequence of the nucleic acid, micro-RNA or micro-RNA precursor described in any one of (1) to (9), comprising using the nucleic acid, micro-RNA or micro-RNA precursor.
(15) A method of screening for a substance that promotes or suppresses the expression or a function of a micro-RNA or micro-RNA precursor, comprising using the nucleic acid, micro-RNA or micro-RNA precursor described in any one of (1) to (9).
(16) A diagnostic reagent for a disease caused by an abnormality of mesenchymal stem cell proliferation and/or differentiation, comprising the nucleic acid, micro-RNA or micro-RNA precursor described in any one of (1) to (9) as an active ingredient.
(17) A therapeutic drug for a disease caused by an abnormality of mesenchymal stem cell proliferation and/or differentiation, comprising the nucleic acid, micro-RNA or micro-RNA precursor described in any one of (1) to (9) as an active ingredient.
(18) A cell incorporating the nucleic acid, micro-RNA or micro-RNA precursor described in any one of (1) to (9).
(19) A cell incorporating the vector described in (13).
(20) A diagnostic reagent or therapeutic drug for a disease caused by a mast cell abnormality, comprising the nucleic acid, micro-RNA or micro-RNA precursor described in any one of (1) to (4) as an active ingredient, wherein the SEQ ID NO: is any one of 1, 17, 18, 19, and 22, as an active ingredient.
(21) An agent for promoting mast cell degranulation, comprising the nucleic acid, micro-RNA or micro-RNA precursor described in any one of (1) to (4) as an active ingredient, wherein the SEQ ID NO: is any one of 1, 17, 18, 19, and 22, as an active ingredient.
(22) A diagnostic reagent or therapeutic drug for a disease caused by a mast cell abnormality, comprising the micro-RNA precursor described in any one of (5) to (7) as an active ingredient, wherein the SEQ ID NO: is any one of 30, 58, 59, 60, 61, and 65, as an active ingredient.
(23) An agent for promoting mast cell degranulation, comprising the micro-RNA precursor described in any one of (5) to (7) as an active ingredient, wherein the SEQ ID NO: is any one of 30, 58, 59, 60, 61, and 65, as the active ingredient.
(24) A diagnostic reagent or therapeutic drug for a disease caused by a mast cell abnormality, comprising a nucleic acid consisting of a nucleotide sequence complementary to the nucleic acid, micro-RNA or micro-RNA precursor described in any one of (1) to (4), wherein the SEQ ID NO: is any one of 1, 17, 18, 19, and 22, or to the micro-RNA precursor described in any one of (5) to (7), wherein the SEQ ID NO: is any one of 30, 58, 59, 60, 61, and 65, as an active ingredient.
(25) An agent for suppressing mast cell degranulation, comprising a nucleic acid consisting of a nucleotide sequence complementary to the nucleic acid, micro-RNA or micro-RNA precursor described in any one of (1) to (4), wherein the SEQ ID NO: is any one of 1, 17, 18, 19, and 22, or to the micro-RNA precursor described in any one of (5) to (7), wherein the SEQ ID NO: is any one of 30, 58, 59, 60, 61, and 65, as an active ingredient.
(26) A diagnostic reagent or therapeutic drug for a disease caused by a mast cell abnormality, comprising a double-stranded nucleic acid consisting of the nucleic acid, micro-RNA or micro-RNA precursor described in any one of (1) to (4), wherein the SEQ ID NO: is any one of 1, 17, 18, 19, and 22, or of the micro-RNA precursor described in any one of (5) to (7), wherein the SEQ ID NO: is any one of 30, 58, 59, 60, 61, and 65, and a nucleic acid consisting of a nucleotide sequence complementary to the nucleotide sequence of the nucleic acid, micro-RNA or micro-RNA precursor, as an active ingredient.
(27) An agent for promoting or suppressing mast cell degranulation, comprising a double-stranded nucleic acid consisting of the nucleic acid, micro-RNA or micro-RNA precursor described in any one of (1) to (4), wherein the SEQ ID NO: is any one of 1, 17, 18, 19, and 22, or of the micro-RNA precursor described in any one of (5) to (7), wherein the SEQ ID NO: is any one of 30, 58, 59, 60, 61, and 65, and a nucleic acid consisting of a nucleotide sequence complementary to the nucleotide sequence of the nucleic acid, micro-RNA or micro-RNA precursor, as an active ingredient.
(28) A diagnostic reagent or therapeutic drug for a disease caused by a cell proliferation abnormality, comprising the nucleic acid, micro-RNA or micro-RNA precursor described in any one of (1) to (4) wherein the SEQ ID NO: is any one of 1, 4, 6, 8, 9, 10, 17, 18, 19, 20, and 22, as an active ingredient.
(29) A diagnostic reagent or therapeutic drug for a disease caused by a cell proliferation abnormality, comprising the micro-RNA precursor described in any one of (5) to (7) wherein the SEQ ID NO: is any one of 30, 34, 42, 43, 44, 46, 47, 48, 58, 59, 60, 61, 62, and 65, as an active ingredient.
(30) A diagnostic reagent or therapeutic drug for a disease caused by a cell proliferation abnormality, comprising a nucleic acid consisting of a nucleotide sequence complementary to the nucleic acid, micro-RNA or micro-RNA precursor described in any one of (1) to (4), wherein the SEQ ID NO: is any one of 1, 4, 6, 8, 9, 10, 17, 18, 19, 20, and 22, or to the micro-RNA precursor described in any one of (5) to (7), wherein the SEQ ID NO: is any one of 30, 34, 42, 43, 44, 46, 47, 48, 58, 59, 60, 61, 62, and 65, as an active ingredient.
(31) A diagnostic reagent or therapeutic drug for a disease caused by a cell proliferation abnormality, comprising a double-stranded nucleic acid consisting of the nucleic acid, micro-RNA or micro-RNA precursor described in any one of (1) to (4), wherein the SEQ ID NO: is any one of 1, 4, 6, 8, 9, 10, 17, 18, 19, 20, and 22, or of the micro-RNA precursor described in any one of (5) to (7), wherein the SEQ ID NO: is any one of 30, 34, 42, 43, 44, 46, 47, 48, 58, 59, 60, 61, 62, and 65, and a nucleic acid consisting of a nucleotide sequence complementary to the nucleotide sequence of the nucleic acid, micro-RNA or micro-RNA precursor, as an active ingredient.
(32) The diagnostic reagent or therapeutic drug described in any one of (28) to (31), wherein the disease is a disease selected from the group consisting of cancers, arteriosclerosis, chronic rheumatoid arthritis, prostatic hyperplasia, blood vessel restenosis after percutaneous transvascular coronary angioplasty, fibroid lung, glomerulonephritis and autoimmune diseases.
(33) An agent for suppressing cell proliferation, comprising the nucleic acid, micro-RNA or micro-RNA precursor described in any one of (1) to (4) wherein the SEQ ID NO: is any one of 1, 4, 6, 8, 9, 10, 17, 19, 20, and 22, as an active ingredient.
(34) An agent for suppressing cell proliferation, comprising the micro-RNA precursor described in any one of (5) to (7) wherein the SEQ ID NO: is any one of 30, 34, 42, 43, 44, 46, 47, 48, 58, 59, 61, 62, and 65, as an active ingredient.
(35) An agent for promoting cell proliferation, comprising a nucleic acid consisting of a nucleotide sequence complementary to the nucleic acid, micro-RNA or micro-RNA precursor described in any one of (1) to (4), wherein the SEQ ID NO: is any one of 1, 4, 6, 8, 9, 10, 17, 19, 20, and 22, or to the micro-RNA precursor described in any one of (5) to (7), wherein the SEQ ID NO: is any one of 30, 34, 42, 43, 44, 46, 47, 48, 58, 59, 61, 62, and 65, as an active ingredient.
(36) An agent for suppressing or promoting cell proliferation, comprising a double-stranded nucleic acid consisting of the nucleic acid, micro-RNA or micro-RNA precursor described in any one of (1) to (4), wherein the SEQ ID NO: is any one of 1, 4, 6, 8, 9, 10, 17, 19, 20, and 22, or of the micro-RNA precursor described in any one of (5) to (7), wherein the SEQ ID NO: is any one of 30, 34, 42, 43, 44, 46, 47, 48, 58, 59, 61, 62, and 65, and a nucleic acid consisting of a nucleotide sequence complementary to the nucleotide sequence of the nucleic acid, micro-RNA or micro-RNA precursor, as an active ingredient.
(37) An agent for promoting cell proliferation, comprising the nucleic acid, micro-RNA or micro-RNA precursor described in any one of (1) to (4) wherein the SEQ ID NO: is 18, as an active ingredient.
(38) An agent for promoting cell proliferation, comprising the micro-RNA precursor described in any one of (5) to (7) wherein the SEQ ID NO: is 60, as an active ingredient.
(39) An agent for suppressing cell proliferation, comprising a nucleic acid consisting of a nucleotide sequence complementary to the nucleic acid, micro-RNA or micro-RNA precursor described in any one of (1) to (4), wherein the SEQ ID NO: is 18, or to the micro-RNA precursor described in any one of (5) to (7), wherein the SEQ ID NO: is 60, as an active ingredient.
(40) An agent for promoting or suppressing cell proliferation, comprising a double-stranded nucleic acid consisting of the nucleic acid, micro-RNA or micro-RNA precursor described in any one of (1) to (4), wherein the SEQ ID NO: is 18, or of the micro-RNA precursor described in any one of (5) to (7), wherein the SEQ ID NO: is 60 and a nucleic acid consisting of a nucleotide sequence complementary to the nucleotide sequence of the nucleic acid, micro-RNA or micro-RNA precursor, as an active ingredient.

### Effect of the Invention

According to the present invention, it is possible to provide a novel nucleic acid, a vector that expresses the nucleic acid, a screening method for a substance that controls the nucleic acid, a method of detecting the expression and a mutation of the nucleic acid, and a diagnostic method or therapeutic method for a disease caused by an abnormality of mesenchymal stem cells, mast cells and the like, and diseases caused by a cell proliferation abnormality, such as cancers, can be provided.

### Brief Description of the Drawings

FIG. 1 shows the secondary structure of the nucleotide sequence of KHK_miR_001 of SEQ ID NO:30.
FIG. 2 is a graph showing the relative expression level of KHK_miR_007 in hMSCs and HeLa cells. The axis of ordinates indicates relative expression levels, taking the expression level of KHK_miR_007 in hMSCs as 1. On the axis of abscissas, 1 indicates hMSCs, and 2 indicates Hela cells.

### Best Mode for Carrying out the Invention

In the present invention, a micro-RNA is a single-stranded RNA consisting of a length of 15 to 26 nucleotides present in a cell, that has a sequence wherein the surrounding genome sequence, including the sequence, is capable of forming a hairpin structure, and that is capable of being cleaved out from either one strand of the hairpin. A micro-RNA complementarily binds to an mRNA to be a target thereof to degradate or suppress the translation of the mRNA, and to make post-transcriptional control of gene expression.

The present invention also relates to a micro-RNA precursor. A micro-RNA precursor of the present invention is a nucleic acid about 50 to about 200 nucleotides long, more preferably about 70 to about 100 nucleotides long, including the above-described nucleic acid of the present invention, and is capable of forming a hairpin structure. A micro-RNA precursor undergoes processing by a protein called Dicer to produce a micro-RNA.
As a nucleic acid of the present invention, a nucleic acid consisting of a nucleotide sequence having an identify of 90% or more to the nucleotide sequence of any one of SEQ ID NO:1 to 29 and 78 to 80, preferably a nucleic acid consisting of a nucleotide sequence having an identify of 95% or more, can be mentioned. Also, a nucleic acid that hybridizes under stringent conditions with a nucleic acid consisting of the nucleotide sequence of any one of SEQ ID NO:1 to 29 and 78 to 80 can be mentioned. Specifically, a micro-RNA consisting of the nucleotide sequence of any one of SEQ ID NO:1 to 29 and 78 to 80 can be mentioned.

As a nucleic acid of the present invention, a nucleic acid consisting of a nucleotide sequence complementary to the nucleic acid, micro-RNA, or micro-RNA precursor described below can be mentioned. Specifically, a nucleic acid consisting of the nucleotide sequence of any one of SEQ ID NO:1 to 29 and 78 to 80, a nucleic acid consisting of a nucleotide sequence having an identify of 90% or more, preferably 95% or more, with the nucleotide sequence of any one of SEQ ID NO:1 to 29 and 78 to 80, a nucleic acid that hybridizes under stringent conditions with a nucleic acid consisting of the nucleotide sequence of any one of SEQ ID NO:1 to 29 and 78 to 80, a micro-RNA comprising these nucleic acids, a micro-RNA precursor consisting of a nucleotide sequence having an identity of 80% or more, preferably 90% or more, and more preferably 95% or more, with the nucleotide sequence of any one of SEQ ID NO:30 to 73 and 81 to 83, a micro-RNA precursor that hybridizes under stringent conditions with a nucleic acid consisting of the nucleotide sequence of any one of SEQ ID NO:30 to 73 and 81 to 83, and a nucleic acid consisting of a nucleotide sequence complementary to a micro-RNA precursor consisting of the nucleotide sequence of any one of SEQ ID NO:30 to 73 and 81 to 83 can be mentioned.

As the nucleic acids of the present invention, a double-stranded nucleic acid consisting of the nucleic acid, micro-RNA, or micro-RNA precursor described below and a nucleic acid consisting of a nucleotide sequence complementary to the nucleotide sequence of the nucleic acid, micro-RNA or micro-RNA precursor can be mentioned. Specifically, a nucleic acid consisting of the nucleotide sequence of any one of SEQ ID NO:1 to 29 and 78 to 80, a nucleic acid consisting of a nucleotide sequence having an identity of 90% or more, preferably 95% or more, with the nucleotide sequence, a nucleic acid that hybridizes under stringent conditions with a nucleic acid consisting of the nucleotide sequence of any one of SEQ ID NO:1 to 29 and 78 to 80, a micro-RNA comprising these nucleic acids, a micro-RNA precursor consisting of a nucleotide sequence having an identity of 80% or more, preferably 90% or more, and more preferably 95% or more, with the nucleotide sequence of any one of SEQ ID NO:30 to 73 and 81 to 83, a micro-RNA precursor that hybridizes under stringent conditions with a nucleic acid consisting of the nucleotide sequence of any one of SEQ ID NO:30 to 73 and 81 to 83, and a double-stranded nucleic acid consisting of a micro-RNA precursor consisting of the.nucleotide sequence of any one of SEQ ID NO:30 to 73 and 81 to 83 and a nucleic acid consisting of a nucleotide sequence complementary to the nucleotide sequence of the nucleic acid, micro-RNA or micro-RNA precursor can be mentioned.

As a micro-RNA precursor of the present invention, a micro-RNA precursor consisting of a nucleotide sequence having an identity of 80% or more to the nucleotide sequence of any one of SEQ ID NO:30 to 73 and 81 to 83, preferably a micro-RNA precursor consisting of a nucleotide sequence having an identify of 90% or more, more preferably 95% or more, can be mentioned. A micro-RNA precursor that hybridizes under stringent conditions with a nucleic acid consisting of the nucleotide sequence of any one of SEQ ID NO:30 to 73 and 81 to 83 can also be mentioned. Specifically, a micro-RNA precursor consisting of the nucleotide sequence of any one of SEQ ID NO:30 to 73 and 81 to 83 can be mentioned.

As the micro-RNA precursors of the present invention, a micro-RNA precursor comprising a nucleic acid consisting of a nucleotide sequence having an identity of 90% or more, preferably 95% or more, with the nucleotide sequence of any one of SEQ ID NO:1 to 29 and 78 to 80, a micro-RNA precursor comprising a nucleic acid that hybridizes under stringent conditions with a nucleic acid consisting of the nucleotide sequence of any one of SEQ ID NO:1 to 29 and 78 to 80, and a micro-RNA precursor comprising a micro-RNA consisting of the nucleotide sequence of any one of SEQ ID NO:1 to 29 and 78 to 80 can be mentioned.

In the present invention, a nucleic acid, micro-RNA or micro-RNA precursor that hybridizes under stringent conditions is, for example, a nucleic acid, micro-RNA or micro-RNA precursor that can be identified by adding a probe RNA labeled γ-³²P-ATP to a Hybridization buffer consisting of 7.5 mL of 20xSSC, 0.6 mL of 1 M Na₂HPO₄ (pH 7.2), 21 mL of 10% SDS, 0.6 mL of 50xDenhardt's solution, and 0.3 mL of 10 mg/mL sonicated salmon sperm DNA, wherein the probe is a nucleic acid having the nucleotide sequence of any one of SEQ ID NO:1 to 73 and 78 to 83 or a partial fragment thereof, carrying out a reaction at 50°C overnight, thereafter washing the membrane with 5xSSC/5% SDS liquid at 50°C for 10 minutes, and further washing the same with 1xSSC/1% SDS solution at 50°C for 10 minutes, thereafter taking out the membrane, and exposing it to an X-ray film.

In the present invention, the nucleic acid may be any molecule, as far as it is a molecule resulting from polymerization of a nucleotide or a molecule functionally equivalent to the nucleotide; for example, an RNA, which is a ribonucleotide polymer, a DNA, which is a deoxyribonucleotide polymer, a mixed polymer of RNA and DNA, and a nucleotide polymer, including a nucleotide analogue, can be mentioned; furthermore, the nucleic acid may be a nucleotide polymer, including a nucleic acid derivative, and may be a single-stranded nucleic acid or a double-stranded nucleic acid.

In the present invention, the nucleotide analogue may be any molecule, as far as it is a molecule prepared by modifying a ribonucleotide, a deoxyribonucleotide, an RNA or a DNA in order to improve the nuclease resistance thereof, to stabilize the same, to increase the affinity thereof for a complementary chain nucleic acid, to increase the cell permeability thereof, or to visualize the same, compared with the RNA or DNA; the analogue may be a naturally occurring molecule or a non-natural molecule; for example, a nucleotide analogue modified at the sugar moiety thereof, a nucleotide analogue modified at phosphoric acid diester bond and the like can be mentioned.

The nucleotide analogue modified at the sugar moiety thereof may be any one, as far as an optionally chosen chemical structural substance has been added to, or substituted for, a portion or all of the chemical structure of the sugar of the nucleotide; for example, a nucleotide analogue substituted by 2'-O-methylribose, a nucleotide analogue substituted by 2'-O-propylribose, a nucleotide analogue substituted by 2'-methoxyethoxyribose, a nucleotide analogue substituted by 2'-O-methoxyethylribose, a nucleotide analogue substituted by 2'-O-[2-(guanidium)ethyl]ribose, a nucleotide analogue substituted by 2'-O-fluororibose, a bridged nucleic acid (BNA) having two cyclic structures as a result of introduction of a bridging structure into the sugar moiety, more specifically a locked nucleic acid (LNA) wherein the oxygen atom at the 2' position and the carbon atom at the 4' position have been bridged via methylene, and an ethylene bridged nucleic acid) (ENA) [Nucleic Acid Research, 32, e175 (2004)] can be mentioned, and a peptide nucleic acid (PNA) [Acc. Chem. Res., 32, 624 (1999)], an oxypeptide nucleic acid (OPNA) [J. Am. Chem. Soc., 123, 4653 (2001)], and a peptide ribonucleic acid (PRNA) [J. Am. Chem. Soc., 122, 6900 (2000)] and the like can also be mentioned.

The nucleotide analogue modified at phosphoric acid diester bond may be any one, as far as an optionally chosen chemical substance has been added to, or substituted for, a portion or all of the chemical structure of the phosphoric acid diester bond of the nucleotide; for example, a nucleotide analogue substituted by a phosphorothioate bond, a nucleotide analogue substituted by an N3'-P5' phosphoamidate bond, and the like can be mentioned.

In the present invention, the nucleic acid derivative may be any molecule, as far as it is a molecule prepared by adding another chemical substance to the nucleic acid in order to improve the nuclease resistance thereof, to stabilize the same, to increase the affinity thereof for a complementary chain nucleic acid, to increase the cell permeability thereof, or to visualize the same, compared with the nucleic acid; for example, a 5'-polyamine addition derivative, a cholesterol addition derivative, a steroid addition derivative, a bile acid addition derivative, a vitamin addition derivative, a Cy5 addition derivative, a Cy3 addition derivative, a 6-FAM addition derivative, a biotin addition derivative and the like can be mentioned.

As examples of nucleic acid derivatives, specifically as derivatives modified at the sugar moiety, an oligonucleotide derivative substituted by 2'-O-propylribose, an oligonucleotide derivative substituted by 2'-methoxyethoxyribose, an oligonucleotide derivative substituted by 2'-O-methylribose, an oligonucleotide derivative substituted by 2'-O-methoxyethylribose, an oligonucleotide derivative substituted by 2'-O-[2-(guanidium)ethyl]ribose, an oligonucleotide derivative substituted by 2'-O-fluororibose and the like can be mentioned; as derivatives modified at the phosphate group, an oligonucleotide derivative wherein a phosphoric acid diester bond in an oligonucleotide has been converted to a phosphorothioate bond, an oligonucleotide derivatives wherein a phosphoric acid diester bond in an oligonucleotide has been converted to an N3'-P5'phosphoamidate bond and the like can be mentioned [SAIBO KOGAKU, 16, 1463-1473 (1997)] [RNAi Method and Antisense Method, Kodansha (2005)].

In the present invention, a mesenchymal stem cell refers to a cell that is present in mesenchymal tissues such as bone marrow, fat tissue, umbilical blood, endometrium, dermis, skeletal muscles, periosteum, dental follicles, periodontal membranes, dental pulps, and dental germs, and has the potency for differentiating into at least mesenchymal cells such as osteoblasts, adipocytes, and muscle cells.
In the present invention, a mast cell refers to a cell that is activated by various stimuli to undergo degranulation and release or produce many inflammatory mediators.

The method of producing a nucleic acid, micro-RNA and micro-RNA precursor of the present invention is not particularly limited; the same can be produced by a method using a publicly known chemical synthesis, or an enzymatic transcription method and the like. As methods using a publicly known chemical synthesis, the phosphoroamidite method, the phosphorothioate method, the phosphotriester method, the CEM method [Nucleic Acid Research, 35, 3287 (2007)] and the like can be mentioned; for example, the same can be synthesized using the ABI3900 high throughput nucleic acid synthesizer (manufactured by Applied Biosystems). As an enzymatic transcription method, transcription with a plasmid or DNA having a desired nucleotide sequence as the template using a typical phage RNA polymerase, for example, T7, T3, or SP6RNA polymerase, can be mentioned.

The method of detecting the expression of a micro-RNA using a nucleic acid, micro-RNA or micro-RNA precursor of the present invention may be any method, as far as the presence of a micro-RNA or micro-RNA precursor in a sample can be detected; for example, (1) Northern hybridization, (2) dot blot hybridization, (3) in situ hybridization, (4) quantitative PCR, (5) differential hybridization, (6) microarray, (7) ribonuclease protection assay and the like can be mentioned.

The method of detecting a mutation of a micro-RNA using a nucleic acid, micro-RNA or micro-RNA precursor of the present invention may be any method, as far as it enables detection of a mutation of the nucleotide sequence of a micro-RNA or micro-RNA precursor in a sample; for example, a method wherein a hetero-double-strand formed by hybridization of a nucleic acid having a non-mutated nucleotide sequence and a nucleic acid having a mutated nucleotide sequence are detected, or a method wherein a sample-derived nucleotide sequence is directly sequenced to detect the presence or absence of a mutation and the like can be mentioned.

The method of separating cells using a nucleic acid, micro-RNA or micro-RNA precursor of the present invention may be any method, as far as it enables separation of cells that express a nucleic acid, micro-RNA or micro-RNA precursor of the present invention, from a mixture of various cells; for example, a method wherein a probe prepared by fluorescently labeling a nucleic acid having a sequence complementary to the nucleotide sequence of a nucleic acid, micro-RNA or micro-RNA precursor of the present invention is introduced to cells to hybridize the nucleic acid, micro-RNA or micro-RNA precursor of the present invention and the probe, and only the cells that have hybridized with the labeled probe are separated using a flow cytometer with a sorting function and the like can be mentioned.

A vector that expresses a nucleic acid, micro-RNA or micro-RNA precursor of the present invention refers to a vector designed to biosynthesize a nucleic acid, micro-RNA or micro-RNA precursor of the present invention when introduced to, and transcribed in, a cell, and may be any vector, as far as it has a promoter capable of transcribing a nucleic acid, micro-RNA or micro-RNA precursor of the present invention in the cell. Specifically, pcDNA6.2-GW/miR (manufactured by Invitrogen), pSilencer 4.1-CMV (manufactured by Ambion), pSINsi-hH1 DNA (manufactured by Takara Bio Inc.), pSINsi-hU6 DNA (manufactured by Takara Bio Inc.), pENTR/U6 (manufactured by Invitrogen) and the like can be mentioned.

The method of suppressing the expression of a gene having a target nucleotide sequence of a nucleic acid, micro-RNA or micro-RNA precursor of the present invention may be any method, as far as it suppresses the expression of a gene having a target nucleotide sequence of a nucleic acid, micro-RNA or micro-RNA precursor of the present invention by means of the activity to suppress the expression of an mRNA having the target nucleotide sequence using a nucleic acid, micro-RNA or micro-RNA precursor of the present invention. Here, to suppress the expression encompasses a case where the translation of an mRNA is suppressed, and a case where cleavage or degradetion of an mRNA results in a decreased amount of protein translated from the mRNA.

A target nucleotide sequence refers to the nucleotide sequence of a nucleic acid consisting of several nucleotides recognized by a nucleic acid, micro-RNA or micro-RNA precursor of the present invention. The translation of an mRNA having the nucleotide sequence is suppressed by a nucleic acid, micro-RNA or micro-RNA precursor of the present invention. Because an mRNA having a nucleotide sequence complementary to the 2nd to 8th nucleotides on the 5' terminal side of a micro-RNA undergoes suppression of the translation thereof by the micro-RNA [Current Biology, 15, R458-R460 (2005)], a nucleotide sequence complementary to the 2nd to 8th nucleotides on the 5' terminal side of a nucleic acid or micro-RNA of the present invention can be mentioned as a target nucleotide sequence of the nucleic acid or micro-RNA. For example, by providing a target sequence complementary to the 2nd to 8th nucleotides on the 5' terminal side of a micro-RNA, and selecting an mRNA comprising a sequence completely identical to a set of 3' UTR nucleotide sequences of human mRNAs by a method such as character string search, the target nucleotide sequence can be determined. A set of 3' UTR nucleotide sequences of human mRNAs can be prepared using information on genome sequences and gene positions that can be acquired from "UCSC Human Genome Browser Gateway (http://genome.ucsc.edu/cgi-bin/hgGateway)". As specific examples of genes having a target nucleotide sequence of a nucleic acid, micro-RNA or micro-RNA precursor of the present invention, the genes shown in Tables 1 to 14, represented by names (Official Symbols) used in the EntreGene database (http://www.ncbi.nlm.nih.gov/Entrez/) of US National Center for Biotechnology Information (NCBI), can be mentioned. Tables 2 to 14 are continuous from Table 1.

**Table 1**

| SEQ ID NO: | Target sequences |
|---|---|
| 1 | SPSB1;LPPR4;SYPL2;PRRX1;LUZP1;PRKAB2;RGS5;ZBTB41;RGPD2;CTDSP1;A TG16L1;RGPD2;ATG9A;APPL;CACNA2D2;DGKG;MTRR;SLC36A2;SH3PXD2B;T RIM7;BRPF3;PAQR8;ATXN1;AGPAT4;TTYH3;HBP1;KIAA0895;CDK6;DLGAP2; RBPMS;GRHL2;PHF20L1;SLC1A1;AK3;GRIN3A;EPB41L4B;HSPA14;PCGF5;NA NOS1;C10orf67;OVOL1;ARHGEF12;BET1L;CD59;SLC1A2;SOX5;KRAS;ADCY6; FMNL3;B4GALNT1;RFP2;FOXO1A;OXGR1;SLC24A4;KIAA0284;NOVA1;TRIM9; GPR135;RDH11;RAB8B;GOLGA;IL16;LOC440295;KIAA0513;ZNF694;NLGN2;A KAP1;ACE;UBE2G1;CACNB1;LIPG;SIN3B;ZNF175;LOC91661;TRIB3;RIMS4;CY YR1;HEMK2;BRWD1;DERL3;MSN;ABCD1;SPRY3;ARHGAP6;RP11-130N24.1;LR CH2;SPRY3;CLSPN;MAPK1;DDIT3;CCNG2;RHOB;MTSS1;PPM1F;RGS2;EDAR; PLAGL1;HDAC9;E2F5;CCND1;MDM2;ING4;FAIM2;FGF9;NF2;RCC1;DFFA;FRA P1;CASP9;SEPT11;NIPBL;PTP4A1;AHR;CASP2;CARD4;ABL1;CUL4A;ELMO2;G AS2L1;MLLT7;BIRC4;AXUD1;RASSF1;FOXO3A;TACC1;PTH;SESN3;HDAC7A;P CTK2;NF1;MAPT;CDK3;KLK10;PMS2;TCF7L2;DOCK1;ATM;TEGT;BCAT1;CAB LES1;ITGB2;PRKCE;PROK2;SEMA6A;UNC13B;RPS6KB2;SCARB1;ACPP;STK1 7A;PAK1;TXNDC;EXT1;SSSCA1;LTBP2;TNFRSF14;ZWINT;ERBB3; |
| 2 | PRDM16;RP13-15M17.2;TNFRSF1B;PLA2G2F;LDLRAD2;FLJ25476;RGL1;C1orf 95;C1orf69;PLA2G2D;IL28RA;RIMS3;SLC6A9;FLJ32011;LAD1;LMOD1;DUSP19; ING5;HDAC4;ZNF621;BSN;SEMA3F;GRM2;PLXNA1;ADIPOQ;SRGAP3;MAP4;P FKFB4;LRRC15;WHSC1;SH3BP2;HD;AFAP;SERF1A;ERGIC1;CPLX2;UNC5A;N 4BP3;TPPP;FCHSD1;COL11A2;CPNE5;MDGA1;KCNK5;NUP43;TTYH3;FOXK1; AQP1;ADCY1;C7orf19;LEP;VIPR2;CLN8;PDLIM2;LRRC14;ANK1;FLJ46072;C9o rf94;TBC1D13;EGFL7;ANGPTL2;TOR2A;FAM102A;ASB6;ABCA2;RAI17;ZFYVE 27;C10orf77;C10orf54;SPOCK2;C10orf56;SH3PXD2A;BRSK2;ESRRA;SYT12;CAP N5;KCNJ5;SLC25A22;ARHGAP1;SCN4B;SCN2B;BCL9L;DDX11;GPD1;FMNL3; NT5DC3;KCTD10;NCOR2;GOLGA3;BCL2L2;C14orf115;VASH1;KIAA0329;C14orf 153;KIAA0284;ABCD4;CAPN3;SNX22;CYP1A2;C15orf37;GRAMD2;LSM16;GBL; PAQR4;JMJD5;CCL22;HSPC065;PSKH1;KIAA0513;FLJ45121;FLJ32130;CMTM 4;C16orf56;KIAA0523;SARM1;RAB11FIP4;RHBDL3;PSME3;TBKBP1;NXPH3;M YO1C;SCARF1;CAMKK1;MYO1D;TBC1D3C;PERLD1;LOC162427;LIMD2;ACOX 1;NPTX1;RNF165;FZR1;JMJD2B;MAP2K7;F2RL3;UPF1;MARK4;FCAR;ZNF587; C19orf19;BTBD2;GNG7;RAXL1;SH3GL1;ELAVL3;LPHN1;LASS1;SF4;FLJ46230; STK35;HM13;PLCG1;SLC9A8;C20orf108;RASSF2;SLA2;EMILIN3;JPH2;CHRNA 4;C21orf124;D21S2056E;TRPM2;HIC2;LIMK2;APOL6;APOL1;MAP3K7IP1;TNR C6B;CSDC2;DERL3;LIF;C1QTNF6;KIAA1904;PDGFB;POLR3H;FLJ41993;SLC1 6A2;IQSEC2;OPHN1;RAB39B; |
| 3 | LRRIQ2;MME;KPNA1;LOC285382;SETD7;FYB;PTP4A1;LOC441179;TRAM2;IGF |

**Table 2**

| | |
|---|---|
| 3 | 2BP3;GPAM;TEAD1;PGM2L1;SCN3B;ADIPOR2;ARL1;APRIN;C14orf111;NIN;RA B8B;GABARAPL2;MGC19764;FOXA3;RP11-130N24.1;ITM2A; |
| 4 | PRDM16;PRAMEF9;EIF2C4;VANGL1;LOC440742;KLHL21;BSDC1;MCOLN2;ST 7L;TNNI1;SYT2;RIPK5;CHML;FLJ30851;FOSL2;FLJ16124;MXD1;MRPL35;KCN IP3;ARID5A;FLJ30294;MARCH7;ASB1;FLJ22746;C2orf19;C3orf19;NR2C2;NKTR ;PLXNA1;AP2M1;CDC25A;CACNA2D2;MGC40579;LRRC15;WHSC1;FLJ21511;P OLN;ELF2;GYPE;SLC1A3;SLC26A2;NDST1;ZDHHC11;GNPDA1;MGC21644;SH 3PXD2B;FLJ45422;RFP;CLIC5;HBS1L;AGPAT4;FOXK1;RALA;ADCY1;TSPAN33 ;RBM33;SCRN1;SLC35B4;FLJ12700;ZNF705CP;SLC7A2;VPS13B;FGFR1;ANKR D20A3;GCNT1;SLC31A1;PAPPA;C9orf7;WDR40A;C9orf48;BICD2;WDR31;SURF4 ;HSPA14;CNNM1;HIF1AN;CASP7;GPR26;MTG1;C10orf54;DNAJB12;KCNMA1; HTR7;TMEM10;SPFH1;FAM26C;C10orf46;ATHL1;P2RY6;FAM55B;MLL;CBL;PK NOX2;CTSC;BACE1;PEX5;GPRC5A;VDR;ADCY6;FMNL3;KITLG;C12orf49;FLJ4 0296;HS6ST3;ARHGEF7;LOC283487;C14orf24;DLST;VASH1;TCL6;NDRG2;DIO2 ;CGNL1;SLC24A1;ALPK3;FLJ43339;CSNK1G1;FLJ11506;TM2D3;PMM2;PSKH1 ;KIAA0174;ZNF694;CMTM4;MAF;MIS12;RAB11FIP4;LASP1;MAPT;IGF2BP1;GA S7;TMEM101;RECQL5;USP36;CANT1;FLJ90757;KIAA0427;MGC26694;LSM14A ;ZNF146;ZNF480;C20orf121;STK4;GTPBP5;FLJ44790;PTPRT;TP53RK;FLJ30313 ;TOM1;TOMM22;ZC3H7B;PPARA;C22orf13;NPTXR;PHF16;EDA;PCDH19;MCC; E2F3;IRF1;DAPK2;FZR1;RBBP4;TCF7L2;BCL2L2;SH3GLB1;BAG4;BMF;FAIM2; INHBA;SUFU;MDK;BCLAF1;TNFRSF7;TNFRSF14;STAT1;CCNE2;HDAC7A;PS EN1;PAFAH1B1;CARD14;APOE;SON;GTSE1;BNIPL;DEDD;PRKCE;ANAPC1;TP 73L;EREG;APBB2;PTP4A1;BAK1;CCNC;HIPK2;C10orf9;UNC5B;DPF2;AKT1;N GFR;MAPK1;G0S2;HCAP-G;FGF1;SASH1;MCM8;LATS1;TRAF2;CDKN2B;TEGT ;MCRS1;RB1;PRKCA;ATF5;DAPK3;SEPT3;FRAP1;PPP1CB;PDCD6IP;SEPT11;C DK6;TP53INP1;DLG5;SOCS2;CHES1;IGF1R;FTS;NF1;DIDO1;VHL;NEK1;HECA ;UNC13B;FGF3;NOL3;CROP;UHMK1;UNC5D;BCL2;MAPRE3;ADRA1A;TESK2; FXR1;SIAH2;RAD50;PIK3R2;PPM1F;BIRC4;SESN2;CIAS1;APPL;MCM2;UNG2; MAPK13;PARC;MAP3K5;PDCD2;TERF1;TNFRSF10B;RELA;HIC1;TNFSF12;NA LP1;CABLES1;PLCB1;BCL2L13;MTCP1;FGF2;NUDC;VEGF;SIPA1;TP53;CDKN 2D;BCL9L;AKT2; |
| 5 | NPAL3;EPB41;C1orf21;PPP1R12B;RIMS3;FLJ39739;BRRN1;BCL2L11;DUSP19; SFXN5;KLHL18;ARGFX;DTX3L;PLXNA1;TLOC1;OXTR;VGLL3;KIAA1727;C4orf 15;KIAA1909;ARHGAP26;ERGIC1;TAPBP;FOXK1;ADCY1;FLJ10803;CDK6;PDL IM2;PNMA2;FGD3;C9orf28;HSPA14;MRPS16;LRRC51;FUT4;ZBTB3;GAB2;B3G AT1;TSPAN9;APAF1;LNK;C14orf119;SPTLC2;BTBD7;CYP1A2;KIAA1924;MGRN 1;SPN;FLJ35848;PLXDC1;ACOX1;MXRA7;NPLOC4;YES1;ICAM1;LOC126536;F 2RL3;PGPEP1;UBA52;IXL;MBD3;ZNF490;CARD8;MGC35402;KIF3B;PARD6B;P DXK;HEMK2;CRKL;TOM1;GTSE1;KIAA1904;POLR3H;ZFX;TRAPPC2; |
| 6 | EFHD2;PADI6;NPAL3;EXTL1;TXLNA;KIAA0467;ARTN;CLCA2;FAM46C;HAPL |

**Table 3**

| | |
|---|---|
| 6 | N2;KIRREL;UCK2;PRELP;C1orf159;DVL1;NADK;SRM;MTHFR;RSPO1;KCNA3; B4GALT3;KLF11;MGC11266;FLJ46838;DDX18;NRP2;CDK5R2;APEG1;FARP2;I NG5;CYP26B1;TACR1;ADRA2B;FLJ14816;SAP130;BSN;PODXL2;RAB43;CAMK 2N2;LOC285382;MUC4;SH3BP2;RGS12;AFF1;PALLD;DGKQ;CTBP1;ADRB2;AB LIM3;CDX1;LOC134145;PCSK1;SEMA6A;IBRDC2;NRN1;LOC401286;C7orf20;LI MK1;GTF2I;POP7;WBSCR16;ACHE;CADPS2;ZNF467;SORBS3;RRS1;GRHL2;C8 orf55;PSD3;FLJ14107;TRIM35;DDEF1;STXBP1;FLJ36268;NDOR1;C9orf90;CNN M2;C10orf39;SEPHS1;DKFZp686O24166;CHEK1;C11orf37;CHID1;PHLDA2;ABC C8;MEN1;KIAA0404;LTBP3;BCL2L14;HOXC6;CTDSP2;SELPLG;MSI1;SLC7A1; COL4A1;KIAA0284;TMEM121;GREM1;PSTPIP1;SEMA4B;ATP10A;MYO5C;BRU NOL6;MMP25;FLJ14154;MGRN1;PMM2;SBK1;ATXN2L;TAOK2;FBXL19;STX4A; MYST1;NDRG4;C16orf54;NIP30;MGC11335;PDF;CHST6;TNFSF12-TNFSF13;M AP2K3;CD300LG;GPS1;FAM101B;RTN4RL1;CAMKK1;TOP3A;TRIM25;SLC39A1 1;CASKIN2;FSTL3;AMH;MCOLN1;MRPL34;KCTD15;MAG;FLJ90805;GEMIN7; VASP;PRRG2;BCL2L12;FPRL2;PRKCG;CACNG7;LMNB2;SGTA;MGC19604;SYT 3;LAIR1;C20orf102;STK4;CDH4;DNAJC5;SCRT2;CST9;SPATA2;C20orf175;FLJ9 0166;C20orf151;HUNK;C21orf125;TRPM2;DSCR1;LOC284861;GTSE1;LOC38888 2;C22orf13;TPST2;RRP22;SF3A1;DUSP18;CBX6;FLJ41993;TUBGCP6;TBL1X;MI D1;NXF5;FOSB;CDT1;HIC1;SEMA4D;C10orf46;IGF1R;TNFSF12;MAPK3;CGRE F1;CARD9;CUL1;SPHK2;UBE2V1;PROK1;NOTCH2;FHIT;UNG2;LZTS1;BIN3;P TCH;TRAF1;CCNL2;E2F2;TAIP-2;NME6;EREG;CASP6;IHPK3;PMS2;CDK6;BNI P3L;PTEN;DLG5;IGF2;PAK1;SESN3;BCAT1;C12orf22;DDIT3;CDK8;CHES1;AKT 1;CIAPIN1;NGFR;MNT;CDC34;AXL;PLCB1;DIDO1;NF2;MAPK1;KNTC1;NBL1; RAD21;PAFAH1B1;BCL2;CDC42;PEA15;RGS2;TGFB2;CIAS1;CASP9;NRAS;PRK CE;BCL2L11;PDCD6IP;FGF5;E2F3;SASH1;LATS1;CYCS;TNFSF8;BNIP3;API5;S SSCA1;RFP2;FOXO1A;NFKBIA;LTBP2;PPP1R13B;FGF7;MAPK6;DAPK2;ANAP C11;GAS7;DNM2;APOE;PPP2R1A;MX1;MN1;ERBB4; |
| 7 | KIF1B;ARID1A;FLJ25476;EIF2C1;KIAA0467;LENEP;C1orf21;ELF3;C1orf69;ZN F238;RERE;AK2;HEYL;RIMS3;SSX2IP;PDE4DIP;PRKAB2;FCRL5;STX6;NMNA T2;SLC41A1;FLJ25102;IL18RAP;PIP5K3;TNRC15;ASB1;FLJ45964;FLJ22746;C UL3;HDAC4;CTDSPL;ZNF621;NKTR;BSN;ARMC8;KLHL24;FAM79B;FAM107A; RAB43;AMOTL2;SHOX2;GP5;WHSC1;KIAA1727;PIK3R1;PHF15;SLC26A2;LAR P1;RPL37;TRIM36;ZCCHC10;MGC21644;SH3PXD2B;CDKN1A;LRRC1;SLC35F1 ;KIAA0319;PPP1R10;DKFZp779B1540;C6orf155;DEADC1;ADCY1;WBSCR1;ZNF 498;MKLN1;MGC33584;CLDN3;SPAG11;PPP2R2A;AP3M2;PPM2C;WISP1;KIAA 1875;LGI3;WHSC1L1;TRPS1;NTRK2;FOXE1;TMEFF1;ABL1;C9orf58;FAM102A; C9orf140;CHST3;PLAU;TSPAN14;SH3PXD2A;AP2A2;DKFZp686O24166;TSPAN 18;SLC35C1;ACAD8;BET1L;ZNF289;CTSC;B3GAT1;GNS;CDX2;SUCLA2;OTUB2 ;KIAA0125;NOVA1;KIAA0317;PPP1R13B;KLF13;PLEKHQ1;TMEM87A;KLHL25; SPN;LYPLA3;PDPR;MARVELD3;KIAA1970;ADAMTS18;GOSR2;WDR68;KCNJ2; |

**Table 4**

| | |
|---|---|
| 7 | C17orf27;CAMKK1;FLJ35773;C17orf76;CCL3L3;NPTX1;CEP192;SS18;NTE;IER2 ;LOC284361;ZNF562;OPA3;ZNF614;RNPC1;ZHX3;ABCC13;HUNK;BRWD1;NF2; GTPBP1;PPARA;PDGFB;LDOC1L;C22orf9;OGT;BCORL1;RGAG4;CD99L2; |
| 8 | H6PD;NPAL3;KPNA6;IL6R;ADORA1;MYOM3;ETNK2;LBH;POLR1B;ING5;C2orf 24;KIF1A;MRPS25;WHSC1;LPAL2;RPS6KA2;MAFK;YKT6;ADCY1;PHKG1;FLJ4 6365;GDF6;MTAP;TRAF2;DIRAS2;FANCC;GPR26;ZFP91;SVOP;ZNF605;RFP2;C GNL1;ALPK3;FLJ43339;FLJ46121;RAB11FIP4;IGFBP4;MAPT;RPRML;ERN1;PS CD1;MGC26694;EGLN2;ELAVL1;C20orf118;FLJ44790;PTPRT;PTK6;ZNF74;P2R XL1;NF2;LIMK2;ZC3H7B;PPARA;PANX2;KIAA1904;DNAL4;CLDN2;AFF2;TNF RSF14;CROP;TNFSF12;NBL1;PEA15;G0S2;DUSP4;C10orf46;DAPK2;GSPT1;BC L2;PIN1;DIDO1;CDC2L2;ABL1;NEK9;CCNF;MAPK7;BAK1;HRAS;FAIM2;DAPK 3;MAPK1;PCTK1;HTRA2;PKMYT1;CDKN2D;SIAH2;FGF1;PRKCA;DFFA;CASP8 ;FGF5;IRF1;BCAP29;MCRS1;GAS7;FOSB;TNFRSF1B;HSMPP8;BRCA2;KPNA2; NALP1;BBC3;BTG3;MN1;SFN;RBBP4;CDC20;DEDD;CFLAR;VHL;FGF2;CASP6; HIPK2;BIN3;TSC1;UNC5B;PARD3;EI24;SESN3;CUL4A;BCL2L2;VASH1;SMPD3 ;PAFAH1B1;APP;SEPT3;GTSE1;ARHGEF6;MCL1;STAT1;HD;CD38;NIPBL;MCC; LY86;MCM3;TACC1;TNFRSF10D;TRIM35;HDAC7A;DDIT3;RB1;CDK10;P2RX1; MAPK4;CDC34;ATF5;TNFSF7;PPM1F;BIRC4;IL19;RCC2;APBB2;SUPT3H;LATS 1;CCNE2;UNC13B;DPF2;H2AFX;PSEN1;SIAH1;NF1;FZR1;APOE;FAM58A;PRO K1;KCNIP3;DTYMK;PTP4A1;RAN;CGRRF1;JAG2;MAPK3;HIC1;SEPT5;DAP;CD KN2B;PPM1D;MLLT7;BRRN1;SLC12A4;E2F3;IER3;TRAF1;DIABLO;IGF1R;ER BB2;BCL9L;BCL9;AKT2; |
| 9 | H6PD;KIF21B;PODXL2;LGI2;FOXK1;RBM33;HR;C9orf25;NR5A1;RPP30;C10orf1 14;C11orf44;LOC283174;MAFG;TUBB4;DMPK;UBE2L3;F8A1;BMF;MCM5;TRIM 35;BIRC5;SCARB1;DIDO1;TACC1;CDC42;PMS2;MAPT;MLLT7;TNFRSF10B;CIA PIN1;CYLD;CGREF1;UNC5B;MDK;CD40;SFN;RAD17;RPS6KB2;E2F3;SEMA4D; BCL2L10;CDC6;CDKN2A;BOK;HD;VASH1;SESN2;PEA15;STK17A;RIPK2;TCF7 L2;BCAT1;HRK;CSK;TRAF7;HMG20B;PLK3;DFFA;PPP1CB;HK2;FANCD2;MCM 2;TADA3L;EREG;FGF5;SPP1;RAD50;CDKN1A;BAK1;CCND3;SSSCA1;EI24;SOC S2;FAIM2;ING1;FOXO1A;PSEN1;TRADD;CABLES1;PIK3R2;PPM1F;NHP2L1;P TCH;TSC1;PCOLN3;PRKCA;NFYC;RAP1A;CKS1B;CDC2L2;BRRN1;BCL2L11;RI F1;VHL;SKP2;FGF1;VEGF;CASP2;TRIAD3;CYCS;PARD3;PPP2R1B;PDE1B;LAT S2;IGF1R;TNFRSF12A;SLC12A4;SMPD3;TERF2;TXNL4B;CDK3;BCL2;TNFSF9; PIN1;UBA52;PDGFB;AKT3; |
| 10 | FAM79A;DFFB;LDLRAD2;EIF2C1;C1orf113;MPL;KIAA0467;HS2ST1;C1orf119;S NX27;QSCN6;RGL1;C1orf27;NAV1;PPFIA4;C1orf95;CHD5;MTHFR;FOXJ3;GNG 12;PTGER3;PAP2D;SORT1;GON4L;MEF2D;GPA33;CD34;ENAH;FLJ12788;RHB DD1;CAPN10;TMEM127;PAX8;FLJ10996;JAGN1;OXNAD1;CTDSPL;DLEC1;KL HL18;SRGAP3;CIDEC;THRB;FAM107A;CD47;LRRC15;TMPRSS11E;FRAS1;KIA A1727;PPP2R2C;RNF150;PCDHA9;N4BP3;UGT3A2;ETF1;DIAPH1;CCDC69;C5o |

**Table 5**

| | |
|---|---|
| 10 | rf4;SH3PXD2B;ALDH5A1;ZNF192;CDKN1A;TPMT;CPNE5;MDGA1;TRAM2;AGP AT4;WIPI2;LOC349114;HIG2;CASP2;ZNF282;MGC11257;FTSJ2;CDCA7L;DRCT NNB1A;HOXA1;URG4;TNS3;LOC401357;BAZ1B;SRCRB4D;PDK4;IMPDH1;KIA A0773;IKBKB;CA13;GRHL2;LRRC14;PAG1;ZNF406;CYP11B2;UBE2R2;NTRK2; NR4A3;PAPPA;GPR107;NDOR1;BAG1;C9orf100;FAM102A;VAV2;LOC399744;ZN F488;PPIF;SEMA4G;BTRC;SFXN2;NANOS1;GPR26;CCDC6;HPS1;COL17A1;FA M53B;GPIAP1;SLC35C1;TNFRSF19L;SNF1LK2;TAGLN;PKNOX2;SIRT3;APOA5 ;BACE1;CDON;C11orf39;ETV6;ZNFN1A4;MARCH9;FLJ11259;IFT81;OAS2;ULK 1;SLC6A12;SLC38A2;FMNL3;LOC144501;KRTHB2;TIMELESS;LRRC10;NUAK1 ;GIT2;CIT;GPR81;HS6ST3;ATP11A;DCAMKL1;KIAA0247;VASH1;C14orf153;KIA A0284;JUB;SPTB;RAB15;SNX1;SNX22;CD276;ARID3B;ARNT2;KIAA1199;IQGA P1;FLJ25222;ZFP106;RGMA;HSPC065;CBFB;E2F4;KIAA1576;KIAA0513;PRSS8 ;N4BP1;DYNC1LI2;C16orf7;MGC3101;ASPA;NLGN2;SARM1;FOXN1;RAPGEFL 1;CNP;IGF2BP1;WDR68;MAP3K3;KCTD2;SLC25A10;P2RX1;SLC13A5;SSH2;TN S4;EZH1;PLCD3;HOXB3;LRRC59;TRIM25;TRIM65;PSCD1;SEH1L;CEP192;C18o rf24;LPIN2;CBLN2;ATP5D;FZR1;ATCAY;BTBD14B;DMRTC2;BCAM;FLJ45445; MBD3;PLAC2;TUBB4;FLJ25758;RASGRP4;FLJ10241;GSK3A;LYPD3;PSMF1;ST K35;C20orf4;C20orf118;PPP1R16B;SLC12A5;STX16;LOC284757;ZGPAT;RASSF2; TRPC4AP;PTPRT;RIMS4;DYRK1A;C21orf124;AGPAT3;GAS2L1;CACNA1I;TEF;F LJ21125;MAPK1;LOC150383;SHOX;OGT;SPRY3;NUDT11;IQSEC2;DCX;CD99L2 ;SHOX;SPRY3;MCC;BIN3;SESN3;BCL2L13;E2F6;PTMS;FGF1;SIRT2;PRKCZ;H TRA2;MTSS1;CDKN2B;CCND2;APAF1;SCARB1;SMARCB1;RBBP4;RAP1A;CD3 8;PRLR;FOXO3A;CCND3;TEGT;TXNL1;DNASE2;SH3GLB1;RCC2;LY86;SESN1; PDCD2;DUSP4;SGPL1;HDAC7A;BTG1;PGF;CHES1;CDK10;TXNL4B;PCOLN3; MAPK4;SON;CUL4B;NUDC;TNFRSF14;NRAS;ZAK;CCNG2;BCAP29;PTCH;TNF SF8;TRAF1;INCENP;SSSCA1;FADD;FGF3;CIDEB;BMF;TRAF7;TNFRSF12A;TN FSF12;CABLES2;GTSE1;PPM1F;FASLG;HK2;TP73L;HD;SEPT11;MAD2L1;BNI P1;DAP;ESCO2;TNFRSF10D;PTEN;SUFU;HRASLS3;FAIM2;DDIT3;MAPK6;PL K1;NGFR;SPHK1;DPF1;SEPT3;SMC1L2;SMC1L1;SEPT6;MTCP1;CDC14A;PFD N1;HECA;DHX16;DOCK1;BNIP3;PDE1B;FOXO1A;UNC5A;GAS1;NF2;PAWR;AK T1;C10orf46;CARD14;TOB2;TNFRSF10B;AKT2;ERBB4; |
| 11 | LDLRAD2;SYPL2;SLC6A17;IL6R;RIMS3;SERBP1;TNRC4;MEF2D;ETNK2;BBS5 ;NEU4;ADRA2B;KIF1A;ZNF651;DAG1;HEMK1;ZNF445;FLJ37478;ERGIC1;N4B P3;ARSB;ZFAND3;ATXN1;CREBL1;PBX2;KCNK5;DKFZp761I2123;ADCY1;FLJ3 9237;LRRC4;KIF13B;MGC70857;FLJ20245;ST6GALNAC6;C9orf140;FRMD4A;SP OCK2;FAM53B;FLJ46300;BRSK2;INPPL1;ABCG4;ST3GAL4;MUCDHL;TOLLIP; OSBPL5;GAB2;SCN4B;TSPAN9;RND1;RARG;CENTG1;PPTC7;FLJ40296;LAMP 1;SLC7A1;POU4F1;VASH1;FLJ39779;FLJ42486;KLF13;BAHD1;PIGQ;FBXL19;P OLR2C;SLC7A6;KIAA0513;CCDC78;C1QTNF8;N-PAC;LOC283849;PRDM7;GOS R1;RASL10B;MAPT;CDK5RAP3;FLJ21865;DHX33;VAMP2;DUSP3;UBTF;APC2; |

**Table 6**

| | |
|---|---|
| 11 | C19orf36;FZR1;MAP2K7;BTBD14B;LOC284361;MKNK2;TNPO2;PRX;ERF;IGSF 4C;SAPS1;TM9SF4;LPIN3;ARFGAP1;FLJ41733;C21orf123;HIC2;GAS2L1;CABP7 ;MGC3731;PPARA;SAPS2;KIAA1904;CBX7;FLJ41993;AFF2;EMD; |
| 12 | PRAMEF9;VANGL1;RUNX3;PIK3R3;FCRL5;ENAH;RASGRP3;FLJ40432;SCLY;A SB1;THADA;ATG9A;BSN;HEMK1;FBXO40;CRBN;IQSEC1;EPM2AIP1;SEMA5B; MASP1;UGT2B15;ANKRD50;PIK3R1;DMXL1;ZNF346;IRF4;WIPI2;MKLN1;EXO C4;FLJ12700;SH2D4A;ADAMTS13;GABBR2;C9orf90;SFTPA1;KCNMA1;SFTPA2; TLL2;BLOC1S2;SH3PXD2A;CBL;DKFZP586D0919;RIC8B;STYK1;SMUG1;ARL1 1;LOC283487;NIPA1;FLJ38723;CIITA;LOC440350;CIAPIN1;MGC3101;RAB11FI P4;DDX52;FLJ90757;PDE6G;GALR1;COLEC12;ZNF497;GAS2L1;SYNGR1;ZC3H 12B;SLC16A2;ARMCX4;PHF6; |
| 13 | EFHD2;SOX11;FLJ32063;ZNF454;OGDH;FLJ39237;FLJ36268;C10orf58;ETV6;H OXC5;DOC2A;RUTBC1;CACNG4;LRRC45;FLJ46026;PTBP1;HSH2D;LOC284434 ;F2RL3;DIRAS1;LOC284296;GAB4;LIF;KIAA1904;PRKX; |
| 14 | SMAP1L;CELSR2;VANGL1;SNX27;SEMA4A;KIAA0446;FAM20B;PPP1R12B;ZN F436;ARNT;ANKRD45;APOBEC4;KIF21B;SMYD1;CNNM4;CYP26B1;AFF3;TFC P2L1;C2orf19;DOCK3;ZNF445;RAB6B;HD;HERC3;SETD7;CPLX2;N4BP3;DNAJ C18;SPRY4;C6orf134;FOXP4;VGLL2;RP5-875H10.1;NAG8;PMS2L2;SLC35B4;CR EB3L2;ADRB3;GPR107;ANKS6;C9orf88;MARVELD1;SFXN3;TMEM86A;ZFP91; MGC33846;CTSC;POU2AF1;DRD2;ZNFN1A4;ING4;C12orf53;GSG1;BAZ2A;FLJ4 0296;ZIC2;ZNF609;PML;SCAMP5;NFATC2IP;NFAT5;KIAA0513;PRSS33;LOC283 849;TERF2;ATP1B2;CNP;IGF2BP1;PRKCA;GAS7;C18orf1;F2RL3;GTPBP3;MGC 71805;PAX1;HNF4A;C20orf96;JPH2;RIMS4;TMEPAI;HMG2L1;KIAA1904;OGT;P OU3F4;IL13RA1;FAM9C;SMC1L1;APLN;MECP2; |
| 15 | PRDM16;NBL1;C1QA;C1orf83;GPR61;C1orf21;ELF3;ZNF672;RERE;IL28RA;LS M10;TTC22;DHCR24;PTGER3;KCNJ10;MPZ;MGC11266;EPAS1;TSN;MAP1D;FZ D7;CDK5R2;COL4A3;FLJ43374;EFEMP1;EN1;FLJ16008;SAP130;LOC440925;AT G9A;C2orf19;FLJ43879;KIF1A;IL17RE;NR2C2;CTDSPL;KLHL18;SEMA3F;MAP KAPK3;KLHDC6;ARMC8;LRRFIP2;SHOX2;MDS1;IGF2BP2;FLJ35725;RFC1;SN CA;UNC5C;DNAJA5;PHF15;ARHGAP26;ZDHHC11;PDE4D;KENAE;ZBED3;OTP ;APXL2;CAMK2A;MGC27121;ID4;KAAG1;LRRC1;SLC35A1;KIAA1913;C6orf208; HLA-DOA;MOCS1;CLIC5;ALDH8A1;FOXK1;EGFR;CLDN4;PRKRIP1;SLC26A4; KIAA0265;GSTK1;RBM33;FLJ44060;UBE2H;LOC401431;ATG9B;PTPRN2;TNKS ;GRHL2;TRMT12;LZTS1;MYST3;PLAT;ZNF704;C8orf54;MTSS1;NAPRT1;FGD3; NEK6;C9orf28;C9orf106;USP20;PAX5;KLF9;ZCCHC6;BICD2;GABBR2;ZYG11BL; TSC1;MGC61598;PLXDC2;PI4KII;BTRC;C10orf26;PDCD11;BAG3;C10orf38;SPO CK2;USP54;ABLIM1;USP47;TSPAN18;C11orf9;PLEKHB1;JAM3;BET1L;RIC3;M RGPRX2;UCP3;ARRB1;GDPD5;MCAM;P53AIP1;SNX19;C12orf59;TMEM117;CE NTG1;DCN;CORO1C;MSI1;HS6ST3;ATP11A;DCAMKL1;LOC283514;EBI2;DKFZ p451A211;LOC440157;PPP2R5C;C14orf80;ARHGAP11A;CAPN3;CD276;AKAP13; |

**Table 7**

| | |
|---|---|
| 15 | LOC56964;LCMT2;GNB5;KIAA1924;ZNF213;MGRN1;JPH3;FLJ45121;THUMPD 1;CMTM4;PHLPPL;CHST6;TUSC5;FAM64A;MGC19764;GRN;GOSR2;WFIKKN2; C17orf27;CAMKK1;SLC13A5;RAD51L3;MYOHD1;MEOX1;HDAC5;CD79B;ABCA 10;FAM104A;C17orf28;MGC15523;CTDP1;STK11;FLJ39501;SLC27A1;MAG;SIPA 1L3;SPINT2;IXL;VASP;PPFIA3;RPL13A;ZNF473;FLJ45684;ADAMTSL5;GNG7;P IP5K1C;LPHN1;DDX39;SF4;CKM;ZNF614;SOX12;STK4;C20orf161;C20orf177;C2 0orf175;C20orf151;IFNAR1;COL6A2;C21orf62;C21orf129;C21orf123;SEC14L2;RA SD2;MPPED1;GTSE1;ADM2;C22orf13;NIPSNAP1;SF3A1;ZNF278;KIAA1904;RA NGAP1;SULT4A1;C22orf9;FLJ41993;MAPK11;LOC440836;CPT1B;CHKB;WWC3 ;MSL3L1;FRMPD4;MAGEB18;RIBC1;ATP2B3;SMC1L1;LDOC1; |
| 16 | ATAD3B;THAP3;FLJ43806;FBLIM1;LIN28;PTPRF;VANGL1;IL6R;TOMM40L;PK P1;PFKFB2;TRIM67;CHD5;HES2;LUZP1;FAM77C;FLJ38984;RIMS3;SCMH1;PR KAB2;UBQLN4;NMNAT2;PLEKHA6;FOXD4L1;STEAP3;ZAK;WDR35;ADRA2B; KIF1A;CRTAP;STAC;ZNF651;LIMD1;CCR2;KLHL18;NUDT16;CHRD;RTP1;OXT R;SH3BP5;C3orf62;IHPK1;BAP1;RAB43;ZFYVE28;PPP2R2C;TMSL3;SETD7;SL C6A7;NDST1;ERGIC1;N4BP3;ARSB;IRF1;PCDH1;IBRDC2;BTN3A2;BTN2A1;PH F1;HMGA1;ANKS1A;CDKN1A;PI16;MDFI;MGC45491;C6orf47;MDGA1;KCNK5; BACH2;ECHDC1;TTYH3;FOXK1;LOC223075;YKT6;LIMK1;C7orf19;RBM33;WB SCR16;EPHB4;PODXL;TRPV6;EXTL3;LY6K;ZFP41;ZNF623;LOC349196;KIF13B ;ST18;GDF6;FLJ46072;C9orf94;UBE2R2;RP11-561O23.3;FLJ45537;DAB2IP;C9or f28;FAM73B;GRIN1;FLJ20245;FOXD4;C9orf25;ANKS6;AK1;GOLGA2;PRKG1;AC ADSB;LRRC20;C11orf11;TBC1D10C;RNF121;CAPN5;ABCG4;CBL;BET1L;MUC DHL;CD59;AGTRL1;DDB1;GAB2;FOXJ2;ETV6;TBK1;MSRB3;VAMP1;VDR;ARF 3;GOLGA2L1;AKAP11;POU4F1;KIAA0323;VASH1;TMEM63C;OTUB2;TCL6;THS D3;ITPK1;BAHD1;PML;KIAA1199;ISG20L1;MAN2A2;BMF;SHF;FLJ22795;HAG HL;CRAMP1L;TRAF7;DCTN5;FBXL19;SLC5A2;HSPC065;SLC7A6;FLJ20186;N- PAC;RRN3;FANCA;NLGN2;FLJ45455;FLJ35934;RAB11FIP4;CNP;ATP6V0A1;TB X21;PDK2;YPEL2;KCTD2;BAIAP2;ABR;SCARF1;SMG6;GAS7;CDRT4;FLJ44815; CCL3L3;ORMDL3;CCR7;SP6;HOXB13;C17orf73;BZRAP1;RNF43;FLJ46026;RNF 165;KIAA0427;P15RS;PQLC1;C19orf36;FZR1;MAP2K7;ILF3;BTBD14B;ZSWIM4; IXL;DMRTC2;HSU79303;MBD3;PRKACA;LASS1;PRX;BBC3;KCNA7;PTPNS1;P OFUT1;LPIN3;SLC23A2;ZHX3;RIMS4;FLJ30313;C21orf49;FLJ41733;AGPAT3;L ZTR1;MGC3731;TRIOBP;H1F0;MAP3K7IP1;TEF;RHBDD3;SYN3;KIAA1904;C22 orf5;TMSL3;RBM3;MLLT7;OGT;CXorf9;DDX26B;MAGEA8;PNMA3;APLN; |
| 17 | KIF1B;NCDN;DNAJC6;RGS4;C1orf76;NAV1;CTSE;SRGAP2;RSPO1;C1orf180;N MNAT2;PLEKHA6;GREB1;VSNL1;DPYSL5;EHD3;KCNIP3;NCK2;GLI2;CNTNA P5;ALS2CR13;MAP2;NRXN1;AFF3;MAP3K2;NR4A2;KLF7;MARCH4;C3orf35;RA B7;UNQ6125;HPS3;OXTR;CACNA2D2;AER61;VGLL3;SLITRK3;LEPREL1;BDH 1;FGFRL1;SRD5A2L;POU4F2;CYP4V2;LRP2BP;SNX2;PURA;UBLCP1;CDH12;S LCO4C1;REEP5;SEMA6A;LRRTM2;SPRY4;LHFPL5;GLP1R;NR2E1;TREML2;B |

**Table 8**

| | |
|---|---|
| 17 | VES;MGC33530;DKFZP434A0131;FLNC;INHBA;SAMD9L;TMEM130;PAX4;CRE B3L2;C8orf49;SCARA3;C8orf13;ANK1;ST18;PAG1;RUNX1T1;CYLC2;SLC31A1;B NC2;CDKN2B;OPRS1;ABO;TCF8;LOC440558;VAX1;ANKRD42;E2F8;SLC43A3;P PP2R1B;CDON;LRTM2;RPH3A;C12orf46;IGF1;DNAJC15;HS6ST3;ATP11A;F7;K ATNAL1;LHFP;C14orf118;CHES1;GABRB3;CCDC32;ITGA11;SBK1;SPN;ASCIZ; N-PAC;LOC283849;MAF;NLK;MNT;RCV1;TNS4;DCAKD;CD22;ZNF382;ZNF134; CYP4F11;TMEM16H;ZNF529;XRCC1;MCM8;KIAA1219;PPP1R16B;ZSWIM1;SL C23A2;CD93;C20orf128;CLIC6;SNAP29;TRIOBP;EP300;PPARA;XG;GLRA2;SSX1 ;SSX2;EFNB1;NLGN3;PCDH11X;CD40LG;AFF2;SSX5;SSX9;SSX3;SSX2;ARHGE F9;EDA2R;CHRDL1;DCX;SEPT6;PCDH11Y;SON;IGF2;HRASLS3;RFP2;LATS2;C CND1;TNFSF8;TXNL4B;ESCO2;DOCK1;TSC2;TRAF3;TRAF5;BCL2;SEPT3;PLC B1;DEDD;NGFR;P2RX1;HDAC9;C12orf22;TNFRSF10C;UNC5A;BCL2L11;ADRA 1A;SESN3;F2R;UBE2V2;DIDO1;APBB2;E2F3;PEA15;PRKCA;IL2RA;HD;ANKR D15;SGPL1;BRRN1;BAG2;TACC1;PTCH;PARD3;FAIM2;DEDD2;SEPT11;ATG12; CDK6;C10orf46;MAPK1;SLC12A4;ALOX15B;MAPRE2;ELMO2;BID;DMC1;TOB2 ;SUFU;MDM2;BMF;SMC1L1;CDK8;RTN4;CCNG2;PMS2;CDK9;DLG5;SCARB1; MCL1;PRKCE;ZAK;TNFSF10;LTA;TNFRSF21;SEMA4D;PTEN;BNIP3;FTS;CAB LES1;CCNL2;BOK;STAG1;FGF2;CASP6;TERF1;IFITM1;NOL3;CDK10;MAPK4; MLLT7;CDC2L2;CASP8;BNIP1;PARC;FOXO3A;TSC1;PDCD7;DNASE1;TNFRSF 14;TGFA;NEK11;LZTS1;PPP1CA;CARD14;MALT1;RASSF1;ADORA2A;ARHGEF 6;SASH1;MKI67;GAS2L3;PNN;CDC20;CASP10;FOXO1A;TAIP-2;PAFAH1B1;BC L9L;PYGO1; |
| 18 | ATAD3C;NMNAT1;KIF1B;DNAJC16;C1orf144;STX12;MED18;EIF2C4;EIF2C1;Z FYVE9;MIER1;FAM73A;CSF1;VANGL1;TBX19;LAMC1;FBXO28;ZNF238;C1orf5 3;EPS15;SERBP1;SEC63D1;WDR47;SHE;STX6;LPGAT1;ANGEL2;NUP133;MXD 1;PCYOX1;EPB41L5;GALNT13;PDK1;ZAK;CREB1;PIP5K3;WDR35;SERTAD2;L OC554251;TGOLN2;ACVR1;PGAP1;TGFBR2;ZNF197;DOCK3;KBTBD8;ACPL2; MBNL1;LRIG1;KIAA1160;PLSCR4;EIF5A2;TBL1XR1;STIM2;EREG;HERC3;TRI M2;PPARGC1A;CENTD1;GABRA4;ERBB2IP;CAST;LOC90355;PCDHB4;JARID2; PTP4A1;COL19A1;POU3F2;GJA1;KIAA1244;HECA;RP5-875H10.1;ESR1;QKI;AT XN1;ENPP5;CDC2L6;PBOV1;TXLNB;PIP3-E;GPR30;PHF14;CREB5;ADCY1;HB P1;MEOX2;STEAP4;FLJ31818;TFEC;WASL;CNOT4;MTMR9;SULF1;ZFPM2;UB E2W;FLJ20366;GDA;HABP4;SLC31A1;KIAA0367;UBQLN1;TSC1;C10orf6;KIAA0 157;FLJ45187;PARVA;FBXL11;FUT4;SBF2;CHST1;SHANK2;PGM2L1;PPP2R1B; DDX6;GOLT1B;KLHDC5;WNT1;APAF1;UBE3B;OAS2;ATP6V0A2;BHLHB3;TMT C1;KRT2B;IGF1;FLJ20674;RSN;PAN3;FLJ30046;ATP11A;RBM25;WDR20;EIF5; CFL2;KIAA0831;MAP3K9;RPS6KA5;DICER1;CHD2;NIPA1;LASS3;RUNDC2A;U BPH;SPN;GPT2;ARL6IP;DYNC1LI2;MAF;MLLT6;ZBTB4;KRTHA8;IER3IP1;LDL R;IXL;SF4;ZNF614;ZNF665;PLCB1;STK4;SPATA2;ZNF217;TMEM50B;BRWD1;T NRC6B;XK;ZC3H12B;OGT;BIRC4;OCRL;ARSD;PIGA;RPS6KA3;PCYT1B;OPHN |

**Table 9**

| | |
|---|---|
| 18 | 1;PDZD11;ACSL4;RAP2C;MECP2;IRF1;CDKL1;PTEN;CUL3;ATG12;ESCO2;RBB P4;TGFB2;CASP8;SEPT11;BTG1;SUGT1;FTS;SH3GLB1;TP53INP1;GADD45A;D EDD;BIRC6;FXR1;STK17A;TNFSF15;PSEN1;BAG5;CSK;CABLES1;UBA52;NGF RAP1;NUDC;PEA15;G0S2;TNFRSF9;SMC4L1;NME6;UBE1C;TFDP2;CASP6;TN F;MAP3K5;C10orf9;PTH;RAN;FAIM2;ING1;SPHK2;DEDD2;KLK10;CDC42;E2F2 ;APPL;SASH1;BCAP29;TACC1;CUL5;ATM;CNAP1;CDK4;FGF9;DAD1;NEK9;CY LD;SESN2;TGFA;EDAR;TP73L;AXUD1;RASSF1;HCAP-G;CCNI;MCC;DLG5;HIP K3;SESN3;CCND2;PAFAH1B1;GADD45B;DNM2;SNF1LK;PPM1F;TNFRSF1B;M DM4;DFFA;MAPRE3;CDKN1A;WEE1;IL18;BCL2L14;SOCS2;MPHOSPH9;TNFR SF19;GSPT1;TERF2;COL4A3;FASTK;TNFSF8;API5;EGLN3;GAS2L1;NF2;MAPK 1;TRIAD3;INHBA;TNFRSF10B;IFITM1;TESK2;DKC1;DFFB;E2F3;KIF11;IL19;H DAC9;CDK6;DUSP4;FOXO1A;SON;TTK;PDCD2;RFP2;ERBB4;ERBB3;BCL9L; |
| 19 | KIF1B;PINK1;C1orf164;C1orf83;NBPF15;SNX27;SNAPAP;VANGL2;PRRX1;BSD C1;EVI5;SLC16A1;NBPF14;CD244;C1orf116;WDR26;TOMM20;CHML;ANTXR1; PTPN18;ZAK;IL8RB;RBJ;RAPH1;ARL4C;FLJ43879;NR2C2;ARL13B;ARGFX;ZIC 1;ST6GAL1;LRCH3;LOC440944;VGLL3;MYLK;CENTB2;SLA/LP;CCDC4;SRD5A 2L2;FLJ11193;FLJ14054;LARP1;SGCD;CPEB4;LIFR;C5orf20;SLC17A4;PGBD1;P ACSIN1;C6orf65;HEY2;QKI;ADCY1;ABCA13;LOC441257;KIAA0828;GIMAP8;JA ZF1;ELMO1;LOC441257;CLN8;MGC34646;COLEC10;LYPLA1;MMP16;COMMD5 ;GNE;C9orf71;CCBL1;HSPA14;ANKRD30A;BTRC;GPR26;SFTPA2;SORBS1;SYT 8;ZNF215;DKFZp686O24166;NAALAD2;C11orf37;OPCML;GABARAPL1;LOH12C R1;KLHDC5;ATP2A2;OAS2;ZNF664;BCAT1;TMTC1;ITGA5;IGF1;RFXAP;ATP11 A;KLF12;KCTD12;SLC24A4;KIAA0317;CHES1;ARID3B;CRAMP1L;UBPH;HSPC 065;CDH5;CYB5B;KIAA0513;AFG3L1;SMG1;THUMPD1;PLLP;COX4NB;PCOLN 3;PAFAH1B1;BCL6B;ANKFY1;LOC641522;LIMD2;ZNF440;PLEKHF1;ZNF562;Z NF100;GIOT-1;KCNA7;ITCH;RBBP9;ETS2;ADARB1;BRWD1;CABP7;APOL6;CA CNA11;TNRC6B;ZC3H7B;MAPK1;LRP5L;CERK;TBL1X;GLRA2;ZXDB;EDA;AFF 2;ZNF185;ARSD;ZMAT1;DCX;IGF2;APOE;RAP1A;TNFRSF9;BCAP29;PLK4;UN C5D;FTS;CDC42;HRAS;PRKCA;PPP1CB;UBE2V2;CUL2;MDM2;TRAF4;PROK1; BCL2L11;CASP8;STAT1;FHIT;HD;FOXO3A;SASH1;CYCS;WEE1;HDAC7A;PCT K2;SUGT1;PNN;MAPK6;UBA52;APP;SEPT5;TGFB2;RAF1;FGF5;TLR2;MCC;TN F;CDKN1A;PTP4A1;CCNC;STK17A;TP53INP1;SEMA4D;ZWINT;GAS2;SESN3;P PP2R1B;DDIT3;BTG1;TSC2;DNASE2;PPM1F;DMC1;SMC1L1;KHDRBS1;NOTC H2;CGREF1;NEK11;PDCD6;HDAC9;CASP2;POLR3D;SULF1;KIF11;BNIP3;SOC S2;DIP13B;PSEN1;EGLN3;GSPT1;CARD8;FXR1;CDC23;MKI67;MAPK3;PPM1D; GAS7;HK2;NIPBL;LY86;DLG5;CD40LG;CIAPIN1;SESN1;JAK2;SGPL1;PTPN6;P ARD6B;CDC7;FADD;PPP1CA;DPF1; |
| 20 | PER3;GPR3;TXLNA;CACHD1;ST6GALNAC3;LRRC8D;VANGL1;C1orf161;RBM8 A;PPP1R12B;ZC3H11A;MDM4;CABC1;KIAA0090;ZNF436;ANKRD13C;ARHGAP 29;SHE;PTGS2;C1orf116;YOD1;DDEF2;ATP6V1C2;ZNF512;BIRC6;PCYOX1;IL18 |

**Table 10**

| | |
|---|---|
| 20 | R1;ACVR2A;FMNL2;ZAK;SLC39A10;CASP8;SUPT7L;REEP1;ARL5A;CD302;PG AP1;TRAK2;RAPH1;SLC16A14;CTDSPL;C3orf23;FLJ10213;ATP6V1A;EPHB1;T RPC1;B3GNT5;IL1RAP;COLQ;ARHGEF3;VGLL3;RABL3;MGC40579;PCGF3;WH SC1;TBC1D1;PKD2;FLJ20032;SYNPO2;TRIM2;KIAA1729;CLOCK;SCD5;DKFZp 686L1814;TIFA;PDE5A;ANKRD50;OTUD4;C4orf13;C4orf18;GPBP1;LOC90355;M EGF10;LOC133619;SMAD5;ARHGAP26;TCERG1;CPEB4;MTMR12;LIFR;PCSK1 ;C5orf13;KIF3A;NR3C1;C6orf151;RNF8;GRIK2;AKAP7;C6orf71;VIP;QKI;CCR6;E LOVL2;ATXN1;TMEM30A;BACH2;ATG5;MCMDC1;MAP7;PARK2;LOC90693;CR EB5;RABGEF1;PFTK1;PRKAR2B;MKLN1;GNA12;MGC42090;OSBPL3;IGFBP3; TNS3;SEMA3E;CDK6;NRCAM;WASL;PODXL;DLGAP2;TNKS;EFHA2;SPFH2;SL C30A8;MSR1;MYST3;PLAG1;VCPIP1;PAG1;RGS22;DMRT3;GDA;RP11-35N6.1;P SIP1;KGFLP2;ZA20D2;ROD1;LHX6;FAM102A;MGC14327;CUGBP2;GALNACT-2 ;SIRT1;GHITM;KIAA1128;CNNM1;BTRC;NMT2;NEBL;KCNMA1;PPP1R3C;TIA L1;ADAM12;GPIAP1;FLJ10661;ATM;CTSC;RAD51AP1;PRH2;CREBL2;ARID2;L ARP4;SLC25A3;FLJ42957;EPS8;RECQL;KRAS;SLC38A4;TMCC3;PAN3;KL;RFC 3;AKAP11;FLJ40296;CLYBL;C13orf6;ATP11A;RNF6;STARD13;DCAMKL1;DLEU 7;WDR23;AKAP6;FANCM;C14orf29;SOCS4;FBXO34;SLC25A21;FBXO33;SEL1L; ATXN3;GREM1;CASC5;SNX1;ARID3B;KIAA1024;ARNT2;LOC440295;ST8SIA2; EXDL1;GATM;PRTG;NOPE;C15orf5;TBC1D2B;MGC24665;ZNF720;CCL22;NFAT 5;FAM86A;THUMPD1;BOLA2;DYNC1LI2;PHLPPL;MAF;ZCCHC14;BCL6B;C17o rf39;STXBP4;DHX33;DDX52;FBXO47;LOC641522;TEX2;USH1G;RAB12;KIAA08 02;DTNA;SERPINB5;OSBPL1A;B4GALT6;MBP;EMR2;C20orf121;ARFGEF2;SS1 8L1;RASSF2;PAK7;PTPRT;DIDO1;PKNOX1;STCH;BRWD1;FLJ33814;TNRC6B;S EPT3;MAPK1;NPTXR;STS;ZFX;MAOA;OGT;dJ341D10.1;ZBTB33;AFF2;PIGA;PC DH19;SYTL4;ZMAT1;COVA1;PCDH11Y;DAZ2;PRLR;SH3GLB1;TNFSF8;GTSE1; PTEN;HK2;BRRN1;HCAP-G;C10orf46;PARD6B;TOB2;PRKCE;TIA1;ESCO2;TNF SF15;CDC2;CARD14;MALT1;CDC14A;FASLG;IL10;PPP1CB;EREG;SUPT3H;UN C5D;TCF7L2;CDKN1B;TNFRSF19;TFDP1;LATS2;BCL2L2;FGF7;RBL2;KATNB1 ;TRADD;CABLES2;DMC1;NOTCH2;TGFA;SMC4L1;TP73L;NIPBL;VEGF;CASP2 ;LZTS1;PTCH;TRAF1;ZWINT;MKI67;HRASLS3;CRADD;PNN;CROP;CIDEA;CA RD8;MN1;EDAR;IL1A;STK17B;RNF7;IL8;PDCD6;FGF1;HECA;SASH1;ELMO1;T RIM35;TP53INP1;RAD21;UNC5B;NOLC1;BNIP3;EXT2;MDM2;BTG1;RFP2;ING 1;VASH1;MAPRE1;NF2;TGFB2;CCNL2;CUL3;ACPP;STAG1;DAXX;SGK;INHBA; BAG4;TSC1;EI24;RAN;PAWR;CCNF;PRKCA;NALP1;SON;ADORA2A;DKC1;CUL 4B;PCAF;NME6;SEPT11;MCM3;AHR;API5;MTL5;DNAJA3;NBL1;BOK;BCAP29; INCENP;CYLD;BCL2L11;CLU;PAPD5;TRAF4;UHMK1;CASP10;F2R;NDN;NF1;P DCD8;DFFB;ERBB4;BCL9; |
| 21 | AGRN;PRKCZ;THAP3;PIK3CD;TMEM51;CTRC;EXTL1;ARID1A;C1orf84;MGC33 556;AQP10;HAPLN2;NOS1AP;RASSF5;GALNT2;UBE2J2;ZUBR1;ID3;IL28RA;CI TED4;SEMA6C;PYGO2;GPA33;OR2C3;SOX11;EMILIN1;HOXD3;ALPP;CAPN10; |

**Table 11**

| | |
|---|---|
| 21 | ING5;C1QL2;FLJ20297;HDAC4;MTERFD2;EDEM1;ARPC4;IL17RE;TATDN2;SL C6A1;DHX30;CACNA1D;PODXL2;DVL3;EIF4G1;PAK2;FOXP1;MCF2L2;CAMK2 N2;MFI2;WHSC1;LOC93622;C1QTNF7;SOD3;DAPP1;DCAMKL2;CPLX1;AFAP;P HOX2B;CXXC5;PCDHGA1;LTC4S;TPPP;SLC12A7;TSSK1;NEUROG1;PCDH12;S OX4;SLC22A7;LTB;TAPBP;LEMD2;LOC441150;OLIG3;C7orf20;MAFK;FOXK1;L OC441208;LOC441193;TRIAD3;PSCD3;HOXA3;LOC441268;TSGA13;C7orf21;FA M62B;VIPR2;C8orf49;BAI1;ZNF696;LRRC14;ANK1;PLAT;REXO1L1;PYCRL;SCR T1;MGC70857;KIAA1688;RG9MTD3;PALM2;NTNG2;KCNT1;CDKN2B;MGC6159 8;CHST3;C10orf132;NKX2-3;C10orf6;TRIM8;FLJ46831;C10orf39;NEUROG3;C10 orf56;VAX1;BRSK2;MRPL23;CD5;MARK2;CDC42EP2;CCND1;P2RY6;ZBTB16;M UCDHL;CHID1;CDKN1C;SF1;CDCA5;DKFZp761E198;MTL5;STARD10;SSPN;S LC35E3;ACADS;BCL7A;SFRS8;CACNA2D4;WASF3;SOX1;RGS6;KIAA0329;ZNF 219;SPTB;AKT1;MAPKBP1;Gcom1;MESP1;CRAMP1L;TNFRSF12A;MMP25;SBK 1;FBXL19;FUS;GPR114;HSPC171;GAS8;FBXL16;PRSS22;MGC13138;CMTM4;L OC283849;BCAR1;PKD1L2;CBFA2T3;ANKRD11;MGC26885;C16orf7;HIC1;NDE L1;NEK8;ANKRD13B;HSD17B1;SP2;CDK5RAP3;EPN3;DYNLL2;SYNGR2;ANA PC11;LRRC45;CAMTA2;TNS4;VAT1;C1QL1;ARHGAP27;SP6;HOXB13;SEPT4;CY GB;CBX4;C17orf55;SECTM1;RALBP1;C19orf20;HCN2;PTBP1;STK11;MIDN;MU M1;APC2;NRTN;MAP2K7;JUNB;SIN3B;F2RL3;PIK3R2;FLJ20850;UPF1;KCTD1 5;SAMD4B;SNRP70;LOC91661;CACNG8;LENG8;KIAA1193;FLJ45684;PRG2;C19 orf25;TCF3;PRSS15;ELAVL1;CACNA1A;DDX39;LASS1;ALKBH6;FLJ13265;SAP S1;ZNF579;DLGAP4;SLC32A1;FLJ44790;NTSR1;OGFR;C20orf28;NKX2-2;DUSP 15;TMEPAI;GATA5;ARFRP1;CRYAA;ADARB1;RAB36;SNRPD3;TOM1;MGAT3;P PARA;CRELD2;DERL3;RRP22;SELM;POLR3H;PACSIN2;C22orf9;TUBGCP6;PL CXD1;CSF2RA;CD99L2;HCFC1;PLCXD1;CSF2RA; |
| 22 | MFN2;ADPRHL2;C1orf113;PPP1R12B;C1orf91;ADAR;C1orf36;SLC1A4;ALPI;FLJ 34931;TMEM127;PAX8;NR4A2;DAG1;FAM55C;SEC61A1;SMC4L1;PDLIM5;MTT P;TRIM2;PGRMC2;NDST1;SEMA6A;RUNX2;FLJ43752;MDGA1;LPAL2;AMZ1;F OXK1;ADCY1;SAMD9;LOC401431;C8orf4;NCALD;PALM2;MRRF;TSCOT;LDB3; SH3PXD2A;GALNAC4S-6ST;LOC220070;PACSIN3;ANKRD13;ADCY6;ZBTB39;G PR109A;RP11-50D16.3;DCUN1D2;FLJ20582;ZFF106;FLJ22795;PDPR;KIAA0513 ;TUSC5;MAPT;MNT;ANKFY1;GAS7;CDRT4;ZNF562;TM6SF2;RAB22A;DYRK1A; ADARB1;SYNGR1;ZC3H7B;LDOC1L;FLJ10945;TMEM29;BIRC4;SPRY3;CRSP2; TMEM29;ACSL4;SPRY3;APOE;PEA15;CDT1;SMPD3;NOTCH2;PPM1F;MAPK3; PRLR;ABL1;STIM1;MLLT7;LZTS2;SCARB1;ATG12;HSMPP8;NOL3;FOSB;SESN 2;BCAP29;PMS2;TP53INP1;C10orf9;HRK;BCL2L2;BNIP2;TRAF7;NGFR;PRKCA ;PROK1;RCC2;DEDD;HK2;HDAC4;TNFSF10;F2R;CDKN1A;FOXO3A;IHPK3;SG PL1;WEE1;APBB1;CDK4;DIP13B;CHES1;AVEN;BMF;CSE1L;DIDO1;PDGFB;SE PT6;YWHAQ;ACPP;TNFSF8;TSC1;FAIM2;DDIT3;CHFR;MAPK4;CCNE1;KLK10; KHDRBS1;PAK1;PAPD5;GSPT1;CDC14A;CKS1B;KCNIP3;CASP10;TGFA;ANAP |

**Table 12**

| | |
|---|---|
| 22 | C1;SEPT11;NIPBL;PPP2CA;LTA;AIF1;HDAC9;TACC1;LZTS1;SEMA4D;ZWINT; GAS2;CDKN1C;FGF3;NFKBIA;MOAP1;E2F4;TNFSF9;CD40;ELMO2;RBBP4;IL2 4;E2F2;ZAK;E2F6;HECA;IER3;PTEN;MDM2;AKT1;CDK10;CROP;SEPT4;UHRF 1;MCM5;ERBB4;BCL9L; |
| 23 | FLJ43806;SEPN1;PKP1;CNTN2;ASB1;ADD2;KLHL18;WHSC1;KIAA1909;SLC36 A1;N4BP3;SLC6A3;TRIM39;MDGA1;PDLIM2;GPR124;LZTS1;PAX5;TRAF1;GPR 26;HPS1;DKFZp686O24166;FBXL11;STS-1;OAS3;KLF12;SPTB;SPTLC2;CSNK1 G1;FLYWCH1;ZNF263;CLCN7;FLJ43980;BRCA1;LOC128439;CD93;TNRC6B;DG CR2;POLDIP3;ATP1B4;ZMAT1; |
| 24 | PRAMEF9;PQLC2;KLF17;MGC52498;HHLA3;PTGFRN;CACNA1E;LAMC1;MAP KAPK2;DISC1;STK40;SEP15;NBPF11;CTSS;SLC39A1;RIPK5;BCL2L11;DDX18; RBJ;MALL;PAX8;NUDT16;CDV3;FAM62C;FETUB;RAB6B;FRAS1;GABRA4;SGC B;ENPP6;ITGA2;ABLIM3;C5orf20;GUCA1A;LOC441179;BVES;CLDN4;STEAP2; CALCR;GPR85;LOC401410;CLEC5A;PDLIM2;PNOC;PSD3;NKX3-1;SLC31A2;E GFL7;KLF9;TRAF1;TOR2A;ENG;ZNF37A;NANOS1;LOC387646;NRP1;SLC16A9; KCNMA1;SH3PXD2A;FGFR2;MKI67;SPRN;RNF31;PTGDR;JUB;RAB15;ZFYVE 1;LOC440295;C15orf42;DKFZp434I1020;UBPH;RNF40;CES2;LOC283849;KSR1; ADAM11;raptor;TCF2;BZRAP1;ST6GALNAC2;LPIN2;ATCAY;FLJ39501;CDS2;P OFUT1;PPP1R16B;YWHAB;PTPRT;AGPAT3;POFUT2;CACNA1I;CSDC2;TMEM3 5;AMMECR1;CD99L2;PEA15;TEGT;TRAF7;SH3GLB1;BIRC1;BAG5;ZMYND11;I L2RA;PPP2R1B;FZR1;SEPT6;MTCP1;HD;CYCS;POLR3D;LZTS1;LALBA;VASH1; CCNF;DNAJA3;NGFR;BCL2;BCL2L12;GTSE1;TAF1;IL10;FGF2;RIPK1;SEMA4D ;C10orf46;SCARB1;KATNB1;GAS7;NOTCH2;DEDD;CARD6;PLAGL1;BNIP3L;U NC5D;DLG5;IGF2;CDKN1B;FGF9;TRAF3;TSC2;HIC1;PPM1D;BBC3;MAPK1;RB BP4;CLSPN;NEK4;E2F3;BIN3;DUSP4;NOLC1;FADD;H2AFX;PAWR;TNFRSF19; BMF;RBL2;MCM8;IL1A;NEK11;PRLR;ABL1;API5;HDAC7A;CSK;PRKCA;F2R;S TRN3;NF1;NF2;SEPT1;MCM6;AKT2;ERBB3; |
| 25 | C1orf188;ARHGEF10L;ZBTB40;EPB41;PODN;SLC30A7;INPP5B;ADAMTS4;EG R4;LOC440925;ZNF621;IHPK1;TRIM59;MCF2L2;LOC285382;WHSC1;KIAA1909 ;SERF1A;TPPP;PDLIM7;RING1;FOXK1;ARHGEF5;NOM1;LOC401357;KIAA077 3;GRHL2;ZFP41;C8orf77;LGI3;MGC70857;DAB2IP;STXBP1;FREQ;DBH;FNBP1; FRAT1;C10orf65;SYT15;ZBTB3;DHCR7;KRT1B;HPD;KIAA0284;BTBD7;ARHGA P11A;KIAA0513;GNG13;LOC283849;ALKBH5;NKIRAS2;RECQL5;SCAMP4;MRP L4;FLJ39501;EHD2;ZNF490;HAPLN4;SOX12;RASD2;KIAA1904;ARHGAP6;TRA PPC2;IQSEC2; |
| 26 | ATAD3C;RER1;ACTRT2;PHF13;SPSB1;KIF1B;ANGPTL7;NBPF3;YBX1;VCAM1; CSF1;RBM15;NBPF15;H4/o;ATP8B2;GJA12;RHOU;CPSF3L;FAM46B;TRAPPC3; MOBKL2C;PDZK1IP1;NBPF11;NBPF14;H4/o;FCRL5;ZNF669;FLJ25102;LBH;PO LE4;STEAP3;DNAJB2;GMPPA;SAG;LOC339768;GPC1;ASXL2;FLJ34931;OXER1 ;TGFA;DUSP2;HDAC4;KIF1A;CRTAP;NISCH;FNDC6;ACTR8;MAGI1;FOXL2;KL |

**Table 13**

| | |
|---|---|
| 26 | HL6;C3orf34;BDH1;FLJ23191;B4GALT7;SLC12A7;DP58;HBEGF;ATOX1;STC2;H FE;FOXP4;SH3BGRL2;C6orf162;TBP;FKBP5;LRFN2;DSCR1L1;COL12A1;C7orf2 0;FOXK1;ADCY1;FZD1;METTL2B;CASP2;RBM33;ECOP;CALN1;PDAP1;GATS;L RCH4;KBTBD11;TNKS;SCARA3;ELP3;TM7SF4;ZNF696;RAB11FIP1;IL7;NRBP2 ;COMMD5;TMOD1;SLC25A25;PPP2R4;FREQ;GTF3C5;UAP1L1;MGC59937;ENG ;MGC61598;AKR1C3;RASSF4;VCL;PI4KII;DPYSL4;IDI1;KLF6;C10orf67;ARL3;V AX1;FAM53B;SPRN;SMPD1;EHF;TBC1D10C;LOH11CR2A;SLC37A2;RAB6IP1;F LJ46154;CD59;FADS1;IL18;FLJ25530;ETS1;DDX11;XPOT;UBE3B;PUS1;ZNF384 ;C12orf53;FOXN4;HSMPP8;IPF1;C13orf6;PCDH9;RNF31;GNG2;PPM1A;CHX10; FLJ25773;C14orf1;LOC400968;ITPKA;FURIN;LOC146325;MKL2;HAS3;WWP2;K IAA0513;FLJ45121;MEFV;FLJ32130;C16orf58;NIP30;ANKRD11;C17orf49;SARM 1;TAOK1;MAPT;AKAP1;METTL2A;FLJ21865;SLC25A10;KIAA1267;COL1A1;TRI M25;SEPT4;AZI1;ATP9B;ICAM4;LDLR;SIPA1L3;GEMIN7;LOC400707;GLTSCR2 ;LENG8;BTBD2;MKNK2;C19orf35;GNG7;SEMA6B;KHSRP;KEAP1;EMR2;CHER P;HAPLN4;SERTAD3;ENTPD6;PYGB;KIF3B;KIAA1219;FKBP1A;B4GALT5;ATP 9A;DIDO1;SAMD10;TMEM1;ADARB1;HSF2BP;C21orf93;HIC2;RAB36;SUSD2;K IAA0376;MPST;TNRC6B;APOL2;KIAA1904;MFNG;C22orf9;TBL1X;FRMPD4;RP 1-112K5.2;ZBTB33;VBP1;P2RY8;SEPT6;MECP2;P2RY8; |
| 27 | CAMTA1;TTF2;DNM3;NR5A2;ZC3H11A;TRIM63;ZCCHC11;ENSA;ARID4B;HTL F;ITGA6;ITGAV;BMPR2;PUM2;PGAP1;RAPH1;NLGN1;OPA1;FYTTD1;WNT5A;P PP3CA;PDE5A;MARCH6;SCAMP1;RICTOR;LYSMD3;ST8SIA4;NR3C1;QKI;ICK; CDC2L6;KIAA0408;AHR;HDAC9;LOC90693;LOC441257;AKAP9;FAM3C;CRISPL D1;OTUD6B;POLR2K;PLAG1;FSD1CL;ROD1;WDR37;BTAF1;MBL2;SORBS1;RA B11FIP2;ZF;NUDT4;FLJ10292;LMO3;ZBTB39;NFYB;PAN3;NBEA;KATNAL1;PC DH8;KLF12;EML1;PPP2R5C;ARPP-19;SOX9;ZNF161;VAPA;MALT1;ZNF537;ITC H;PARD6B;RAB22A;CBLN4;APOL6;EGFL6;AFF2;EIF1AX;ATRX;AMMECR1 |
| 28 | CAMTA1;TTF2;DNM3;NR5A2;ZC3H11A;TRIM63;ZCCHC11;ENSA;ARID4B;HTL F;ITGA6;ITGAV;BMPR2;PUM2;PGAP1;RAPH1;NLGN1;OPA1;FYTTD1;WNT5A;P PP3CA;PDE5A;MARCH6;SCAMP1;RICTOR;LYSMD3;ST8SIA4;NR3C1;QKI;ICK; CDC2L6;KIAA0408;AHR;HDAC9;LOC90693;LOC441257;AKAP9;FAM3C;CRISPL D1;OTUD6B;POLR2K;PLAG1;FSD1CL;ROD1;WDR37;BTAF1;MBL2;SORBS1;RA B11FIP2;ZF;NUDT4;FLJ10292;LMO3;ZBTB39;NFYB;PAN3;NBEA;KATNAL1;PC DH8;KLF12;EML1;PPP2R5C;ARPP-19;SOX9;ZNF161;VAPA;MALT1;ZNF537;ITC H;PARD6B;RAB22A;CBLN4;APOL6;EGFL6;AFF2;EIF1AX;ATRX;AMMECR1 |
| 29 | KIF1B;ARID1A;FLJ25476;EIF2C1;KIAA0467;LENEP;C1orf21;ELF3;C1orf69;ZN F238;RERE;AK2;HEYL;RIMS3;SSX2IP;PDE4DIP;PRKAB2;FCRL5;STX6;NMNA T2;SLC41A1;FLJ25102;IL18RAP;PIP5K3;TNRC15;ASB1;FLJ45964;FLJ22746;C UL3;HDAC4;CTDSPL;ZNF621;NKTR;BSN;ARMC8;KLHL24;FAM79B;FAM107A; RAB43;AMOTL2;SHOX2;GP5;WHSC1;KIAA1727;PIK3R1;PHF15;SLC26A2;LAR P1;RPL37;TRIM36;ZCCHC10;MGC21644;SH3PXD2B;CDKN1A;LRRC1;SLC35F1 |

**Table 14**

| | |
|---|---|
| 29 | ;KIAA0319;PPP1R10;DKFZp779B1540;C6orf155;DEADC1;ADCY1;WBSCR1;ZNF 498;MKLN1;MGC33584;CLDN3;SPAG11;PPP2R2A;AP3M2;PPM2C;WISP1;KIAA 1875;LGI3;WHSC1L1;TRPS1;NTRK2;FOXE1;TMEFF1;ABL1;C9orf58;FAM102A; C9orf140;CHST3;PLAU;TSPAN14;SH3PXD2A;AP2A2;DKFZp686O24166;TSPAN 18;SLC35C1;ACAD8;BET1L;ZNF289;CTSC;B3GAT1;GNS;CDX2;SUCLA2;OTUB2 ;KIAA0125;NOVA1;KIAA0317;PPP1R13B;KLF13;PLEKHQ1;TMEM87A;KLHL25; SPN;LYPLA3;PDPR;MARVELD3;KIAA1970;ADAMTS18;GOSR2;WDR68;KCNJ2; C17orf27;CAMKK1;FLJ35773;C17orf76;CCL3L3;NPTX1;CEP192;SS18;NTE;IER2 ;LOC284361;ZNF562;OPA3;ZNF614;RNPC1;ZHX3;ABCC13;HUNK;BRWD1;NF2; GTPBP1;PPARA;PDGFB;LDOC1L;C22orf9;OGT;BCORL1;RGAG4;CD99L2 |
| 78 | PFKFB2;RCOR3;PRKAB2;RGS5;RIPK5;LYST;ACTR2;CASP10;YWHAQ;MLH1;F AM79B;CD47;C1QTNF7;KLF3;DKFZp761B107;RFC1;RCHY1;KCTD16;GCNT2;Z NF192;C6orf71;SERINC1;RPS6KA2;ST7;PURB;TMEM55A;ROD1;NANOS1;CTS C;C11orf45;PRH2;DNM1L;TMEM19;KRAS;NT5DC3;KATNAL1;RGS6;C14orf129; SNX1;ST8SIA2;FLJ36492;VAPA;SEH1L;SIN3B;ZNF562;ZNF571;C19orf37;OPA3; C20orf121;TMEM1;MN1;ARHGEF9; |
| 79 | VPS13D;C1orf21;CSMD2;SORT1;PRKAB2;C1orf36;SH3BP4;PAX8;PGAP1;IGFBP 5;OXNAD1;WHSC1;STIM2;KLHDC3;PAQR8;MDGA1;FOXK1;LOC441257;PRKRI P1;CREB3L2;LOC401431;PAPPA;TRAF1;ZNF37A;CCNJ;DKFZp686O24166;SCN 2B;AQP2;TXNRD1;SUDS3;FMNL3;ITR;EFNB2;GPR68;Gcom1;FLJ22795;TUSC5; CNP;PTRF;RNF24;BCR;ZC3H7B;KIAA1904;SLC6A8;SEPT6;APLN; |
| 80 | KIF1B;EIF2C1;ZFYVE9;IL6R;RIPK5;ENAH;POLR1B;C2orf24;CDC25A;CELSR3; RAB6B;WHSC1;N4BP3;PACSIN1;C6orf85;CREBL1;BVES;LPAL2;ADCY1;FLJ46 365;ABL1;RXRA;FANCC;PHF19;GPR26;LRRC20;NRXN2;ETS1;TSPAN9;RNF41; THRAP2;C14orf1;GPR68;JAG2;SNX1;C16orf34;CX3CL1;FLJ45121;C17orf59;GFA P;HOXB13;TRIM25;LIMD2;NPTX1;CTAGE1;F2RL3;ELAVL1;ATRN;SLA2;PDXK; NF2;PPARA;FLJ21125;LDOC1L;DCX; |

The method of screening for a substance that promotes or suppresses the expression or a function of a micro-RNA or micro-RNA precursor using a nucleic acid, micro-RNA or micro-RNA precursor of the present invention may be any method, as far as it is a method of screening for a substance that promotes or suppresses the expression or a function of a micro-RNA or micro-RNA precursor of the present invention. For example, a method can be mentioned wherein a vector that expresses a nucleic acid, micro-RNA or micro-RNA precursor of the present invention,is introduced into a cell, and a substance that enhances the suppressive effect on the expression of an mRNA having a target nucleotide sequence thereof, or a substance that promotes the expression of an mRNA having a target nucleotide sequence thereof, is screened for.

The cell incorporating a nucleic acid, micro-RNA, micro-RNA precursor or vector of the present invention may be any cell, as far as a nucleic acid, micro-RNA, micro-RNA precursor or vector has been introduced in vitro. Specifically, mesenchymal stem cells and cells resulting from differentiation of mesenchymal stem cells, for example, cells that are present in tissues such as bone marrow, fat tissue, umbilical blood, endometrium, dermis, skeletal muscles, periosteum, dental follicles, periodontal membranes, dental pulps, and dental germs, can be mentioned; for example, osteoblasts, adipocytes, and muscle cells and the like can be mentioned.

A pharmaceutical with a nucleic acid, micro-RNA or micro-RNA precursor of the present invention as an active ingredient can be used for the diagnosis or treatment of a disease caused by an abnormality of mesenchymal stem cell proliferation and/or differentiation.
As diseases caused by an abnormality of mesenchymal stem cell proliferation and/or differentiation, specifically, cancers and dysosteogenesis, achondroplasia, diabetes and the like can be mentioned.

A pharmaceutical with a nucleic acid, micro-RNA or micro-RNA precursor of the present invention as an active ingredient can also be used to diagnose or treat a disease caused by a mast cell abnormality. Because mast cell abnormalities include degranulation abnormalities, a nucleic acid, micro-RNA or micro-RNA precursor of the present invention can also be used as an agent for promoting or suppressing mast cell degranulation.
As diseases caused by a mast cell abnormality, specifically, atopic dermatitis, asthma, chronic obstructive lung disease, and allergic disease and the like can be mentioned.

Furthermore, a pharmaceutical with a nucleic acid, micro-RNA or micro-RNA precursor of the present invention as an active ingredient can be used to diagnose or treat a disease caused by an abnormality of cell proliferation and the like, tissue hyperplasia and the like. A nucleic acid, micro-RNA or micro-RNA precursor of the present invention can also be used as an agent for suppressing or promoting cell proliferation. Here, an abnormality of cell proliferation refers to a condition wherein cells are proliferating at a rate that is not a normal proliferation rate in a living organism.

As diseases caused by a cell proliferation abnormality, tissue hyperplasia and the like, specifically, cancers, arteriosclerosis, rheumatoid arthritis, prostatic hyperplasia, blood vessel restenosis after percutaneous transvascular coronary angioplasty, fibroid lung, glomerulonephritis, autoimmune diseases and the like can be mentioned.
Hereinafter, the present invention is described in detail with reference to cases wherein the nucleic acid, micro-RNA or micro-RNA precursor of the present invention is an RNA.

### 1. Identification of micro-RNAs and micro-RNA precursors derived from mesenchymal stem cells

### (1-1) Acquisition and cultivation of mesenchymal stem cells

The method of acquiring mesenchymal stem cells from bone marrow is not particularly limited, as far as it ensures safe and efficient acquirement; for example, a method described in S.E. Haynesworth et al. Bone, 13, 81 (1992) can be mentioned.

In the sternal bone or iliac bone, the skin at the site for bone marrow paracentesis is disinfected, and particularly the subperiosteal region is sufficiently anesthetized topically. The inner cylinder of the bone marrow paracentesis needle is removed, a 10-ml syringe containing 5,000 units of heparin is attached, and a necessary volume, usually 10 ml to 20 ml, of bone marrow fluid is aspirated. The bone marrow paracentesis needle is detached, and compressive hemostasis is performed for about 10 minutes. The bone marrow fluid acquired is centrifuged at 1,000×g, and marrow cells are recovered, thereafter the marrow cells are washed with phosphate buffer solution (phosphate-buffered saline) (PBS). After centrifugation and washing are repeated in 2 cycles, the marrow cells are suspended in a medium for cell culture such as α-MEM (α-modified MEM), DMEM (Dulbecco's modified MEM) or IMDM (Isocove's modified Dulbecco's medium), containing 10% fetal bovine serum (FBS), whereby a marrow cell fluid is obtained. The method of isolating mesenchymal stem cells from the marrow cell fluid is not particularly limited, as far as it allows the removal of other cells that are also present in the marrow cell fluid, for example, hematopoietic lineage cells, hematopoietic stem cells, vascular stem cells, fibroblasts and the like; for example, a method described in M.F. Pittenger et al. Science, 284, 143-147 (1999) can be mentioned. The marrow cell fluid is overlain on Percoll having a density of 1.073 g/ml, and then centrifuged at 1,100×g for 30 minutes, whereby the cells in the interface can be isolated as mesenchymal stem cells. Also, an equal volume of Percoll diluted to 9/10 by the addition of 10×PBS is added to, and mixed with, the marrow cell fluid, after which the mixture is centrifuged at 20,000×g for 30 minutes, whereby cells in a fraction having a density of 1.075 to 1.060 can be isolated as mesenchymal stem cells.

Mesenchymal stem cells derived from human bone marrow can also be purchased from Cambrex and Takara Bio Inc.
The method of acquiring mesenchymal stem cells from the umbilical cord is not particularly limited, as far as it ensures efficient acquirement; for example, a method described in Stem Cells, 21, 105-110 (2003) can be mentioned. A cannula is inserted into both ends of the umbilical vein, and the vein is washed with an appropriate buffer solution, for example, EBSS (Earle's balanced salt solution). An antibiotic is added to a 199 medium containing a protease, for example, 0.1% collagenase, and this is injected into the blood vessel, and incubated at 4 to 40°C, preferably at 37°C, for 1 to 60 minutes. The blood vessel is washed with EBSS, and the umbilical cord is gently massaged, after which a suspension of endothelial cells and cells under the endothelium are recovered. The suspension is centrifuged at 600×g for 10 minutes, and the cells obtained are suspended in, for example, a medium prepared by adding 20 mM HEPES, 100 units/ml penicillin, 100 µg/ml streptomycin, 2 mM L-glutamine, 1 mM sodium pyruvate and 10% FBS to a DMEM medium containing a low concentration of glucose (DMEM-LG, Gibco). The cell density is adjusted to 10² to 10⁶ cells/cm², and the cells are inoculated to a culture flask and cultured under the conditions of 37°C and 5% CO₂. While changing the medium every 1 to 7 days, the cultivation is continued for 1 to 3 weeks, whereby mesenchymal stem cells can be acquired.

The method of acquiring mesenchymal stem cells from the endometrium is not particularly limited, as far as it ensures safe and efficient acquirement; for example, a method described in Am. J. Pathol., 163, 2259-2269 (2003) can be mentioned. Human endometrial tissue extirpated by surgical operation is shredded, and cultured with a medium that allows cell culture, preferably a medium prepared by adding 1 to 20% animal-derived serum, preferably 5 to 10% FBS, to α-MEM, DMEM, IMDM and the like. The medium may be supplemented with antibiotics such as penicillin and streptomycin. Furthermore, to facilitate cell separation, a collagenase such as type 3 collagenase and a DNAase such as deoxyribonuclease I are added to the medium, and the medium is gently shaken at 20 to 40°C, preferably at 37°C, for 10 minutes to 5 hours, preferably for 1 hour. Each endometrial gland is separated while examining microscopically, and cultured in an appropriate culture vessel, for example, a 24-well culture dish, under the conditions of 37°C and 5% CO₂, whereby mesenchymal stem cells can be acquired.

The method of acquiring mesenchymal stem cells from a tooth, a dental germ, or periodontal tissue is not particularly limited, as far as it ensures safe and efficient acquirement; for example, a method described in Lancet, 364, 149-155(2004), Proc. Natl. Acad. Sci. USA, 97, 13625-13630 (2000) can be mentioned. The human tooth may be any one of deciduous teeth and permanent teeth such as incisor teeth, canine teeth, premolar teeth, and molar teeth. For example, the periodontal ligament is carefully separated from the surface of the root of a third molar (wisdom tooth) extracted, and a digestive reaction is carried out using a protease such as collagenase, trypsin, pronase, elastase, dispase, or hyaluronidase at 37°C for 1 hour. The tissue residue is removed using a strainer, a mesh, a filter and the like, whereby mesenchymal stem cells can be acquired. Mesenchymal stem cells can also be acquired by washing the surface of a third molar (wisdom tooth) extracted with PBS and the like, thereafter cutting the joint of the cement and the enamel to expose the pulp, carefully separating the dental pulp tissue from the dental crown and root, and treating the dental pulp tissue with a protease as described above, thereafter removing the tissue residue.

As methods of mesenchymal stem cell separation other than those described above, methods can be mentioned wherein mesenchymal stem cells are isolated using a surface antigen expressed in mesenchymal stem cells or a reporter vector having a promoter and enhancer of a gene that is specific for mesenchymal stem cells. Specifically, a method of isolating stem cells isolation using the AC133 antigen (US6468794), methods of isolating mesenchymal stem cells using a reporter vector having a promoter and enhancer of the Sox gene (US2002/0135539), the Nestin gene or the Musashi gene (JP-A-2002-034580), and the like can be mentioned.

Stem cells can also be separated using a method wherein FACS fractionation with the Hoechst33342 efflux activity as an index is used to concentrate stem cells in a side population (SP) [Journal of Experimental Medicine, 183, 1797-806 (1996)]. A method wherein SH2-positive, SH4-positive, CD29-positive, CD44-positive, CD71-positive, CD90-positive, CD106-positive, CD120a-positive, CD124-positive, CD14-negative, CD34-negative, and CD45-negative cells as mesenchymal stem cells are separated using a cell sorter or magnetic beads [Science, 284, 143-147 (1999)] can also be used.

As examples of the medium used to culture mesenchymal stem cells, media for cell culture described in Soshiki Baiyou No Gijyutsu, Kiso-hen, 3rd edition, Asakura Shoten (1996) and the like can be mentioned; media for cell culture such as α-MEM, DMEM, and IMDM, supplemented with 1 to 20% of a serum such as bovine or human serum, are preferable. Although the culture conditions may be any conditions that allow cultivation of the cells, culturing temperature is preferably 33 to 37°C, and the cultivation is preferably performed in an incubator filled with 5 to 10% gaseous CO₂. Mesenchymal stem cells are preferably proliferated in adhesion to a plastic culture dish for ordinary tissue culture. When the cells have proliferated over the entire surface of the culture dish, the medium is removed, and a trypsin-EDTA solution is added to suspend the cells. The cells suspended are washed with PBS or a medium for cell culture, after which the cells are 2 fold to 20 fold diluted with a medium for cell culture, and plated to a new culture dish, whereby further subculture can be performed.

### (1-2) Acquirement of small RNA

Total RNA is extracted from mesenchymal stem cells acquired by the various methods described above, and using the RNA, a small RNA comprising a micro-RNA expressed in mesenchymal stem cells can be acquired as described below.

As a method of acquiring a small RNA, specifically, a method wherein separation and cutting out of a small RNA by 15% polyacrylamide gel electrophoresis, 5' terminal dephosphorylation, 3'-adapter ligation, phosphorylation, 5'-adapter ligation, reverse transcription, PCR amplification, concatemerization, and ligation to a vector are performed sequentially, thereafter the small RNA is cloned, and the nucleotide sequence of the clone is determined, as described in Genes & Development 15, 188-200 (2000), and the like can be mentioned. Alternatively, for example, a method wherein separation and cutting off of a small RNA by 15% polyacrylamide gel electrophoresis, 5'-adenylation 3'-adapter ligation, 5'-adapter ligation, reverse transcription, PCR amplification, concatemerization, and ligation to a vector are performed sequentially, thereafter the small RNA is cloned, and the nucleotide sequence of the clone is determined, as described in Science 294, 858-862 (2001), and the like can be mentioned.

Alternatively, separation and cutting off of a small RNA by 15% polyacrylamide gel electrophoresis, 5' terminal dephosphorylation, 3'-adapter ligation, phosphorylation, 5'-adapter ligation, reverse transcription, PCR amplification, and ligation to a microbead vector are performed sequentially, thereafter the small RNA is cloned, and the nucleotide sequence of the microbeads is read to determine the nucleotide sequence, whereby the small RNA can also be acquired, as described in Nucleic Acids Research 34, 1765-1771 (2006).

A small RNA can also be acquired using a small RNA Cloning Kit (manufactured by Takara Bio Inc.).

### (1-3) Identification of micro-RNA

Whether or not the small RNA sequence acquired is a micro-RNA can be determined on the basis of whether or not the criteria described in RNA, 9, 277-279 (2003) are met. For example, in cases where the small RNA was acquired and the nucleotide sequence thereof was determined by a method described above, this can be performed as described below.

Specifically, a surrounding genome sequence wherein a DNA sequence corresponding to the nucleotide sequence of the small RNA acquired is extended by about 50 nt toward the 5' terminal side and the 3' terminal side, respectively, is acquired, and the secondary structure of the RNA expected to be transcribed from the genome sequence is predicted. If the result shows that a hairpin structure is present and the nucleotide sequence of the small RNA is located in one chain of the hairpin, the small RNA can be judged to be a micro-RNA. Genome sequences are open to the general public, and are available from, for example, UCSC Genome Bioinformatics (http://genome.ucsc.edu/). For prediction of secondary structures, various programs are open; for example, RNAfold [Nucleic Acids Research 31, 3429-3431 (2003)], Mfold [Nucleic Acids Research 31, 3406-3415 (2003)] and the like can be used. Existing micro-RNA sequences are registered in a database called miRBase (1157097436265_13); whether or not a micro-RNA is identical to an existing micro-RNA can be determined on the basis of whether or not the sequence thereof is identical to one of the sequences listed therein.

As examples of micro-RNAs thus identified from mesenchymal stem cells, nucleic acids having the nucleotide sequence of any one of SEQ ID NO:1 to 29 and 78 to 80 and the like can be mentioned.
A genome sequence corresponding to the micro-RNA identified and the genome sequence of another organism may be compared, and a nucleic acid having a nucleotide sequence having an identity of 60% or more to the nucleotide sequence of any one of SEQ ID NO:1 to 29 and 78 to 80, preferably a nucleic acid having a nucleotide sequence having an identify of 80% or more, more preferably 90% or more, and still more preferably 95% or more, can be identified as a micro-RNA in the organism.

### (1-4) Identification of micro-RNA precursor

On the basis of the nucleotide sequence of the micro-RNA identified in (1-3) above, a genome sequence comprising a micro-RNA can be identified as a micro-RNA precursor. As examples of a micro-RNA precursor derived from mesenchymal stem cells of the present invention, a nucleic acid having the nucleotide sequence of any one of SEQ ID NO:30 to 73 and 81 to 83 and the like can be mentioned.

Furthermore, a genome sequence corresponding to the micro-RNA precursor identified and the genome sequence of another organism may be compared, and a nucleic acid having a nucleotide sequence having an identity of 60% or more to the nucleotide sequence of any one of SEQ ID NO:30 to 73 and 81 to 83, preferably a nucleic acid having a nucleotide sequence having an identify of 80% or more, more preferably 90% or more, and most preferably 95% or more, can be identified as a micro-RNA precursor in the organism.

### 2. Synthesis of nucleic acid

After a micro-RNA and a micro-RNA precursor are once identified and have the nucleotide sequences thereof determined, as described in 1 above, not only an RNA, which is a polymer of a ribonucleotide, but also a DNA, which is a polymer of a deoxyribonucleotide, can be synthesized on the basis of the nucleotide sequences. For example, on the basis of the nucleotide sequence of the RNA identified in 1 above, the nucleotide sequence of a DNA can be determined. The nucleotide sequence of a DNA corresponding to the nucleotide sequence of an RNA can be determined, without exception, by reading the U (uracil) contained in the sequence of the RNA as T (thymine). A polymer being a mixture of a ribonucleotide and a deoxyribonucleotide and a polymer comprising a nucleotide analogue can also be synthesized in the same manner.

The method of synthesizing a nucleic acid, micro-RNA or micro-RNA precursor of the present invention is not particularly limited; the same can be produced by a method using a publicly known chemical synthesis, or an enzymatic transcription method and the like. As methods using a publicly known chemical synthesis, the phosphoroamidite method, the phosphorothioate method, the phosphotriester method, the CEM method [Nucleic Acid Research, 35, 3287 (2007)] and the like can be mentioned; for example, a nucleic acid, micro-RNA or micro-RNA precursor of the present invention can be synthesized using the ABI3900 high throughput nucleic acid synthesizer (manufactured by Applied Biosystems). As an enzymatic transcription method, transcription with a plasmid or DNA having a desired nucleotide sequence as the template using a typical phage RNA polymerase, for example, T7, T3, or SP6RNA polymerase, can be mentioned.

### 3. Method of detecting functions of micro-RNA and precursor of the micro-RNA

A micro-RNA is produced via processing of a micro-RNA precursor having a hairpin structure by a protein called Dicer, a kind of RNaseIII endonuclease, in cytoplasm, and suppresses the translation of an mRNA having a target nucleotide sequence. Therefore, whether or not the nucleic acid obtained is a micro-RNA can be determined on the basis of whether or not the function is present.

For example, a function can be measured on the basis of whether or not a single-stranded RNA is processed by RNaseIII endonuclease. Specifically, a single-stranded RNA whose function is to be detected is reacted with RNaseIII endonuclease, and the reaction product is electrophoresed; if a function as a micro-RNA precursor is possessed, a band about 20 to 25 nucleotides long resulting from the processing will be detected, whereby the RNA is judged to possess a function as a micro-RNA. Although the RNaseIII endonuclease is not particularly limited, as far as it possesses an activity to process a micro-RNA precursor, it is preferable to use a Dicer protein. Specifically, a Dicer protein is available in si-RNAse III^{™} (manufactured by Takara Bio Inc.), Cold Shock-DICER (manufactured by Takara Bio Inc.), Recombinant Dicer Enzyme (manufactured by Stratagene), BLOCK-iT Dicer RNAi Transfection Kit (manufactured by Invitrogen), X-treme GENE siRNA Dicer Kit (manufactured by Roche-Applied Science) and the like, and the reaction can be carried out according to the conditions noted therein.

As another method of detecting a function of a micro-RNA, a method can be mentioned wherein the function is measured on the basis of whether or not the translation of a mRNA having a target nucleotide sequence is suppressed.
Micro-RNAs are known to suppress the translation of an mRNA comprising a target nucleotide sequence thereof in the untranslated region on the 3' side (3'UTR) [Current Biology, 15, R458-R460 (2005)]. Hence, a DNA wherein a target nucleotide sequence for the single-stranded RNA to be measured is inserted into the 3'UTR of an appropriate reporter gene expression vector is prepared and introduced into a host cell suitable for the expression vector, and the expression of the reporter gene is measured when the cell is allowed to express the single-stranded RNA, whereby whether or not a function of a micro-RNA is possessed can be determined.

The reporter gene expression vector may be any one, as far as it has a promoter upstream of a reporter gene, and is capable of expressing the reporter gene in the host cell. Any reporter gene can be used; for example, the firefly luciferase gene, the Renilla luciferase gene, the chloramphenicol acetyltransferase gene, the β-glucuronidase gene, the β-galactosidase gene, the β-lactamase gene, the aequorin gene, the green fluorescent protein gene, the DsRed fluorescent gene and the like can be utilized. As examples of reporter gene expression vectors having these properties, psiCHECK-1 (manufactured by Promega), psiCHECK-2 (manufactured by Promega), pGL3-Control (manufactured by Promega), pGL4 (manufactured by Promega), pRNAi-GL (manufactured by Takara Bio Inc.), pCMV-DsRed-Express (manufactured by CLONTECH) and the like can be mentioned. A single-stranded RNA can be expressed by the method described in 6 below.

Specifically, detection can be achieved as described below. First, host cells are cultured on a multi-well plate and the like, and a reporter gene expression vector having a target sequence and a single-stranded RNA are expressed. Thereafter, reporter activity is measured both when the single-stranded RNA is and is not expressed, based on which the function of the micro-RNA can be detected.

### 4. Method of detecting the expression of micro-RNA and micro-RNA precursor using a nucleic acid, micro-RNA or micro-RNA precursor of the present invention

Hereinafter, methods of detecting the expression of a micro-RNA and a precursor thereof using a nucleic acid, micro-RNA or micro-RNA precursor of the present invention are described.

As examples of methods of detecting the expression levels of a micro-RNA and a precursor thereof, (1) Northern hybridization, (2) dot blot hybridization, (3) in situ hybridization, (4) quantitative PCR, (5) differential hybridization, (6) microarray, (7) ribonuclease protection assay and the like can be mentioned.

The Northern blot method is a method wherein a sample-derived RNA is separated by gel electrophoresis, then transferred to a solid support such as a nylon filter, and an appropriately-labeled probe is prepared on the basis of the nucleotide sequence of a nucleic acid, micro-RNA or micro-RNA precursor of the present invention, and hybridization and washing are performed, whereby a band specifically bound to the nucleic acid, micro-RNA or micro-RNA precursor of the present invention is detected; specifically, for example, this method can be performed as described in Science 294, 853-858 (2001) and the like.

A labeled probe can be prepared by incorporating a radioisotope, biotin, digoxigenin, a fluorescent group, a chemiluminescent group and the like in a DNA, RNA, or LNA having a sequence complementary to the nucleotide sequence of a nucleic acid, micro-RNA or micro-RNA precursor of the present invention by a method, for example, nick translation, random priming or 5'-terminal phosphorylation. Because the amount of labeled probe bound reflects the expression level of a nucleic acid, micro-RNA or micro-RNA precursor of the present invention, the expression level of a nucleic acid, micro-RNA or micro-RNA precursor of the present invention can be quantified by quantifying the amount of labeled probe bound. Electrophoresis, membrane transfer; probe preparation, hybridization, and nucleic acid detection can be achieved by a method described in Molecular Cloning, 3rd edition.

Dot blot hybridization is a method wherein an RNA extracted from a tissue or a cell is spotted in dot forms and immobilized on a membrane, and hybridized with a labeled polynucleotide to be a probe, and an RNA that specifically hybridizes with the probe is detected. The probe used may be the same as that used for Northern hybridization. RNA preparation, RNA spotting, hybridization, and RNA detection can be achieved by a method described in Molecular Cloning, 3rd edition.

In situ hybridization is a method wherein a paraffin-embedded or cryostat-treated section of a tissue acquired from a living organism, or a cell fixed, is used as a sample and subjected to steps for hybridization with a labeled probe and washing, and the distribution and localization of a nucleic acid, micro-RNA or micro-RNA precursor of the present invention in the tissue or cell are examined by microscopic examination [Methods in Enzymology, 254, 419 (1995)]. The probe used may be the same as that used for Northern hybridization. Specifically, a micro-RNA can be detected in accordance with a method described in Nature Method 3, 27 (2006).

In quantitative PCR, a cDNA synthesized from a sample-derived RNA using a primer for reverse transcription and a reverse transcriptase (hereunder, this cDNA is referred to as a sample-derived cDNA) is used for the measurement. As a primer for reverse transcription to be supplied for cDNA synthesis, a random primer or a specific RT primer and the like can be used. A specific RT primer refers to a primer having a sequence complementary to a nucleotide sequence corresponding to a nucleic acid, micro-RNA or micro-RNA precursor of the present invention and a genome sequence therearound.

For example, a sample-derived cDNA is synthesized, after which a PCR is performed with this cDNA as the template, using a template-specific primer designed from a nucleotide sequence corresponding to a nucleic acid, micro-RNA or micro-RNA precursor of the present invention and a genome sequence therearound, or from a nucleotide sequence corresponding to a primer for reverse transcription, to amplify a cDNA fragment containing the nucleic acid, micro-RNA or micro-RNA precursor of the present invention, and the amount of the nucleic acid, micro-RNA or micro-RNA precursor of the present invention contained in the sample-derived RNA is detected from the number of cycles for reach to a given amount of the fragment. As the template-specific primer, an appropriate region corresponding to a nucleic acid, micro-RNA or micro-RNA precursor of the present invention and a genome sequence therearound is selected, and a pair of a DNA or LNA consisting of a sequence of 20 to 40 nucleotides at the 5' terminus of the nucleotide sequence of the region, and a DNA or LNA consisting of a sequence complementary to a sequence of 20 to 40 nucleotides at the 3' terminus can be used. Specifically, this can be performed in accordance with a method described in Nucleic Acids Research, 32, e43 (2004) and the like.

Alternatively, as the primer for reverse transcription to be supplied for cDNA synthesis, a specific RT primer having a stem-loop structure can also be used. Specifically, this can be performed using a method described in Nucleic Acid Research, 33, e179 (2005), or TaqMan MicroRNA Assays (manufactured by Applied Biosystems).
As still another method of synthesizing a sample-derived cDNA, a polyA sequence is added to a sample-derived RNA by means of polyA polymerase, and a nucleotide sequence comprising an oligodT sequence is used as a primer for reverse transcription, whereby a reverse transcription reaction can be performed. Specifically, this can be performed using the miScript System (manufactured by QIAGEN) or the QuantiMir RT Kit (manufactured by System Biosciences).
Furthermore, by hybridizing a sample-derived RNA or cDNA to a DNA or LNA corresponding to a nucleotide sequence comprising at least one or more of a nucleic acid, micro-RNA and micro-RNA precursor of the present invention immobilized on a substrate such as a filter, glass slide, or silicone having, and performing washing, a change in the amount of the nucleic acid, micro-RNA or micro-RNA precursor of the present invention can be detected. As such methods based on hybridization, methods using differential hybridization [Trends Genet., 7, 314 (1991)] or a microarray [Genome Res., 6, 639 (1996)] can be mentioned. Both methods enable accurate detection of a difference in the amount of a nucleic acid of the present invention between a control sample and a target sample. The internal control, such as a nucleotide sequence corresponding to U6RNA, are immobilized on a filter or a substrate. Also, by synthesizing labeled cDNAs using differently labeled dNTPs (mixtures of dATP, dGTP, dCTP, and dTTP) on the basis of RNAs derived from a control sample and a target sample, and simultaneously hybridizing the two labeled cDNAs to a single filter or a single substrate, accurate quantitation of a nucleic acid, micro-RNA or micro-RNA precursor of the present invention can be performed. Furthermore, quantitation of a nucleic acid, micro-RNA or micro-RNA precursor of the present invention can also be performed by directly labeling and hybridizing an RNA derived from a control sample and/or a target sample. For example, a micro-RNA can be detected using a microarray described in Proc.Natl.Acad.Sci. USA, 101, 9740-9744 (2004), Nucleic Acid Research, 32, e188 (2004), RNA, 13, 151-159 (2007) and the like. Specifically, a micro-RNA can be detected or quantified in the same manner as mirVana miRNA Bioarray (manufactured by Ambion).

In ribonuclease protection assay, first, a promoter sequence such as the T7 promoter or the SP6 promoter is bound to the 3' terminus of a nucleotide sequence corresponding to a nucleic acid, micro-RNA or micro-RNA precursor of the present invention or a genome sequence therearound, and a labeled antisense RNA is synthesized with an in vitro transcription system using a labeled NTP (a mixture of ATP, GTP, CTP, and UTP) and an RNA polymerase. The labeled antisense RNA is bound to a sample-derived RNA to form an RNA-RNA hybrid, after which the hybrid is digested with ribonuclease A, which degrades single-stranded RNAs only. The digest is subjected to gel electrophoresis to detect or quantify an RNA fragment protected against the digestion by forming the RNA-RNA hybrid, as a nucleic acid, micro-RNA or micro-RNA precursor of the present invention. Specifically, the fragment can be detected or quantified using the mirVana miRNA Detection Kit (manufactured by Ambion).

### 5. Method of detecting mutations of micro-RNA and precursor of the micro-RNA precursor using a nucleic acid, micro-RNA or micro-RNA precursor of the present invention

Hereinafter, methods of detecting mutations of a micro-RNA and a precursor thereof using a nucleic acid, micro-RNA or micro-RNA precursor of the present invention are described.

As a method of detecting mutations of a micro-RNA and micro-RNA precursor, a method can be used wherein a hetero-double-strand formed by hybridization of the normal form of the micro-RNA and a mutated form of the micro-RNA, or of the normal form of the micro-RNA precursor and a mutated form of the micro-RNA precursor, is detected.
As methods of detecting a hetero-double-strand, (1) detection of a hetero-double-strand by polyacrylamide gel electrophoresis [Trends genet., 7, 5 (1991)], (2) single-strand conformation polymorphism analysis [Genomics, 16, 325-332 (1993)], (3) chemical cleavage of mismatches (CCM) [Human Genetics (1996), Tom Strachan and Andrew P. Read, BIOS Scientific Publishers Limited], (4) enzymatic cleavage of mismatches [Nature Genetics, 9, 103-104 (1996)], (5) denatured gel electrophoresis [Mutat. Res., 288, 103-112 (1993)] and the like can be mentioned.

Detection of a hetero-double-strand by polyacrylamide gel electrophoresis is, for example, performed as described below. First, with a sample-derived DNA or a sample-derived cDNA as the template, and using a primer designed on the basis of a genome nucleotide sequence comprising the nucleotide sequence of a nucleic acid, micro-RNA or micro-RNA precursor of the present invention, a fragment smaller than 200 bp is amplified. Hetero-double-strands, if formed, are slower in mobility than mutation-free homo-double-strands, and can be detected as extra bands. Better separation is achieved using a custom-made gel (Hydro-link, MDE and the like). In the case of search for a fragment smaller than 200 bp, insertions, deletions, and most single-nucleotide substitutions can be detected. It is desirable that hetero-double-strand analysis be performed using a single gel in combination with the single strand conformation analysis described below.

In single strand conformation polymorphism analysis (SSCP analysis), a DNA amplified as a fragment smaller than 200 bp with a sample-derived DNA or sample-derived cDNA as the template, using a primer designed on the basis of a genome nucleotide sequence comprising the nucleotide sequence of a nucleic acid, micro-RNA or micro-RNA precursor of the present invention, is denatured, after which it is electrophoresed in non-denatured polyacrylamide gel. By labeling the primer with an isotope or a fluorescent dye at the time of DNA amplification, or by silver-staining the non-labeled amplification product, the amplified DNA can be detected as a band. To clarify a difference from the wild type pattern, a control sample may be electrophoresed simultaneously, whereby a fragment with a mutation can be detected on the basis of a difference in mobility.

In chemical cleavage of mismatches (CCM method), a DNA fragment amplified with a sample-derived DNA or sample-derived cDNA as the template, using a primer designed on the basis of a genome nucleotide sequence comprising the nucleotide sequence of a nucleic acid, micro-RNA or micro-RNA precursor of the present invention, is hybridized to a labeled nucleic acid prepared by allowing a nucleic acid, micro-RNA or micro-RNA precursor of the present invention to incorporate an isotope or a fluorescent target, and treated with osmium tetraoxide to cleave one strand of the DNA at the mismatched portion, whereby a mutation can be detected. CCM is one of the most sensitive methods of detection, and can be applied to samples of kilobase length.

In place of osmium tetraoxide used above, T4 phage resolvase and an enzyme involved in mismatch repair in cells, such as endonuclease VII, and RNaseA may be used in combination to enzymatically cleave a mismatch.
In denaturing gradient gel electrophoresis (DGGE method), a DNA amplified with a sample-derived DNA or sample-derived cDNA as the template, using a primer designed on the basis of a genome nucleotide sequence comprising the nucleotide sequence of a nucleic acid, micro-RNA or micro-RNA precursor of the present invention, is electrophoresed using a gel having a chemical denaturant density gradient or a temperature gradient. The DNA fragment amplified migrates in the gel to a position where it denatures to a single strand, and no longer migrates after the denaturation. Because the migration of the amplified DNA in the gel differs between the presence and absence of a mutation, the presence of the mutation can be detected. To increase the detection sensitivity, the addition of a poly (G:C) end to each primer is effective.

By directly determining and analyzing the nucleotide sequence of a sample-derived DNA or sample-derived cDNA, a mutation of a nucleic acid, micro-RNA or micro-RNA precursor of the present invention can also be detected.

### 6. Method of expressing a nucleic acid, micro-RNA or micro-RNA precursor of the present invention

A nucleic acid, micro-RNA or micro-RNA precursor of the present invention can be expressed by using a vector such that a nucleic acid, micro-RNA or micro-RNA precursor of the present invention is transcribed and hence biosynthesized when the vector is introduced into a cell. Specifically, by preparing a DNA fragment comprising a hairpin portion on the basis of the nucleotide sequence of a nucleic acid, a micro-RNA or micro-RNA precursor of the present invention, or a genome nucleotide sequence comprising the foregoing nucleotide sequence, and inserting the fragment downstream of a promoter in the expression vector to construct an expression plasmids, and then introducing the expression plasmid into a host cell suitable for the expression vector, whereby a nucleic acid, micro-RNA or micro-RNA precursor of the present invention can be expressed.

As an expression vector, one capable of self-replication in the host cell, and capable of being incorporated in the chromosome, that comprises a promoter at a position enabling the transcription of a genomic gene comprising the nucleotide sequence of a nucleic acid, micro-RNA or micro-RNA precursor of the present invention is used. The promoter may be any one, as far as it is capable of expressing in the host cell; for example, a RNA polymerase II (pol II) system promoter, a RNA polymerase III (pol III) system promoter being a U6RNA and H1RNA transcription system and the like can be mentioned. As examples of pol II system promoters, the promoter of the cytomegalovirus (human CMV) IE (immediate early) gene, the early promoter of SV40 and the like can be mentioned. As examples of expression vectors using them, pCDNA6.2-GW/miR (manufactured by Invitrogen), pSilencer 4.1-CMV (manufactured by Ambion) and the like can be mentioned. As pol III system promoters, U6RNA, H1RNA or tRNA promoters can be mentioned. As examples of expression vectors using them, pSINsi-hH1 DNA (manufactured by Takara Bio Inc.), pSINsi-hU6 DNA (manufactured by Takara Bio Inc.), pENTR/U6 (manufactured by Invitrogen) and the like can be mentioned.

Alternatively, by inserting a genomic gene comprising the nucleotide sequence of a nucleic acid, micro-RNA or micro-RNA precursor of the present invention into the downstream of the promoter in a viral vector to construct a recombinant viral vector, and introducing the vector into a packaging cell to produce a recombinant virus, the genomic gene comprising the nucleic acid, micro-RNA or micro-RNA precursor of the present invention can be expressed.

The packaging cell may be any cell, as far as it is capable of supplementing a recombinant viral vector deficient in any one of the genes that encode the proteins necessary for the packaging of the virus with the lacked protein; for example, human kidney-derived HEK293 cells, mouse fibroblasts NIH3T3 and the like can be used. As the protein supplemented by the packaging cell, in the case of a retrovirus vector, proteins derived from a mouse retrovirus, such as gag, pol, and env, can be used; in the case of a lentivirus vector, proteins derived from a HIV virus, such as gag, pol, env, vpr, vpu, vif, tat, rev, and nef, can be used; in the case of an adenovirus vector, proteins derived from an adenovirus, such as E1A and E1B, can be used; in the case of an adeno-associated viral vector, proteins such as Rep (p5, p19, p40) and Vp(Cap), can be used.

In addition to using an expression vector, a nucleic acid, micro-RNA or micro-RNA precursor of the present invention can also be introduced directly into a cell, without using a vector. As the nucleic acid used in this technique, a DNA, an RNA, or a nucleotide analogue, as well as a chimeric molecule thereof, or a derivative of the nucleic acid can also be used. Specifically, in the same way as with Pre-miR^{™} miRNA Precursor Molecules (manufactured by Ambion) or miRIDIAN microRNA Mimics (manufactured by GE Healthcare), a nucleic acid, micro-RNA or micro-RNA precursor of the present invention can be expressed.

### 7. Method of suppressing the expression of micro-RNA and micro-RNA precursor using a nucleic acid, micro-RNA or micro-RNA precursor of the present invention

The expression of a nucleic acid, micro-RNA or micro-RNA precursor of the present invention can be suppressed using an antisense technology [Bioscience and Industry, 50, 322 (1992); Kagaku, 46, 681 (1991), Biotechnology, 9, 358 (1992), Trends in Biotechnology, 10, 87 (1992), Trends in Biotechnology, 10, 152 (1992); Cell Technology, 16, 1463 (1997)], triple helix technology [Trends in Biotechnology, 10, 132 (1992)], ribozyme technology [Current Opinion in Chemical Biology, 3, 274 (1999), FEMS Microbiology Reviews, 23, 257 (1999), Frontiers in Bioscience, 4, D497 (1999), Chemistry & Biology, 6, R33 (1999), Nucleic Acids Research, 26, 5237 (1998), Trends In Biotechnology, 16, 438 (1998)], decoy DNA method [Nippon Rinsho - Japanese Journal of Clinical Medicine, 56, 563 (1998), Circulation Research, 82, 1023 (1998), Experimental Nephrology, 5, 429 (1997), Nippon Rinsho - Japanese Journal of Clinical Medicine, 54, 2583 (1996)], or a siRNA (short interfering RNA).

An antisense refers to one that allows nucleotide sequence-specific hybridization of a nucleic acid having a nucleotide sequence complementary to a certain target nucleic acid to suppress the expression of the target nucleic acid. As the nucleic acid used as the antisense, a DNA, an RNA or a nucleotide analogue, as well as a chimeric molecule thereof, or a derivative of the nucleic acid can also be used. Specifically, by preparing an antisense in accordance with a method described in Nature 432, 226 (2004) and the like, the expression can be suppressed. Specifically, in the same way as with Anti-miR^{™} miRNA Inhibitors (manufactured by Ambion) and miRIDIAN microRNA Inhibitors (manufactured by GE Healthcare), the expression of a nucleic acid, micro-RNA or micro-RNA precursor of the present invention can be suppressed.

An siRNA refers to a short double-stranded RNA comprising the nucleotide sequence of a certain target nucleic acid, that is capable of suppressing the expression of the target nucleic acid by RNA interference (RNAi). The sequence of an siRNA can be designed as appropriate from the target nucleotide sequence on the basis of conditions shown in the literature [Genes Dev., 13, 3191 (1999)]. By synthesizing two RNAs having a 19-nucleotide sequence selected and a complementary sequence, with TT added to the 3' terminus of each thereof using a DNA synthesizer, and performing annealing, an siRNA can be prepared. By inserting a DNA corresponding to the above-described 19-nucleotide sequence selected into an siRNA expression vector such as pSilencer 1.0-U6 (manufactured by Ambion) or pSUPER (OligoEngine), a vector that expresses an siRNA capable of suppressing the expression of the gene can be prepared.

For example, using an antisense or siRNA specific for a micro-RNA expressed in mesenchymal stem cells, mast cells or cancer cells, or a precursor of the micro-RNA, the expression of a micro-RNA expressed in mesenchymal stem cells, mast cells or cancer cells or a precursor of the micro-RNA, can be suppressed. Specifically, by administering an antisense or siRNA specific for the micro-RNA or the micro-RNA precursor, the expression of the micro-RNA can be suppressed, and the action of the micro-RNA or micro-RNA precursor in mesenchymal stem cells, mast cells or cancer cells can be controlled.

For example, in the case of a patient affected by an abnormality of the expression of a micro-RNA expressed in mesenchymal stem cells, mast cells or cancer cells or a precursor thereof, by administering an antisense or siRNA specific for the micro-RNA or precursor thereof to the patient, it is possible to control a function of mesenchymal stem cells, mast cells or cancer cells to treat a disease that develops as a result of the above-described expression abnormality. Hence, an antisense or siRNA that is specific for the micro-RNA or precursor thereof is useful as a therapeutic agent for a disease caused by an abnormality of mesenchymal stem cells or mast cells, or a disease caused by a cell proliferation abnormality.

When an antisense or siRNA that is specific for the micro-RNA or precursor thereof is used as the above-described therapeutic agent, the antisense or siRNA, alone or after being inserted into an appropriate vector such as a retrovirus vector, adenovirus vector, or adeno-associated viral vector, can be administered in the form of a pharmaceutical preparation prepared by the conventional methods described in 10 and 11 below.

### 8. Method of suppressing a gene function using a nucleic acid, micro-RNA or micro-RNA precursor of the present invention

The method of suppressing a function of a gene using a nucleic acid, micro-RNA or micro-RNA precursor of the present invention may be any method, as far as a function of a gene having a target sequence is suppressed by means of the activity of the micro-RNA to suppress the translation of an mRNA having the target nucleotide sequence; for example, a method can be mentioned wherein a nucleic acid, micro-RNA or micro-RNA precursor of the present invention is expressed to increase the amount of micro-RNA in the cell, whereby a function of an mRNA having a target sequence is suppressed to suppress a function of the gene. A nucleic acid, micro-RNA or micro-RNA precursor of the present invention can be expressed by the method described in 6 above. As examples of an mRNA having a target nucleotide sequence of a micro-RNA consisting of the nucleotide sequence of any one of SEQ ID NO:1 to 29 and 78 to 80, the above-described genes shown in Tables 1 to 14 can be mentioned.

### 9. Method of separating cells using a nucleic acid, micro-RNA or micro-RNA precursor of the present invention

As a method of separating cells that express a nucleic acid, micro-RNA or micro-RNA precursor of the present invention from various cells taken out from a living organism, a probe prepared by fluorescently labeling a nucleic acid having a nucleotide sequence complementary to the nucleotide sequence of a nucleic acid, micro-RNA or micro-RNA precursor of the present invention is introduced to a cell to hybridize the same to the nucleic acid, micro-RNA or micro-RNA precursor of the present invention, and only the cells that have hybridized with the labeled probe are separated using a flow cytometer with a sorting function.

The fluorescently labeled probe may be any one, as far as it emits specific fluorescence upon hybridization; for example, a molecular beacon [Biochimica et Biophysica Acta 1479, 178 (1998)], FRET [Proc.Natl.Acad.Sci. USA 103, 263 (2006)] and the like can be mentioned.
A molecular beacon is a nucleic acid wherein a fluorescent functional group has been introduced via a covalent bond at one end thereof, and a dabsyl group and the like that cause fluorescence quenching has been introduced at the other end, and the nucleotide sequence thereof is designed to assume a hairpin structure in an ordinary aqueous solution. Because the fluorescent functional group and the fluorescence quenching group introduced to both ends are adjacent to each other, no fluorescence is observed with the probe alone, but if the probe is hybridized with a nucleic acid, micro-RNA or micro-RNA precursor of the present invention, the fluorescent functional group and the fluorescence quenching group are disjoined, and intense fluorescence is observable.

FRET is the phenomenon in which molecule excitation energy transfer occurs between two kinds or more of fluorescent functional groups. Specifically, two nucleic acid probes are provided: a nucleic acid probe incorporating an energy donor at one end thereof and another nucleic acid probe incorporating an energy receptor at the other end. If the nucleotide sequences of the two probes are designed to allow the two fluorescent functional groups, introduced after hybridization with a nucleic acid, micro-RNA or micro-RNA precursor of the present invention, to come into close contact with each other, only emission from the donor is observed with the probe alone, but if the probes are hybridized with a nucleic acid, micro-RNA or micro-RNA precursor of the present invention, the two probes come in close contact with each other, the energy of the donor transfers to the acceptor, and emission based on the acceptor is mainly observable.

As methods of cell separation using a flow cytometer with a sorting function, the water charge method, the cell capture method and the like can be mentioned (Flow Cytometer Jiyu Jizai, p14-23, SHUJUNSHA, 1999). In both methods, cell fluorometry is performed and fluorescence intensity is converted to electrical signals to quantify fluorescence intensity, whereby cells can be separated according to the amount quantified. Specifically, fluorescence intensity can be measured using the BD FACSAria cell sorter (manufactured by Becton Dickinson Immunocytometry Systems), EPICS ALTRA HyPerSort (manufactured by Beckman Coulter, K.K.) and the like.

### 10. Method of screening for a substance that promotes or suppresses the expression or a function of micro-RNA and precursor of the micro-RNA using a nucleic acid, micro-RNA or micro-RNA precursor of the present invention

Using a nucleic acid, micro-RNA or micro-RNA precursor of the present invention, a substance that promotes or suppresses the expression or a function of a micro-RNA or a precursor thereof can be screened for. For example, from among the nucleotide sequences of micro-RNAs and micro-RNA precursors of the present invention, a nucleotide sequence to be targeted for the screening is chosen, and by means of a cell that expresses a nucleic acid having the nucleotide sequence, a substance that promotes or suppresses the expression or a function of the chosen micro-RNA or precursor thereof can be screened for.

As cells that express a nucleic acid having the nucleotide sequence of a micro-RNA or micro-RNA precursor, used for screening, mesenchymal stem cells, mast cells or cancer cells, as well as transformant cells obtained by introducing a vector that expresses a nucleic acid having the nucleotide sequence into a host cell such as an animal cell or yeast, cells incorporating a nucleic acid having the nucleotide sequence introduced directly without using a vector and the like as described in 6 above can also be used.

As specific methods of screening, (a) a method wherein a change in the expression level of a micro-RNA or precursor thereof being a target for screening is used as an index, as well as (b) a method wherein a change in the expression level of an mRNA having a target sequence of a micro-RNA or precursor thereof being a target for screening is used as an index, since a micro-RNA suppresses the translation of an mRNA having a target sequence, can be mentioned.
(a) Screening method wherein a change in the expression level of a micro-RNA or precursor thereof being a target for screening is used as an index
   A test substance is brought into contact with a cell that expresses a nucleic acid having the nucleotide sequence, and with a change in the expression level of the nucleic acid o selected as an index, a substance that promotes or suppresses the expression of a micro-RNA and precursor thereof is obtained. The expression level of a nucleic acid can be detected by the method described in 3 above.
(b) Screening method wherein a change in the expression level of an mRNA having a target sequence of a micro-RNA or precursor thereof being a target for screening is used as an index
   A test substance is brought into contact with a cell that expresses a nucleic acid having the nucleotide sequence, and with a change in the expression level of an mRNA having a target sequence of the nucleic acid selected as an index, a substance that promotes or suppresses the expression or a function of a micro-RNA and a precursor thereof is obtained. Alternatively, a DNA incorporating a target sequence for a single-stranded RNA having a nucleotide sequence of the present invention introduced into the 3' UTR of an appropriate reporter gene expression vector is prepared and introduced into a host cell suitable for the expression vector, a test substance is brought into contact with the cell, and with a change in the expression level of the reporter gene as an index, a substance that promotes or suppresses the expression or a function of a micro-RNA and precursor thereof is obtained.

Choice of a target sequence can be achieved by the method described in 8 above; as examples of an mRNA having a target sequence of a micro-RNA consisting of the nucleotide sequence of any one of SEQ ID NO:1 to 29, the above-described genes shown in Tables 1 to 14 above can be exemplified.

### 11. Diagnostic reagent and therapeutic drug comprising a nucleic acid, micro-RNA or micro-RNA precursor of the present invention

Because a nucleic acid, micro-RNA or micro-RNA precursor of the present invention and a nucleic acid, micro-RNA or micro-RNA precursor having a nucleotide sequence complementary to the nucleotide sequence thereof suppresses the expression of a nucleic acid, micro-RNA or micro-RNA precursor of the present invention, they can be utilized as therapeutic drugs for a disease caused by an abnormality of mesenchymal stem cells or mast cells, or a disease caused by a cell proliferation abnormality. A nucleic acid, micro-RNA or micro-RNA precursor of the present invention can also be utilized as a diagnostic reagent for a disease caused by an abnormality of mesenchymal stem cells or mast cells, or a disease caused by a cell proliferation abnormality, by quantifying a nucleic acid, micro-RNA or micro-RNA precursor of the present invention, or detecting a mutation of a nucleic acid, micro-RNA or micro-RNA precursor of the present invention. As an abnormality of mesenchymal stem cells, an abnormality of proliferation or differentiation and the like can be mentioned; as diseases caused thereby, cancer, dysosteogenesis, achondroplasia, diabetes and the like can be mentioned. As mast cell abnormalities, abnormalities of mast cell differentiation and degranulation, inflammatory mediator production, cytokine production, chemokine production and the like can be mentioned; as diseases caused thereby, atopic dermatitis, asthma, chronic obstructive lung disease, allergic disease and the like can be mentioned. As diseases caused by a cell proliferation abnormality, cancers and diseases caused by abnormal proliferation of cells, tissue hyperplasia and the like, such as arteriosclerosis, rheumatoid arthritis, prostatic hyperplasia, blood vessel restenosis after percutaneous transvascular coronary angioplasty, fibroid lung, glomerulonephritis, and autoimmune diseases can be mentioned.

A diagnostic reagent comprising a nucleic acid, micro-RNA or micro-RNA precursor of the present invention, according to the desired diagnostic method, may comprise reagents necessary for quantitation or detection of mutation of a nucleic acid, micro-RNA or micro-RNA precursor of the present invention, for example, buffering agents, salts, reaction enzymes, labeled proteins that bind to a nucleic acid, micro-RNA or micro-RNA precursor of the present invention, and a color developer for detection and the like.

Although a therapeutic agent containing a nucleic acid, micro-RNA or micro-RNA precursor of the present invention or a nucleic acid, micro-RNA or micro-RNA precursor having a nucleotide sequence complementary to the nucleotide sequence thereof as an active ingredient can be administered alone, the same is normally desirably administered as a pharmaceutical preparation produced by an optionally chosen method known well in the technical field of pharmaceutical making with one or more pharmacologically acceptable carriers blended therein.

The route of administration used is desirably the most effective one in treatment; oral administration, or parenteral administration such as intraoral administration, airway administration, intrarectal administration, subcutaneous administration, intramuscular administration and intravenous administration can be mentioned, and desirably intravenous administration can be mentioned.
As dosage forms, sprays, capsules, tablets, granules, syrups, emulsions, suppositories, injection formulations, ointments, tapes and the like can be mentioned.

As preparations appropriate for oral administration, emulsions, syrups, capsules, tablets, powders, granules and the like can be mentioned.
Liquid preparations like emulsions and syrups can be produced using water, saccharides such as sucrose, sorbitol, and fructose, glycols such as polyethylene glycol and propylene glycol, oils such as sesame oil, olive oil, and soybean oil, antiseptics such as p-hydroxybenzoic acid esters, flavors such as strawberry flavor and peppermint and the like as additives.

Capsules, tablets, powders, granules and the like can be produced using excipients such as lactose, glucose, sucrose, and mannitol, disintegrants such as starch and sodium alginate, lubricants such as magnesium stearate and talc, binders such as polyvinyl alcohol, hydroxypropylcellulose, and gelatin, surfactants such as fatty acid esters, plasticizers such as glycerin and the like as additives.

As appropriate preparations for parenteral administration, injection formulations, suppositories, sprays and the like can be mentioned.
An injection formulation is prepared using a carrier consisting of a salt solution, a glucose solution or a mixture of both and the like. A suppository is prepared using a carrier such as cacao butter, hydrogenated fat or carboxylic acid. A spray is prepared using a carrier that does not stimulate the recipient's oral cavity and airway mucosa, and that disperses the active ingredients as fine particles to facilitate the absorption thereof, and the like.

As examples of the carrier, specifically, lactose, glycerin and the like can be exemplified. Depending on the nature of the nucleic acid, micro-RNA or micro-RNA precursor of the present invention, and of the carrier used, preparations such as aerosols and dry powders are possible. In these parenteral preparations, components exemplified as additives for oral preparations can be added.
The dose or frequency of administration varies depending on desired therapeutic effect, method of administration, duration of treatment, age, body weight and the like, and is normally 10 µg/kg to 20 mg/kg per day for an adult.

A therapeutic drug comprising a nucleic acid, micro-RNA or micro-RNA precursor of the present invention and a nucleic acid, micro-RNA or micro-RNA precursor having a nucleotide sequence complementary to the nucleotide sequence thereof as an active ingredient can also be produced by blending a nucleic acid, micro-RNA or micro-RNA precursor of the present invention or a vector that expresses a nucleic acid, micro-RNA or micro-RNA precursor having a nucleotide sequence complementary to the nucleotide sequence thereof and a nucleotide used in a nucleic acid therapeutic drug [Nature Genet., 8, 42(1994)].

The base used in the nucleic acid therapeutic agent may be any base for ordinary use in injection formulations; distilled water, solutions of salts such as sodium chloride or a mixture of sodium chloride and an inorganic salt, solutions of mannitol, lactose, dextran, and glucose, solutions of amino acids such as glycine and arginine, mixed solutions of organic acid solutions or salt solutions and glucose solution and the like can be mentioned. In accordance with a conventional method, using auxiliary agents such as an osmotic pressure o regulator, a pH regulator, a vegetable oil such as sesame oil or soybean oil, lecithin, and a surfactant such as a non-ionic surfactant in these bases, an injection formulation may be prepared as a solution, suspension, or dispersion. These injection formulations can also be prepared as preparations for dissolution before use, by procedures such as powdering and lyophilization. A therapeutic agent of the present invention can be used for treatment as is in the case of a liquid, or after being dissolved in a base described above, optionally sterilized, in the case of a solid, just before treatment.

As a vector comprising a nucleic acid, micro-RNA or micro-RNA precursor of the present invention, the recombinant viral vector prepared in 6 above can be mentioned, more specifically, retrovirus vector and lentivirus vector and the like can be mentioned.
For example, by combining a nucleic acid, micro-RNA or micro-RNA precursor of the present invention with a polylysine-conjugated antibody that is specific for adenovirus hexon protein to prepare a complex, and binding the complex obtained to an adenovirus vector, a viral vector can be prepared. The viral vector is capable of stably reaching the desired cell, being incorporated into cells by endosome, being decomposed in the cells, and efficiently expressing the nucleic acid.

A viral vector based on Sendai virus, which is a (-) strand RNA virus, has been developed (WO97/16538, WO97/16539), a Sendai virus incorporating a nucleic acid, micro-RNA or micro-RNA precursor of the present invention can be prepared using the Sendai virus.
A nucleic acid, micro-RNA or micro-RNA precursor of the present invention can also be migrated by a non-viral nucleic acid migration method. The same can be migrated by, for example, calcium phosphate co-precipitation [Virology, 52, 456-467 (1973); Science, 209, 1414-1422 (1980)], microinjection method [Proc.Natl.Acad.Sci. USA, 77, 5399-5403 (1980); Proc.Natl.Acad.Sci. USA, 77, 7380-7384 (1980); Cell, 27, 223-231 (1981); Nature, 294, 92-94 (1981)], membrane fusion-mediated migration mediated by liposome [Proc.Natl.Acad.Sci. USA, 84, 7413-7417 (1987); Biochemistry, 28, 9508-9514 (1989); J. Biol. Chem., 264, 12126-12129 (1989); Hum. Gene Ther., 3, 267-275, (1992); Science, 249, 1285-1288 (1990); Circulation, 83, 2007-2011 (1992)] or direct DNA uptake and receptor-mediated DNA migration method [Science, 247, 1465-1468 (1990); J. Biol. Chem., 266, 14338-14342 (1991); Proc.Natl.Acad.Sci. USA, 87, 3655-3659 (1991); J. Biol. Chem., 264, 16985-16987 (1989); BioTechniques, 11, 474-485 (1991); Proc.Natl.Acad.Sci. USA, 87, 3410-3414 (1990); o Proc.Natl.Acad.Sci. USA, 88, 4255-4259 (1991); Proc.Natl.Acad.Sci. USA, 87, 4033-4037 (1990); Proc.Natl.Acad.Sci. USA, 88, 8850-8854 (1991); Hum. Gene Ther., 3, 147-154 (1991)] and the like.

Membrane fusion-mediated migration mediated by liposome allows a nucleic acid, micro-RNA or micro-RNA precursor of the present invention to be incorporated locally in the tissue, and to be expressed, by administering a liposome preparation directly to the target tissue [Hum. Gene Ther., 3, 399 (1992)]. For direct targeting of a DNA to a focus, direct DNA uptake technology is preferable.
For receptor-mediated DNA transfer, for example, a method performed by binding a DNA (usually assuming the form of a covalently cyclized supercoiled plasmid) to a protein ligand via polylysine can be mentioned. A ligand is chosen on the basis of the presence of a corresponding ligand receptor on the cell surface of the desired cell or tissue. The ligand-DNA conjugate can be injected directly into a blood vessel as desired, and can be directed to a target tissue wherein receptor binding and DNA-protein complex internalization occur. To prevent the destruction of the DNA in a cell, an adenovirus may be infected simultaneously to destroy the endosome function.

### 12. Differentiation from mesenchymal stem cells to osteoblasts and method of evaluation

As a method of examining the influence on the process of differentiation from mesenchymal stem cells to osteoblasts, for example, confirmation is made as described below. Specifically, under conditions that induce differentiation from mesenchymal stem cells to osteoblasts, a nucleic acid, micro-RNA micro-RNA precursor or an siRNA for the target gene for the micro-RNA used in the present invention is introduced into mesenchymal stem cells according to the method in 6 above, and cultured. Genes or proteins whose expression increased with the progression of differentiation into osteoblasts are analyzed, and compared with negative control.

As a method of inducing differentiation from mesenchymal stem cells to osteoblasts, any method can be used, as far as it enables induction of differentiation from mesenchymal stem cells to osteoblasts; for example, a method described in Science, 284, 143-147 (1999) can be mentioned. Specifically, by sowing mesenchymal stem cells to an incubator, and thereafter continuing to culture the cells in a medium for cell culture containing dexamethasone, ascorbic acid-diphosphate, and β-glycerophosphate for 1 to 4 weeks, mesenchymal stem cells can be differentiated into osteoblasts.

As quantitative analytical methods for genes whose expression increases as a result of differentiation into osteoblasts, RT-PCR (reverse transcription-polymerase chain reaction), Northern blot analysis, dot blot hybridization, DNA microarray and the like can be mentioned.
As quantitative analytical methods for proteins whose expression increases as a result of differentiation into osteoblasts, Western blot analysis, immunohistological staining using an antibody that specifically reacts on the protein, ELISA and the like can be mentioned.

As genes or proteins whose expression increases as a result of differentiation into osteoblasts, type I collagen, osteocalcin, osteonectin, osteopontin, bone sialoprotein, Runx2 (runt-related gene 2), alkaline phosphatase (ALP) and the like can be mentioned.
As methods of evaluating the degree of differentiation into osteoblasts, staining cells by means of the ALP enzyme activity in the osteoblasts, and a method wherein ALP enzyme activity is measured can be mentioned. More specifically, as a method of such cell staining, a method can be mentioned wherein the alcohol moiety of the phosphoric acid ester of the substrate hydrolyzed by ALP enzyme in osteoblasts is coupled with a diazonium salt, and precipitated with azo dye in the enzyme activity portion. As an example of the substrate, Naphthol AS-MX phosphate can be mentioned; as an example of the azo dye, Fast Violet'Blue can be mentioned. A kit comprising the same, for example, leukocyte alkaline phosphatase (manufactured by Sigma) and the like may be used. As kits for measuring ALP enzyme activity, for example, Alkaline Phospha B-Test Wako (manufactured by Wako Pure Chemical Industries, Ltd.) and the like may be used.

Furthermore, by detecting a calcified component produced by osteoblasts, differentiation into osteoblasts can also be confirmed. As methods of detecting a calcified component, staining methods such as von Kossa staining and Alizarin Red staining can be mentioned.
Von Kossa staining is a method of detecting calcium phosphate, a calcified component, using silver nitrate. Specifically, a 1 to 5% aqueous solution of silver nitrate is reacted with cells fixed with paraffin and the like and exposed to light, and the portion that develops a black color in which calcium phosphate is present is quantified by, for example, measuring the area where the color is developed, whereby the degree of differentiation into osteoblasts can be evaluated.

Alizarin Red staining is a method based on the fact that Alizarin Red S exhibits specific binding to calcium to form a lake. Specifically, 0.01 to 5% Alizarin Red S solution is reacted with cells fixed with paraffin and the like, and the portion that develops a red-purple to orange-red color is quantified by, for example, measuring the area where the color is developed, whereby the degree of differentiation into osteoblasts can be evaluated.

### 13. Method of measuring the degree of mast cell activation

The method of acquiring human mast cells is not particularly limited, as far as it ensures safe and efficient acquirement; for example, human mast cells can be prepared from the human lung, skin, fetal liver and the like by a publicly known method [J. Immunol. Methods, 169, 153 (1994); J. Immunol., 138, 861 (1987); J. Allergy Clin. Immunol., 107, 322 (2001); J. Immunol. Methods., 240, 101 (2000)]. Human mast cells can also be prepared by culturing mononuclear cells prepared from human umbilical blood, peripheral blood, bone marrow, lung or skin in the presence of stem cell factor (hereinafter abbreviated as SCF) to differentiate them into mast cells in accordance with a publicly known method [J. Immunol., 157, 343, (1996); Blood, 91, 187 (1998); J. Allergy Clin. Immunol., 106, 141 (2000); Blood, 97, 1016 (2001); Blood, 98, 1127 (2001); Blood, 100, 3861 (2002); Blood, 97, 2045 (2001)].

A cell line established from a human mast cell can also be used. As a human mast cell line, LAD2, which is known to well retain the nature of human mast cells [Leuk. Res., 27, 671 (2003); Leuk. Res., 27, 677 (2003)] and the like can be mentioned.
As a method of assessing the activation of a mast cell includes, for example, a method of confirming at least one of its actions to suppress activation, suppress degranulation, suppress inflammatory mediator production, suppress cytokine production and suppress chemokine production can be mentioned. Specifically, a nucleic acid, micro-RNA or micro-RNA precursor used in the present invention, or an siRNA for a target gene of the micro-RNA is introduced into a mast cell, and the cell is cultured with the addition of IgE, thereafter human mast cells are activated by the addition of an anti-IgE antibody and the like to measure a released substance such as (i) histamine or β-hexosaminidase, which can be an index of degranulation, (ii) an inflammatory mediator such as LTC4, LTD4, LTE4, or PGD2, (iii) a cytokine such as TNF-α or GM-CSF, or (iv) a chemokine such as IL-8, I-309, or MIP-1α, and comparing the result with that obtained when a nucleic acid, micro RNA or micro-RNA precursor used in the present invention, or an siRNA for a target gene of the micro-RNA, is not introduced. Also, by introducing a nucleic acid, micro RNA or micro-RNA precursor used in the present invention, or an siRNA for a target gene of the micro-RNA, into a mast cell, and detecting the induction of apoptosis by a measurement of the fragmentation of chromatin DNA, the TUNEL method and the like, the fact that the siRNA possesses apoptosis inducing action can be confirmed.

Mast cell activation can also be examined by measuring, in place of degranulation, production of cytokines such as TNF-α and GM-CSF, production of chemokines such as IL-8, I-309, and MIP-1α, production of inflammatory mediators such as LTC4, LTD4, LTE4, and PGD2 and the like [Blood 100, 3861(2002)].

### 14. Method of measuring proliferation of cancer cells and other cells

The method of measuring cell proliferation is not particularly limited, as far as it enables a measurement of an index that reflects cell count or cell proliferation rate. Viable cell counting, DNA synthesis rate measurements, total protein content measurements and the like can be used. As a method of evaluating viable cell counts, a method wherein the ATP content in cells is measured can be mentioned. It is known that the ATP content in cells is proportional to the number of cells in culture (J. Immunol. Meth. 160, 81-88 (1993)). As more specific methods of measuring the ATP content in cells, the MTT method, the XTT method and the like can be mentioned (J. Immunol. Meth. 65, 55-63 (1983)). A method can also be mentioned wherein ATP is measured by luminescence of a luciferin substrate by the ATP-dependent enzyme luciferase. As a kit for measuring the ATP content in cells, for example, CellTite-Glo^{R} Luminescent Cell viability Assay (manufactured by Promega) and the like may be used.

### 15. Methods of measuring the degree of cell death of cancer cells

As methods of measuring the degree of cell death, a method wherein dead cells are stained with a dye such as Propium Iodide, a method wherein the activity of an enzyme leaked extracellularly as a result of cell death is measured, and the like can be used. For the latter, for example, a method wherein the enzyme activity of extracellularly leaked adenylate kinase is measured can be utilized. More specifically, ToxiLight(R) Non-Destructive Cytotoxicity BioAssay Kit (manufactured by Lonza) and the like may be used.

It is also possible to measure the degree of apoptosis (programmed cell death), which is important in relation to cancers, out of the various types of cell death. As methods of evaluating cell apoptosis, a method wherein the degree of DNA fragmentation is measured, a method wherein changes in cell o membrane constituent lipids are measured, and a method wherein the activity of caspase 3/7, an intracellular protease induced upon apoptosis, is measured, can be mentioned. As a more specific method of measuring caspase 3/7 activity in cells, a method wherein a luciferin substrate liberated by caspase 3/7 activity is measured by luciferin luminescence by a luciferase enzyme reaction can be mentioned. As kits for measuring caspase 3/7 activity in cells, for example, Caspase-Glo^{R} 3/7 Assay (manufactured by Promega) and the like may be used.

Hereinafter, the present invention is described specifically by means of the following examples.

### [Example 1]

### Extraction of micro-RNAs from mesenchymal stem cells

(1) RNA extraction
   Human mesenchymal stem cells (hereinafter referred to as hMSCs) were obtained from Cambrex, and cultured with an IMDM medium (manufactured by Invitrogen) containing 20% fetal bovine serum (FBS) (manufactured by JRH Bioscience) in a 37°C 5% CO₂ concentration incubator. Using the TRIZOL reagent (manufactured by Invitrogen), total RNA was extracted from the hMSCs in culture per the directions attached to the product.
(2) Cloning of small RNAs
   Using 200 µg of the hMSC-derived total RNA acquired in (1) above, and according to the method of Lau et al. (Science 294, 858-862, 2001), excision for small RNAs by means of 15% polyacrylamide gel electrophoresis, 5'-adenylated 3'-adapter ligation, 5'-adapter ligation, reverse transcription, PCR amplification, concatemerization, and ligation to the pCR2.1-TOPO vector were performed sequentially, to achieve cloning of the small RNAs. Next, the nucleotide sequences of the small RNAs cloned were determined. The 5'-adenylated 3'-adapter used was miRNA Cloning Linker, manufactured by IDT.

### [Example 2]

### Identification of micro-RNAs

From among the small RNAs obtained in Example 1, first, those whose nucleotide sequences did not agree with any one in miRBase (http://microrna.sanger.ac.uk/), which is a database for known micro-RNAs, were selected. Surrounding genome sequences wherein DNA sequences corresponding to those nucleotide sequences were extended by about 50 nt toward the 5' side and the 3' side, respectively, were acquired from UCSC Genome Bioinformatics (http://genome.ucsc.edu/), and the secondary structures of the RNAs expected to be transcribed from the genome sequences were predicted using RNAfold. As a result, two species were found to be novel micro-RNAs located in one strand of the hairpin structure. The nucleotide sequences thereof and the nucleotide sequences of micro-RNA precursors comprising these micro-RNAs are shown in Tables 15 to 20. The micro-RNAs having the respective nucleotide sequences were given the names KHK_miR_001 to 029. If one micro-RNA can assume hairpin structures derived from genome sequences at different positions, all thereof are shown. Tables 16 to 20 are continuous from Table 15.

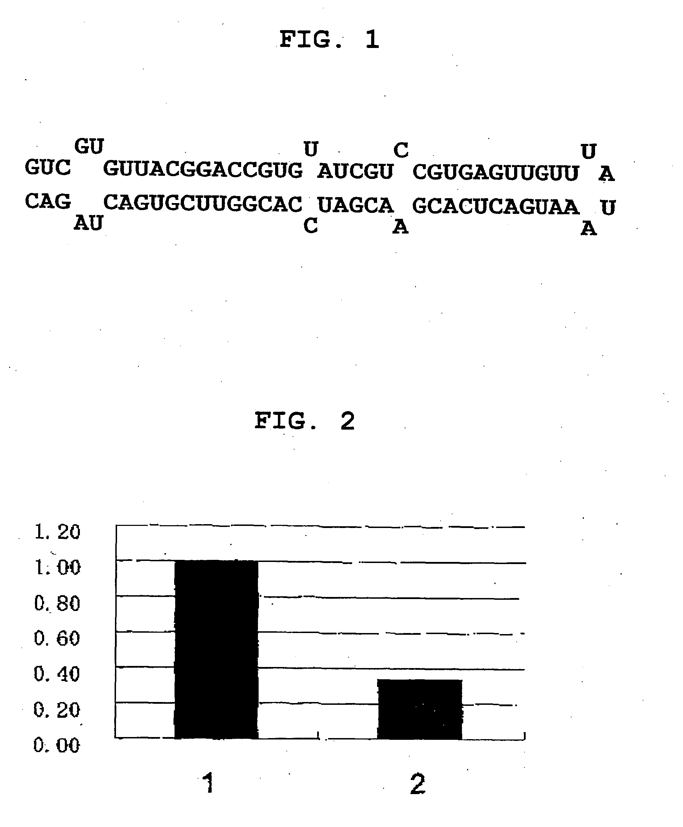

As an example secondary structure, the hairpin structure of KHK_miR_001 is shown in FIG. 1.

**Table 15**

| nucleic acid name | Micro-RNA (5' → 3') | Micro-RNA precursor (5' - 3') |
|---|---|---|
| KHK_miR_001 | CUUGGCACCUAGCAAGCACUCA (SEQ ID NO:1) | CAGAUCAGUGCUUGGCACCUAGCAAGCACUCAG UAAAUAUUUGUUGAGUGCCUGCUAUGUGCCAGG CAUUGUGCUG (SEQ ID NO:30) |
| KHK_miR_002 | UCCUGGCCUCUCUUCUAG (SEQ ID NO:2) | UUCCUGGCCUCUCUUCUAGAGAUUUUGAUUCAG CAGGAUGGGGGUUUCCUGAAAAU (SEQ ID NO:31) |
| | | CUGCUGGCAGACCCUGUGUUCGGCCCGAUCCUG GCCUCUCUUCUAGGUGAGCCUGAGAGCACAGGC ACAGUGGGUACGGGGCAGGUGCUGAGGGAG (SEQ ID NO:32) |
| KHK_miR_003 | GCUCCAACGGUAGGUGCA (SEQ ID NO:3) | GGCUCCAACGGUAGGUGCAGGGCGCUCCGCUGC AGGGGCCCGGCGCGGCCGGGAGAGCC (SEQ ID NO:33) |
| KHK_miR_004 | AUCCCACUCCUGACACCA (SEQ ID NO:4) | UUGGUCGCUCAGAACUGGGGUUGUUAGAAGCU CUACUUCAGAUCCCACUCCUGACACCAU (SEQ ID NO:34) |
| KHK_miR_005 | CCCACUGCUUCACUUGACUAGC (SEQ ID NO:5) | GUGUGUUAGUUAUAGAAGCACUACACUAAGGAU AGAAUUAUGAACGUAUCCCACUGCUUCACUUGA CUAGCCUAAAAAAUAAAAAUAUAUGUGAACAUU ACACA (SEQ ID NO:35) |
| | | CCUUCUCCACUCCCACUGCUUCACUUGACUAGC CUUAAAAACAAAAAAGAAAAGAGAGAGAAAGCA GGCCGGGUGUGGGGCU (SEQ ID NO:36) |
| | | UCCACUCCCACUGCUUCACUUGACUAGCCUAAA AAAUUAAUUAAUUAAAUAAAACAGGUUAUAGGA GAAGGGAGAAGA (SEQ ID NO:37) |

**Table 16**

| | | |
|---|---|---|
| KHK_miR_005 | CCCACUGCUUCACUUGACUAGC (SEQ ID NO:5) | UUCCUUCUUUACUCCCACUGCUUCACUUGACUA GCCUUUAAAAAAGAAAGGCUUGGUUUGAUGAAU GGGUGAGAGAAAAGGUGAG [?] (SEQ ID NO:38) |
| | | UCUACACGCCCACUGCUUCACUUGACUAGCUUU AAAAAACAAUAAAAAUAAAUUGCAUGGCCGGGU GCGGUGGCUCACGC (SEQ ID NO:39) |
| | | CAUAGCUAAUCAUCAGUUACAGAUUCCUUGGUU CCUUCCCCACUCCCACUGCUUCACUUGACUAGC (SEQ ID NO:40) |
| | | AGCUAGUUGAUCACAAUCAGAGAUUUUAUGUU CCUUCUCCAAUCCCACUGCUUCACUUGACUAGC (SEQ ID NO:41) |
| KHK_miR_006 | UGGUCCGAUGGUAGUGGGUU (SEQ ID NO:6) | AUUCAUUUUUAGGCUGGUCCGAUGGUAGUGGGU UAUCAGAAAUUAACAUCAGUGUUACUAAAGUCG GUAUACAACCCUACACUG (SEQ ID NO:42) |
| | | AAGUUCAGAGACACCCUGCUGUCAAACAACAGU GUAACUCCGGCUGGUCCGAUGGUAGUGGUUAU CAGAACUU (SEQ ID NO:43) |
| | | GGGACUGGUCCGAUGGUAGUGGUUAUCAGAAC UUAUUAACAUUCAUGUCCC (SEQ ID NO:44) |
| KHK_miR_007 | GCAGUCCAUGGGCAUAUACACU (SEQ ID NO:7) | GGCGUGAGGGUAUGUGCCUUUGGACUACAUCGU GGAAGCCAGCACCAUGCAGUCCAUGGGCAUAUA CACUUGCCUCAAGGCC (SEQ ID NO:45) |
| KHK_miR_008 | CCUCCCACACCCAAGGCUUGCA (SEQ ID NO:8) | GACUUGCUUUCUCUCCUCCAUGCCUUGAGUGUA GGACCGUUGGCAUCUUAAUUACCCUCCCACACC CAAGGCUUGCAAAAAGCGAGCCUUC |

**Table 17**

| | | |
|---|---|---|
| KHK_miR_009 | GAAGCGGGUGCUCGUAUUUU (SEQ ID NO:9) | GCAGCGAUGGCCGAGUGGUUAAGGCGUUGGACU UGAAAUCCAAUGGGGUCUCCCCGCGCAGGUUCG AACCCUGCUCGCUGCGGAAGCGGGUGCUCUUAU UUUUUCUAUUUUUUUAAAUAAACACCUUCAAAU GAAAACUUUUACUCU (SEQ ID NO:47) |
| KHK_miR_010 | AAGGAGCUUACAAUCUAGCUGGG (SEQ ID NO:10) | GCCCUCAAGGAGCUUACAAUCUAGCUGGGGGUA AAUGACUUGCACAUGAACACAACUAGACUGUGA GCUUCUAGAGGGC (SEQ ID NO:48) |
| KHK_miR_011 | CCCCCCACAACCGCG (SEQ ID NO:11) | AGUUGGUCCGAGUGUUGUGGGUUAUUGUUAAGU UGAUUUAACAUUGUCUCCCCCCACAACCGCGCU UGACU (SEQ ID NO:49) |
| KHK_miR_012 | AGUCCCAUCUGGGUCGCCA (SEQ ID NO:12) | GGACAGAAUAGCAAGCGAAAGGGGUGGUGCUCA CUGCUUGGCAAUAGUCCCAUCUGGGUCGCCAAA AUGUGUCU (SEQ ID NO:50) |
| KHK_miR_013 | UCCCGGGUUUCGGCACCX (SEQ ID NO:13) | UCUAUUGCCUUCCCGGGUUUCGGCACCAGAAUA UAAGGUCAGCCGAGAGAAAGGAUGAGA (SEQ ID NO:51) |
| KHK_miR_014 | AGAAGGGAAGUACAU (SEQ ID NO:14) | AUAAUUCAAUGUGACAUCUCUUUGAAUGACCAU CACCUGCUUGCUCCUUAGAAGGGAAGUACAUUU UUAU (SEQ ID NO:52) |
| KHK_miR_015 | AUCCGAGUCACGGCACCA (SEQ ID NO:15) | GGAGGACAGGGGAUUGAUCUCGGAUUGUGACUC CCCAGAUCCGAGUCACGGCACCAAAAUUUC (SEQ ID NO:53) |
| KHK_miR_016 | ACCCCACUCCUGGUACCA (SEQ ID NO:16) | UGGCUGCUGGGCCUGGCAAUGCCCCUAGGACAU AUCAGAGACACCCCACUCCUGGUACCA (SEQ ID NO:54) |
| | | UUCUAUUAGGUGGCAGCCCUGGUACAAAGACUG GAGUCAAAAUUAAACCCCACUCCUGGUACCAGU |

**Table 18**

| | | |
|---|---|---|
| | | UUCUAUUAGUUAGAG (SEQ ID NO:55) |
| KHK_miR_016 | ACCCCACUCCUGGUACCA (SEQ ID NO:16) | CAGUUCCAAAGUUGUUUCCACUUUUUGGGUAU UUUAUCAGCAAUACCCCACUCCUGGUACCAACU UACUG (SEQ ID NO:56) |
| | | UGGCACCCCACUCCUGGUACCAAAGUCUGAAUU UGUUUGCUUGGGUUGCCA (SEQ ID NO:57) |
| KHK_miR_017 | UGUGUGUGUGCUUGUAUAUAUA (SEQ ID NO:17) | UAUAUGUGUGUGUGCUUGUAUAUAUAUAUAUAU AUAUAUAUAUAUACAAGCACACACACACAAAUG (SEQ ID NO:58) |
| | | UCUUGCAUUUGUGUGUGUGCUUGUAUAUAUAUA UAUAUAUAUAUAUAUAUACAAGCACACACACAU AUAUAGUA (SEQ ID NO:59) |
| KHK_miR_018 | UAGUGCAAUAUUGCUUAUAGGGU (SEQ ID NO:18) | CUAGAACCCUAUCAAUAUUGUCUCUGCUGUGUA AAUAGUUCUGAGUAGUGCAAUAUUGCUUAUAGG GUUUGG (SEQ ID NO:60) |
| KHK_miR_019 | UAUGUCUGCUGACCAUCACC (SEQ ID NO:19) | GGGUAAGUGGAAAGAUGGUGGGCCGCAGAACAU GUGCUGAGUUCGUGCCAUAUGUCUGCUGACCAU CACCUUUAGAAGCCC (SEQ ID NO:61) |
| KHK_miR_020 | AAACAUUCGCGGUGCACUUCUUU (SEQ ID NO:20) | GACGUGCGGUACUUAAUGAGAAGUUGCCCGUGU UUUUUUCGCUUUAUUUGUGACGAAACAUUCGCG GUGCACUUCUUUUUCAGUAUCCUAUUC (SEQ ID NO:62) |
| KHK_miR_021 | CCCGCCGCGGCCCCAGG (SEQ ID NO:21) | GCCUGGGCUGCAGCGGGAGCGACGCGCCGGCCG CCGGGUGGGGUGGAGCCCCGCCGCGGCCCCAG GU (SEQ ID NO:63) |
| | | GUCCUGGGACAGGUCGCGUUCAGCGUGGGCGUC |

**Table 19**

| | | |
|---|---|---|
| | | GGCCCCGCCGCGGCCCCAGGC (SEQ ID NO.64) |
| KHK_miR_022 | AGUCCCUUCGUGGUCGCCA (SEQ ID NO:22) | UUGGCGAUAGGCGAUGGUCUCACCGCUCAGCGA CAGGCAAAAGUCCCUUCGUGGUCGCCAA (SEQ ID NO:65) |
| KHK_miR_023 | UUCCUCUGAUGACUUC (SEQ ID NO:23) | UGGGAUUGAGAAGGGAGCAGUGAAGGACCGGG AGGUUAUACUUUCCUCUGAUGACUUCCUGUUAG UGCCA (SEQ ID NO:66) |
| KHK_miR_024 | AGCUCAGUGGAAUGGCUCAUGCUUU (SEQ ID NO:24) | AGCUCAGUGGAAUGGCUCAUGCUUUUAAUCCCA GCACUUUGGGAGGCUGAGGCAGGAGGAUUACUU GAGCU (SEQ ID NO:67) |
| KHK_miR_025 | CGGGCGUGGUGGUGGGGG (SEQ ID NO:25) | CAGGCAACAUGGCGAAAACCCGUCUCUAAUGAA AAUACAAAAAUUAGCCGGGCGUGGUGGUGGGGG CCUG (SEQ ID NO:68) |
| | | CAUGGUGAAACCCUGUCCCUACUAAAGAUACAA AAAUUAGCCGGGCGUGGUGGUGGGGCCUAUAA UCCCAGCUACUCGGGAGGCUGAGGUAGGAGAAU CGCUUG (SEQ ID NO:69) |
| | | UUAGCCGGGCGUGGUGGUGGGGGCCUGUAGUCC CAGCUACUCGGGAGGCUGA (SEQ ID NO:70) |
| | | UUAGCCGGGCGUGGUGGUGGGGGCCUAUAAUCC CAGCUACUGACGAGGCUGA (SEQ ID NO:71) |
| | | UUAGCCGGGCGUGGUGGUGGGGGCCUGUAAUCC CAGCUACUCGGGAGGCUGA (SEQ ID NO:72) |
| KHK_miR_026 | CCCGGCAUCUCCACCA | GUGUCAUSGGACAGUGGGCCCUGGUGCCACAAU |

**Table 20**

| | | |
|---|---|---|
| | (SEQ ID NO:26) | GCCCGGCAUCUCCACCAGAUAC (SEQ ID NO.73) |
| KHK_miR_027 | CUAUACFAUCUAUUGCCUUCC (SEQ ID NO:27) | UCAGAGUGAGGUAGUAGAUUGUAUAGUUGUGGG GUAGUGAUUUUACCCUGUUCAGGAGAUAACUAU ACAAUCUAUUGCCUUCCCUGA (Homo sapiens let-7f-1 stem loop) |
| KHK_miR_028 | CUAUACAACCUACUGCCUUCC (SEQ ID NO:28) | CGGGGUGAGGUAGUAGGUUGUGUGGUUUCAGGG CAGUGAUGUUGCCCCUCGGAAGAUAACUAUACA ACCUACUGCCUUCCCUG (Homo sapiens let-7b stem loop) |
| KHK_miR_029 | GCAGUCCAUGGGCAUAUACACU (SEQ ID NO:29) | UCCCUGGCGUGAGGGUAUGUGCCUUUGGACUAC AUCGUGGAAGCCAGCACCAUGCAGUCCAUGGGC AUAUACACUUGCCUCAAGGCCUAUGUCAUC (Homo sapiens miR-455 stem loop) |
| KHK_miR_030 | UGGAAUACCGGGUGCUGUAGGCU U (SEQ ID NO:78) | AGGUCGGGUCAGUUAGUACUUGGAUGGAGACU GCCUGGAAUACCGGGUGCUGUAGGCUUUUGGCC U (SEQ ID NO:81) |
| KHK_miR_031 | AUCCCUUCGUGGUUGCCA (SEQ ID NO:79) | UAGAACAGAAUUGCAAGUGAAAGGGUCCGAUG GUACUCACUACUUGGUGAUUAUCCCUUCGUGGU UGCCAAAUGUGUCUG (SEQ ID NO:82) |
| KHK_miR_032 | AUCCCACCACUGCCACCA (SEQ ID NO:80) | CGGGCGGCUGCUGCUCUCCGUUUAUCCCACCAC UGCCACCAUUAUUGCUACUGUUCAGCAGGUGCU GCUGGUGGUGAUGGUGAUAGUCUGGUGGGGGCG GUGGGGCUGCUGUUGCUGCUGCUUG (SEQ ID NO:83) |

### [Example 3]

### Detection of functions of micro-RNAs

By determining whether or not a micro-RNA obtained in Example 2 undergoes processing by Dicer protein, whether or not the same functions as a micro-RNA can be determined.
Of the micro-RNAs obtained in Example 2, the micro-RNA of KHK_miR_001 can have a function thereof detected as described below. First, a single-stranded RNA having the nucleotide sequence of SEQ ID NO:30 is synthesized, and reacted with the Dicer Enzyme attached to the X-treme GENE siRNA Dicer Kit (manufactured by Roche-Applied Science). Next, the reaction product is electrophoresed with 15% polyacrylamide gel; detection of a band 20 to 25 nucleotides in size indicates that the possession of a function as a micro-RNA.

### [Example 4]

### Detection of expression levels of micro-RNAs

On the micro-RNAs obtained in Example 2, expression levels in hMSCs and HeLa cells were examined using quantitative RT-PCR.
The hMSCs and HeLa cells were treated in the same manner as Example 1 to extract total RNA, and respective template cDNAs were synthesized using Thermo-X (manufactured by Invitrogen) and a random hexamer (manufactured by Invitrogen), per the directions attached to the products. Each template cDNA was synthesized from 1 µL of the total RNA, and diluted with water to obtain a final volume of 200 µL. Of this, 2 µL was used in the following quantitative RT-PCR analysis.

To examine the expression of the micro-RNA of KHK_miR_007 out of the micro-RNAs obtained in Example 2, quantitative RT-PCR was performed as described below. The reagents used were in a kit manufactured by Takara Shuzo Co., Ltd. (For Real Time PCR TaKaRa Ex Taq R-PCR Version 2.1). After preparing a reaction liquid consisting of 2.0 µL of the above-described template cDNA, 4.0 µL of 5×R-PCR buffer (Mg²⁺ free), 0.4 µL of 250 mM Mg²⁺ solution, 0.6 µl of dNTPs (10 mmol/L), 1.0 µL of SYBR Green I (diluted to 1/2500 volume), 0.06 µL of each micro-RNA-specific primer (100 µmol/L), 0.2 µL of ExTaq R-PCR Version, and 11.68 µL of water, PCR was performed using an ABI sequence detection system (ABI PRISM 7700, manufactured by Applied Biosystems).

The micro-RNA-specific primers were designed as described below.
A primers was synthesized that had the nucleotide sequences of SEQ ID NO:74 and SEQ ID NO:75, respectively, on the 5'-terminal side and 3'-terminal side of the nucleotide sequence of SEQ ID NO:45. For background correction, to detect the expression level of U6RNA, a primer having the nucleotide sequences of SEQ ID NO:76 and SEQ ID NO:77 was synthesized.

The reaction conditions used for detection of KHK_miR_007 consisted of initial heating at 95°C for 5 minutes, followed by heating at 95°C for 15 seconds and at 65°C for 1 minute, repeated in 45 cycles, and final heating at 25°C for 2 minutes; the conditions used for detection of U6RNA consisted of initial heating at 95°C for 5 minutes, followed by heating at 95°C for 15 seconds and at 60°C for 1 minute, repeated in 45 cycles, and final heating at 25°C for 2 minutes. For negative control, a PCR was also performed using sterile water in place of the template cDNA.

An expression level was calculated by determining the number of cycles for reach to a signal intensity of 1000 (Ct value), and then subtracting the above-described value from the Ct value for the negative control to obtain a ΔCt value. Likewise, the ΔCt value of U6RNA was calculated, and a correction was made on the basis of the difference from the mean of the ΔCt values of U6RNA in hMSCs and HeLa cells, as the background. Since the expression level decreases 2.0 folds as the ΔCt value decreases by 1.0, corrected ΔCt values of KHK_miR_007 for the hMSCs and HeLa cells were calculated as relative expression levels. As a result, as shown in FIG. 2, KHK_miR_007 was found to be strongly expressed in the hMSCs.

### [Example 5]

### Differentiation of mesenchymal stem cells with micro-RNA expressed forcibly therein into osteoblasts

Each micro-RNA precursor obtained in Example 1 was introduced into hMSCs, the cells were cultured under conditions that induce differentiation into osteoblasts, and the influence of the micro-RNA precursor was examined.
The hMSCs were sown to a 24-well plate at 6.2x10³ cells per well, and cultured with an IMDM medium containing 20% FBS overnight. One day later, themicro-RNA precursor was introduced into the hMSCs using a lipofection method, specifically, Lipofectamine 2000 (manufactured by Invitrogen), to obtain a final concentration of 20 nM. The micro-RNA precursors used were KHK_miR_001, 006, 008, 009, 010, 012, 017, 018, 019, 020, 022, and 024, synthesized as Pre-miR^{™} miRNA Precursor Molecules by Ambion. These were chemically synthesized double-stranded nucleic acid molecules, and were designed to allow nucleic acids consisting of the nucleotide sequences of SEQ ID NO:1, 6, 8, 9, 10, 12, 17, 18, 19, 20, 22, and 24, respectively, to be incorporated by a complex similar to RISC, which is a factor for the activity of an miRNA, to exhibit the same function as the miRNA. For the sequence corresponding to the human genome sequence of SEQ ID NO:4, a nucleic acid consisting of a sequence having two nucleotides added to the 3' side of SEQ ID NO:4 (SEQ ID NO:84) was provided, and KHK_miR_004_2, synthesized as Pre-miR^{™} miRNA Precursor Molecules by Ambion, was also used. Lipofection was performed per the directions attached to the product.

One day after thelipofection, the medium was replaced with an osteoblast differentiation induction medium [an IMDM medium containing 20% FBS, supplemented with 0.1 µmol/L dexamethasone, 50 µmol/L ascorbic acid-diphosphoric acid (manufactured by Sigma), and 10 mmol/L β-glycerophosphite (manufactured by Sigma)], and cultivation was continued with renewal of the osteoblast differentiation induction medium at a frequency of once per 3 days.
Two weeks after the start of the cultivation, cell morphology was examined under a phase-contrast microscope (manufactured by Nikon), and alkaline phosphatase staining was performed, to detect osteoblasts. Specifically, first, the cells were once washed with phosphate buffer solution (phosphate-buffered saline: PBS) (manufactured by Invitrogen), and fixed with a fixative solution (10% formalin/PBS) for 5 minutes. The cells were washed with distilled water, and thereafter reacted with a mixed solution of Naphthol AS-MX phosphate (manufactured by Sigma) and Fast Violet B solution (manufactured by Sigma) in the dark for 30 minutes to achieve an alkaline phosphatase reaction. Furthermore, the cells were washed with distilled water, and osteoblasts stained red were examined under a phase contrast microscope and photographed using a digital camera (manufactured by Nikon).

As a result, it was found that the hMSCs incorporating Pre-miR^{™} miRNA Precursor Molecules of KHK_miR_001, 006, 010, 017, 019, and 024 had a fewer cells than the hMSCs not incorporating any micro-RNA precursor, and also had a fewer positive cells stained by alkaline phosphatase. Because micro-RNA precursors are converted to micro-RNAs in cells, it was found that the micro-RNAs used in this Example exhibit a suppressive activity on the proliferation of hMSCs and suppressive activity on the differentiation thereof into osteoblasts.

### [Example 6]

### Action on degranulation of human mast cells with micro-RNA expressed forcibly therein

Each micro-RNA precursor obtained in Example 1 was introduced to LAD2, a human mast cell line, to induce degranulation, and the influence of the micro-RNA precursor was examined.
LAD2 is a recently established human mast cell line, and is known to well retain the nature of human mast cells [Leuk. Res., 27, 671 (2003); Leuk. Res., 27, 677 (2003)]. LAD2 was obtained from the National Institute of Allergy and Infectious Diseases, National Institutes of Health (Bethesda, MD 20892-1881, USA), and cultured in a Stem Pro-34 medium [manufactured by Invitrogen] containing 100 ng/mL SCF.

The LAD2 was sown to a 6-well plate at 5x10⁵ cells per well, and a micro-RNA precursor was introduced using a lipofection method, specifically, Gene Silencer (manufactured by Genlantis), to obtain a final concentration of 30 nM. The micro-RNA precursors used were KHK_miR_001, 004_2, 006, 008, 009, 010, 012, 017, 018, 019, 020, 022, and 024, synthesized as Pre-miR^{™} miRNA Precursor Molecules by Ambion. Lipofection was performed per the directions attached to the product.

Two days after introduction of the micro-RNA precursor by a lipofection method, 1 µg/mL human myeloma IgE (manufactured by Cosmo Bio Co., Ltd.) was added, and the cells were cultured in a 37°C 5% CO₂ concentration incubator overnight. On the day that followed, the medium was removed via centrifugation, and the plate was washed with a Tyrode buffer solution (126.1 mmol/L NaCl, 4.0 mmol/L KCl, 1.0 mmol/L CaCl, 0.6 mmol/L MgCl₂, 0.6 mmol/L KH₂PO₄, 10 mM HEPES, 5.6 mmol/L D-glucose, 0.1% bovine serum albumin, pH 7.4), after which 3.9 mL of the Tyrode buffer solution was added to suspend the cells, and the suspension was dispensed to a 96-well plate at 100 µL per well. Next, a rabbit anti-human IgE antibody (manufactured by DAKO) was added to obtain a final concentration of 10 µg/mL, and this was followed by incubation in a 37°C 5% CO₂ concentration incubator for 20 minutes to induce degranulation. The supernatant was recovered via centrifugation, and the β-hexosaminidase activity in the supernatant was measured, whereby the degree of degranulation was determined. The β-hexosaminidase activity was measured by adding 50 µL of 4 mmol/L p-nitrophenyl N-acetyl-β-glucosaminide (manufactured by Sigma) dissolved in 40 mmol/L citrate buffer solution (pH 4.5) to 50 µL of the supernatant recovered, and incubating the mixture at 37°C for 1 hour, thereafter adding 100 µL of 0.2 mol/L glycine (pH 10.7), and measuring the absorbance of the sample at 405 nm using the plate reader 1420 ARVOsx (manufactured by Perkin Elmer). By performing the same experiment but without the addition of the rabbit anti-human IgE antibody, the amount of β-hexosaminidase released spontaneously without stimulation via the IgE receptor was measured. Also, by performing the same experiment but with the addition of Triton X-100 at a final concentration of 1% in place of the rabbit anti-human IgE antibody, the total β-hexosaminidase activity in LAD2 was measured. The ratio of degranulation was calculated as the ratio (%) of the β-hexosaminidase activity in the supernatant relative to total β-hexosaminidase activity, and is shown in Table 21.

**Table 21**

| Micro-RNA precursor introduced | Anti-IgE stimulation | β-Hexosamidase activity (%) |
|---|---|---|
| Lipofection reagent only | - | 2.19 |
| | + | 14.15 |
| KHK_miR_001 | - | 1.85 |
| | + | 27.81 |
| KHK_miR_017 | - | 3.00 |
| | + | 31.61 |
| KHK_miR_018 | - | 2.78 |
| | + | 29.78 |
| KHK_miR_019 | - | 1.71 |
| | + | 21.90 |
| KHK_miR_022 | - | 0.95 |
| | + | 27.93 |

As a result, it was found that by introduction of KHK_miR_001, 017, 018, 019, or 022, the degranulation of LAD2 by stimulation with IgE receptor is promoted.

### [Example 7]

### Viable cell ratio and apoptotic activity in colon cancer-derived cell line with micro-RNA expressed forcibly therein

Each micro-RNA precursor obtained in Example 1 was introduced to a colon cancer-derived cell line, and the effects of the micro-RNA precursor on viable cell ratio and apoptotic activity were examined.
The DLD-1 human colorectal cancer-derived cell line (hereinafter referred to as DLD-1) was obtained from the American Type Culture Collection (ATCC) (hereinafter referred to as ATCC) (ATCC CCL-221). Cells were cultured with an RPMI1640 medium (manufactured by Invitrogen) containing 10% fetal bovine serum (FBS) (manufactured by JRH Biosciences) in a 37°C 5% CO₂ concentration incubator.

DLD-1 was sown to a 96-well plate at 2500 cells per well, and cultured in an RPMI medium containing 10% FBS overnight. After 1 day, a micro-RNA precursor was introduced to the DLD-1 using a lipofection method, specifically Lipofectamine 2000 (manufactured by Invitrogen), to obtain a final concentration of 5 nM or 25 nM. The micro-RNA precursors used were KHK_miR_001, 004_2, 006, 008, 009, 010, 012, 017, 018, 019, 020, 022, and 024 synthesized as Pre-miR^{™} miRNA Precursor Molecules by Ambion. Pre-miR^{™} miRNA Precursor Molecules-Negative Control #2 (hereinafter referred to as miR-negacon #2) (manufactured by Ambion) was also introduced to DLD-1, and this was used as the negative control. Lipofection was performed per the directions attached to the product.

Three days after introduction of the micro-RNA precursor by a lipofection method, viable cell ratios were measured using CellTiter-Glo^{™} Luminescent Cell Viability Assay (manufactured by Promega). Taking the viable cell ratio of DLD-1 in a control plot (Lipofectamine 2000 only) as 1.0, the relative viable cell ratio of each was calculated. As a result, as shown in Table 22, with introduction of KHK_miR_008, 010, or 017, a decrease of 40% or more in viable cell ratio was observed.

**Table 22**

| Micro-RNA precursor introduced | Viable cell ratio (5nM) | Viable cell ratio (25nM) |
|---|---|---|
| KHK_miR_010 | 0.39 | 0.33 |
| KHK_miR_017 | 0.40 | 0.33 |
| KHK_miR_008 | 0.64 | 0.34 |
| miR-negacon#2 | 0.87 | 0.87 |

Two days after introduction of the micro-RNA precursor by a lipofection method, caspase 3/7 activity was measured using Caspase-Glo^{R} 3/7 assay (manufactured by Promega) per the directions attached to the product. Taking the caspase 3/7 activity value of DLD-1 in a control plot (Lipofectamine 2000 only) as 1.0, the relative caspase 3/7 activity value of each was calculated. As a result, as shown in Table 23, with introduction of KHK_miR_008, 009, 010, or 017, an increase of 150% or more in caspase 3/7 activity was observed.

**Table 23**

| Micro-RNA precursor introduced | Caspase 3/7 activity |
|---|---|
| KHK_miR_010 | 2.48 |
| KHK_miR_017 | 2.41 |
| KHK_miR_008 | 2.32 |
| KHK_miR_009 | 1.86 |
| miR-negacon#2 | 0.85 |

### [Example 8]

### Viable cell ratio and apoptotic activity in ovarian cancer-derived cell line with micro-RNA expressed forcibly therein

Each micro-RNA precursor obtained in Example 1 was introduced to an ovarian cancer-derived cell line, and the effects of the micro-RNA precursor on viable cell ratio and apoptotic activity were examined.
The A2780 human ovarian cancer-derived cell line (Nature, 295, 116-119 (1982); Science, 224, 994-996 (1984); Semin. Oncol., 11, 285-298 (1984)) was cultured with an RPMI1640 medium (manufactured by Invitrogen) containing 5% FBS (manufactured by JRH Biosciences) in a 37°C 5% CO₂ concentration incubator.

A2780 was sown to a 96-well plate at 2500 cells per well, and cultured with an RPMI medium containing 10% FBS overnight. After 1 day, the micro-RNA precursor was introduced to A2780 using a lipofection method, specifically Lipofectamine 2000 (manufactured by Invitrogen), to obtain a final concentration of 5 nM or 25 nM. The micro-RNA precursors used were KHK_miR_001, 004_2, 006, 008, 009, 010, 012, 017, 018, 019, 020, 022, and 024 synthesized as Pre-miR^{™} miRNA Precursor Molecules of by Ambion. miR-negacon #2 (manufactured by Ambion) was also introduced to A2780, and this was used as the negative control.

Three days after introduction of the micro-RNA precursor by a lipofection method, viable cell ratios were measured using CellTiter-Glo^{™} Luminescent Cell Viability Assay (manufactured by Promega). Taking the viable cell ratio of A2780 in a control plot (Lipofectamine 2000 only) as 1.0, the relative viable cell ratio of each was calculated. As a result, as shown in Table 24, with introduction of KHK_miR_001, 004_2, 006, 008, 010, 017, 019, 020, or 022, a decrease of 40% or more in viable cell ratio was observed. Conversely, with introduction of KHK_miR_018, an increase in viable cell ratio was observed.

**Table 24**

| Micro-RNA precursor introduced | Viable cell ratio (5nM) | Viable cell ratio (25nM) |
|---|---|---|
| KHK_miR_017 | 0.26 | 0.34 |
| KHK_miR_010 | 0.27 | 0.34 |
| KHK_miR_001 | 0.35 | 0.44 |
| KHK_miR_004_2 | 0.44 | 0.49 |
| KHK_miR_020 | 0.49 | 0.67 |
| KHK_miR_008 | 0.50 | 0.48 |
| KHK_miR_019 | 0.53 | 0.72 |
| KHK_miR_006 | 0.57 | 0.62 |
| KHK_miR_022 | 0.58 | 0.73 |
| KHK_miR_018 | 1.21 | 1.42 |
| miR-negacon#2 | 1.00 | 1.05 |

Two days after introduction of the micro-RNA precursor by a lipofection method, caspase 3/7 activity was measured using Caspase-Glo^{R} 3/7 assay (manufactured by Promega) per the directions attached to the product. Taking the caspase 3/7 activity value of A2780 in a control plot (Lipofectamine 2000 only) as 1.0, the relative caspase 3/7 activity value of each was calculated. As a result, as shown in Table 25, with introduction of KHK_miR_010, an increase of 150% or more in caspase 3/7 activity was observed.

**Table 25**

| Micro-RNA precursor introduced | Caspase 3/7 activity |
|---|---|
| KHK_miR_010 | 1.69 |
| miR-negacon#2 | 0.90 |

### Industrial Applicability

Provided by the present invention is a nucleic acid, micro-RNA and micro-RNA precursor having a novel nucleic acid sequence. A nucleic acid, micro-RNA or micro-RNA precursor of the present invention makes it possible to detect the expression or a mutation of a micro-RNA, to separate cells, to suppress the translation of a gene having a target sequence, to screen for a substance that promotes or suppresses a function of a micro-RNA, to diagnose or treat a disease caused by an abnormality of mesenchymal stem cell proliferation or differentiation, to diagnose or treat a disease caused by a mast cell abnormality, and to diagnose and treat a cancer and a disease caused by abnormal proliferation of cells or tissue hyperplasia and the like.

### [Sequence Listing]

### [Sequence Listing free text]

Sequence No. 74-explanation of artificial sequence: synthetic DNA
Sequence No. 75-explanation of artificial sequence: synthetic DNA
Sequence No. 76-explanation of artificial sequence: synthetic DNA
Sequence No. 77-explanation of artificial sequence: synthetic DNA

## Claims

1. A nucleic acid consisting of a nucleotide sequence having an identity of 90% or more with the nucleotide sequence of any one of SEQ ID NO:1 to 29 and 78 to 80.

2. A nucleic acid that hybridizes under stringent conditions with a complementary strand of a nucleic acid consisting of the nucleotide sequence of any one of SEQ ID NO:1 to 29 and 78 to 80.

3. A micro-RNA consisting of the nucleotide sequence of any one of SEQ ID NO:1 to 29 and 78 to 80.

4. A micro-RNA precursor comprising the nucleic acid described in any one of claims 1 to 3.

5. The micro-RNA precursor described in claim 4, consisting of a nucleotide sequence having an identity of 80% or more with the nucleotide sequence of any one of SEQ ID NO:30 to 73 and 81 to 83.

6. The micro-RNA precursor described in claim 4 or 5, which hybridizes under stringent conditions with a complementary strand of a nucleic acid consisting of the nucleotide sequence of any one of SEQ ID NO:30 to 73 and 81 to 83.

7. A micro-RNA precursor consisting of the nucleotide sequence of any one of SEQ ID NO:30 to 73 and 81 to 83.

8. A nucleic acid consisting of a nucleotide sequence complementary to the nucleic acid, micro-RNA or micro-RNA precursor described in any one of claims 1 to 7.

9. A double-stranded nucleic acid consisting of the nucleic acid, micro-RNA or micro-RNA precursor described in any one of claims 1 to 7 and a nucleic acid consisting of a nucleotide sequence complementary to the nucleotide sequence of the nucleic acid, micro-RNA or micro-RNA precursor.

10. A method of detecting the expression of a micro-RNA, comprising using the nucleic acid, micro-RNA or micro-RNA precursor described in any one of claims 1 to 9.

11. A method of detecting a mutation of a micro-RNA, comprising using the nucleic acid, micro-RNA or micro-RNA precursor described in any one of claims 1 to 9.

12. A method of separating a cell, comprising using the nucleic acid, micro-RNA or micro-RNA precursor described in any one of claims 1 to 9.

13. A vector that expresses the nucleic acid, micro-RNA or micro-RNA precursor described in any one of claims 1 to 9.

14. A method of suppressing the expression of a gene having a target nucleotide sequence of the nucleic acid, micro-RNA or micro-RNA precursor described in any one of claims 1 to 9, comprising using the nucleic acid, micro-RNA or micro-RNA precursor.

15. A method of screening for a substance that promotes or suppresses the expression or a function of a micro-RNA or micro-RNA precursor, comprising using the nucleic acid, micro-RNA or micro-RNA precursor described in any one of claims 1 to 9.

16. A diagnostic reagent for a disease caused by an abnormality of mesenchymal stem cell proliferation and/or differentiation, comprising the nucleic acid, micro-RNA or micro-RNA precursor described in any one of claims 1 to 9 as an active ingredient.

17. A therapeutic drug for a disease caused by an abnormality of mesenchymal stem cell proliferation and/or differentiation, comprising the nucleic acid, micro-RNA or micro-RNA precursor described in any one of claims 1 to 9 as an active ingredient.

18. A cell incorporating the nucleic acid, micro-RNA or micro-RNA precursor described in any one of claims 1 to 9.

19. A cell incorporating the vector described in claim 13.

20. A diagnostic reagent or therapeutic drug for a disease caused by a mast cell abnormality, comprising the nucleic acid, micro-RNA or micro-RNA precursor described in any one of claims 1 to 4 wherein the SEQ ID NO: is any one of 1, 17, 18, 19, and 22, as an active ingredient.

21. An agent for promoting mast cell degranulation, comprising the nucleic acid, micro-RNA or micro-RNA precursor described in any one of claims 1 to 4 wherein the SEQ ID NO: is any one of 1, 17, 18, 19, and 22, as an active ingredient.

22. A diagnostic reagent or therapeutic drug for a disease caused by a mast cell abnormality, comprising the micro-RNA precursor described in any one of claims 5 to 7, wherein the SEQ ID NO: is any one of 30, 58, 59, 60, 61, and 65, as an active ingredient.

23. An agent for promoting mast cell degranulation, comprising the micro-RNA precursor described in any one of claims 5 to 7, wherein the SEQ ID NO: is any one of 30, 58, 59, 60, 61, and 65, as an active ingredient.

24. A diagnostic reagent or therapeutic drug for a disease caused by a mast cell abnormality, comprising a nucleic acid consisting of a nucleotide sequence complementary to the nucleic acid, micro-RNA or micro-RNA precursor described in any one of claims 1 to 4, wherein the SEQ ID NO: is any one of 1, 17, 18, 19, and 22, or to the micro-RNA precursor described in any one of claims 5 to 7, wherein the SEQ ID NO: is any one of 30, 58, 59, 60, 61, and 65, as an active ingredient.

25. An agent for suppressing mast cell degranulation, comprising a nucleic acid consisting of a nucleotide sequence complementary to the nucleic acid, micro-RNA or micro-RNA precursor described in any one of claims 1 to 4, wherein the SEQ ID NO: is any one of 1, 17, 18, 19, and 22, or to the micro-RNA precursor described in any one of claims 5 to 7, wherein the SEQ ID NO: is any one of 30, 58, 59, 60, 61, and 65, as an active ingredient.

26. A diagnostic reagent or therapeutic drug for a disease caused by a mast cell abnormality, comprising a double-stranded nucleic acid consisting of the nucleic acid, micro-RNA or micro-RNA precursor described in any one of claims 1 to 4, wherein the SEQ ID NO: is any one of 1, 17, 18, 19, and 22, or of the micro-RNA precursor described in any one of claims 5 to 7, wherein the SEQ ID NO: is any one of 30, 58, 59, 60, 61, and 65, and a nucleic acid consisting of a nucleotide sequence complementary to the nucleotide sequence of the nucleic acid, micro-RNA or micro-RNA precursor, as an active ingredient.

27. An agent for promoting or suppressing mast cell degranulation, comprising a double-stranded nucleic acid consisting of the nucleic acid, micro-RNA or micro-RNA precursor described in any one of claims 1 to 4, wherein the SEQ ID NO: is any one of 1, 17, 18, 19, and 22, or of the micro-RNA precursor described in any one of claims 5 to 7, wherein the SEQ ID NO: is any one of 30, 58, 59, 60, 61, and 65, and a nucleic acid consisting of a nucleotide sequence complementary to the nucleotide sequence of the nucleic acid, micro-RNA or micro-RNA precursor, as an active ingredient.

28. A diagnostic reagent or therapeutic drug for a disease caused by a cell proliferation abnormality, comprising the nucleic acid, micro-RNA or micro-RNA precursor described in any one of claims 1 to 4, wherein the SEQ ID NO: is any one of 1, 4, 6, 8, 9, 10, 17, 18, 19, 20, and 22, as an active ingredient.

29. A diagnostic reagent or therapeutic drug for a disease caused by a cell proliferation abnormality, comprising the micro-RNA precursor described in any one of claims 5 to 7, wherein the SEQ ID NO: is any one of 30, 34, 42, 43, 44, 46, 47, 48, 58, 59, 60, 61, 62, and 65, as an active ingredient.

30. A diagnostic reagent or therapeutic drug for a disease caused by a cell proliferation abnormality, comprising a nucleic acid consisting of a nucleotide sequence complementary to the nucleic acid, micro-RNA or micro-RNA precursor described in any one of claims 1 to 4, wherein the SEQ ID NO: is any one of 1, 4, 6, 8, 9, 10, 17, 18, 19, 20, and 22, or to the micro-RNA precursor described in any one of claims 5 to 7, wherein the SEQ ID NO: is any one of 30, 34, 42, 43, 44, 46, 47, 48, 58, 59, 60, 61, 62, and 65, as an active ingredient.

31. A diagnostic reagent or therapeutic drug for a disease caused by a cell proliferation abnormality, comprising a double-stranded nucleic acid consisting of the nucleic acid, micro-RNA or micro-RNA precursor described in any one of claims 1 to 4, wherein the SEQ ID NO: is any one of 1, 4, 6, 8, 9, 10, 17, 18, 19, 20, and 22, or of the micro-RNA precursor described in any one of claims 5 to 7, wherein the SEQ ID NO: is any one of 30, 34, 42, 43, 44, 46, 47, 48, 58, 59, 60, 61, 62, and 65, and a nucleic acid consisting of a nucleotide sequence complementary to the nucleotide sequence of the nucleic acid, micro-RNA or micro-RNA precursor, as an active ingredient.

32. The diagnostic reagent or therapeutic drug described in any one of claims 28 to 31, wherein the disease is a disease selected from the group consisting of cancers, arteriosclerosis, chronic rheumatoid arthritis, prostatic hyperplasia, blood vessel restenosis after percutaneous transvascular coronary angioplasty, fibroid lung, glomerulonephritis and autoimmune diseases.

33. An agent for suppressing cell proliferation, comprising the nucleic acid, micro-RNA or micro-RNA precursor described in any one of claims 1 to 4, wherein the SEQ ID NO: is any one of 1, 4, 6, 8, 9, 10, 17, 19, 20, and 22, as an active ingredient.

34. An agent for suppressing cell proliferation, comprising the micro-RNA precursor described in any one of claims 5 to 7, wherein the SEQ ID NO: is any one of 30, 34, 42, 43, 44, 46, 47, 48, 58, 59, 61, 62, and 65, as an active ingredient.

35. An agent for promoting cell proliferation, comprising a nucleic acid consisting of a nucleotide sequence complementary to the nucleic acid, micro-RNA or micro-RNA precursor described in any one of claims 1 to 4, wherein the SEQ ID NO: is any one of 1, 4, 6, 8, 9, 10, 17, 19, 20, and 22, or to the micro-RNA precursor described in any one of claims 5 to 7, wherein the SEQ ID NO: is any one of 30, 34, 42, 43, 44, 46, 47, 48, 58, 59, 61, 62, and 65, as an active ingredient.

36. An agent for suppressing or promoting cell proliferation, comprising a double-stranded nucleic acid consisting of the nucleic acid, micro-RNA or micro-RNA precursor described in any one of claims 1 to 4, wherein the SEQ ID NO: is any one of 1, 4, 6, 8, 9, 10, 17, 19, 20, and 22, or of the micro-RNA precursor described in any one of claims 5 to 7, wherein the SEQ ID NO: is any one of 30, 34, 42, 43, 44, 46, 47, 48, 58, 59, 61, 62, and 65, and a nucleic acid consisting of a nucleotide sequence complementary to the nucleotide sequence of the nucleic acid, micro-RNA or micro-RNA precursor, as an active ingredient.

37. An agent for promoting cell proliferation, comprising the nucleic acid, micro-RNA or micro-RNA precursor described in any one of claims 1 to 4, wherein the SEQ ID NO: is 18, as an active ingredient.

38. An agent for promoting cell proliferation, comprising the micro-RNA precursor described in any one of claims 5 to 7, wherein the SEQ ID NO: is 60, as an active ingredient.

39. An agent for suppressing cell proliferation, comprising a nucleic acid consisting of a nucleotide sequence complementary to the nucleic acid, micro-RNA or micro-RNA precursor described in any one of claims 1 to 4, wherein the SEQ ID NO: is 18, or to the micro-RNA precursor described in any one of claims 5 to 7, wherein the SEQ ID NO: is 60, as an active ingredient.

40. An agent for promoting or suppressing cell proliferation, comprising a double-stranded nucleic acid consisting of the nucleic acid, micro-RNA or micro-RNA precursor described in any one of claims 1 to 4, wherein the SEQ ID NO: is 18, or of the micro-RNA precursor described in any one of claims 5 to 7, wherein the SEQ ID NO: is 60 and a nucleic acid consisting of a nucleotide sequence complementary to the nucleotide sequence of the nucleic acid, micro-RNA or micro-RNA precursor, as an active ingredient.


SEQUENCE LISTING

<110> KYOWA HAKKO KOGYO CO., LTD.

<120> Novel nucleic acids

<130> 1885

<150> JP2006-238459
<151> 2006-09-04

<160> 84

<170> PatentIn Ver. 2.1

<210> 1
<211> 22
<212> RNA
<213> Human

<400> 1
cuuggcaccu agcaagcacu ca                                                22

<210> 2
<211> 18
<212> RNA
<213> Human

<400> 2
uccuggccuc ucuucuag                                                     18

<210> 3
<211> 18
<212> RNA
<213> Human

<400> 3
gcuccaacgg uaggugca                                                     18

<210> 4
<211> 18
<212> RNA
<213> Human

<400> 4
aucccacucc ugacacca                                                     18

<210> 5
<211> 22
<212> RNA
<213> Human

<400> 5
cccacugcuu cacuugacua gc                                                22

<210> 6
<211> 20
<212> RNA
<213> Human

<400> 6
ugguccgaug guaguggguu                                                   20

<210> 7
<211> 22
<212> RNA
<213> Human

<400> 7
gcaguccaug ggcauauaca cu                                                22

<210> 8
<211> 22
<212> RNA

<213> Human

<400> 8
ccucccacac ccaaggcuug ca                                                22

<210> 9
<211> 20
<212> RNA
<213> Human

<400> 9
gaagcgggug cucguauuuu                                                   20

<210> 10
<211> 23
<212> RNA
<213> Human

<400> 10
aaggagcuua caaucuagcu ggg                                               23

<210> 11
<211> 15
<212> RNA
<213> Human

<400> 11
ccccccacaa ccgcg                                                        15

<210> 12
<211> 19
<212> RNA
<213> Human

<400> 12
agucccaucu gggucgcca                                                    19

<210> 13
<211> 18
<212> RNA
<213> Human

<400> 13
ucccggguuu cggcacca                                                     18

<210> 14
<211> 15
<212> RNA
<213> Human

<400> 14
agaagggaag uacau                                                        15

<210> 15
<211> 18
<212> RNA
<213> Human

<400> 15
auccgaguca cggcacca                                                     18

<210> 16
<211> 18
<212> RNA
<213> Human

<400> 16
accccacucc ugguacca                                                     18

<210> 17
<211> 22
<212> RNA
<213> Human

<400> 17
ugugugugug cuuguauaua ua                                                22

<210> 18
<211> 23
<212> RNA
<213> Human

<400> 18
uagugcaaua uugcuuauag ggu                                               23

<210> 19
<211> 20
<212> RNA
<213> Human

<400> 19
uaugucugcu gaccaucacc                                                   20

<210> 20
<211> 23
<212> RNA
<213> Human

<400> 20
aaacauucgc ggugcacuuc uuu                                               23

<210> 21
<211> 17
<212> RNA
<213> Human

<400> 21
cccgccgcgg ccccagg                                                      17

<210> 22
<211> 19
<212> RNA
<213> Human

<400> 22
agucccuucg uggucgcca                                                    19

<210> 23
<211> 16
<212> RNA
<213> Human

<400> 23
uuccucugau gacuuc                                                       16

<210> 24
<211> 25
<212> RNA
<213> Human

<400> 24
agcucagugg aauggcucau gcuuu                                             25

<210> 25
<211> 18
<212> RNA
<213> Human

<400> 25
cgggcguggu gguggggg                                                     18

<210> 26
<211> 16
<212> RNA
<213> Human

<400> 26
cccggcaucu ccacca                                                       16

<210> 27
<211> 21
<212> RNA
<213> Human

<400> 27
cuauacaauc uauugccuuc c                                                21

<210> 28
<211> 21
<212> RNA
<213> Human

<400> 28
cuauacaacc uacugccuuc c                                                21

<210> 29
<211> 22
<212> RNA
<213> Human

<400> 29
gcaguccaug ggcauauaca cu                                               22

<210> 30
<211> 76
<212> RNA
<213> Human

<400> 30
cagaucagug cuuggcaccu agcaagcacu caguaaauau uuguugagug ccugcuaugu 60

gccaggcauu gugcug                                                      76

<210> 31
<211> 57
<212> RNA
<213> Human

<400> 31
uuccuggccu cucuucuaga gauuuugauu cagcaggaug gggguuucc ugaaaau     57

<210> 32
<211> 96
<212> RNA
<213> Human

<400> 32
cugcuggcag acccuguguu cggcccgauc cuggccucuc uucuagguga gccugagagc 60

acaggcacag uggguacggg gcaggugcug agggag                                96

<210> 33
<211> 59
<212> RNA
<213> Human

<400> 33
ggcuccaacg guaggugcag ggcgcuccgc ugcaggggcc cggcgcggcc gggagagcc  59

<210> 34
<211> 61
<212> RNA
<213> Human

<400> 34
uuggucgcuc agaacugggg guuguuagaa gcucuacuuc agaucccacu ccugacacca 60

u                                                                      61

<210> 35
<211> 104
<212> RNA

<213> Human

<400> 35
guguguuagu uauagaagca cuacacuaag gauagaauua ugaacguauc ccacugcuuc 60

acuugacuag ccuaaaaaau aaaaauauau gugaacauua caca 104

<210> 36
<211> 83
<212> RNA
<213> Human

<400> 36
ccuucuccac ucccacugcu ucacuugacu agccuuaaaa acaaaaaaga aaagagagag 60

aaagcaggcc gggugugggg gcu 83

<210> 37
<211> 78
<212> RNA
<213> Human

<400> 37
uccacuccca cugcuucacu ugacuagccu aaaaaauuaa uuaauuaaau aaaacagguu 60

auaggagaag ggagaaga 78

<210> 38
<211> 85
<212> RNA
<213> Human

<400> 38
uuccuucuuu acucccacug cuucacuuga cuagccuuua aaaaagaaag gcuugguuug 60

augaaugggu gagagaaaag gugag 85

<210> 39
<211> 80
<212> RNA
<213> Human

<400> 39
ucuacacgcc cacugcuuca cuugacuagc uuuaaaaaac aauaaaaaua aauugcaugg 60

ccgggugcgg uggcucacgc 80

<210> 40
<211> 66
<212> RNA
<213> Human

<400> 40
cauagcuaau caucaguuac agauuccuug guuccuuccc cacucccacu gcuucacuug 60

acuagc 66

<210> 41
<211> 66
<212> RNA
<213> Human

<400> 41
agcuaguuga ucacaaucag agauuuuuau guuccuucuc caaucccacu gcuucacuug 60

acuagc 66

<210> 42
<211> 84
<212> RNA
<213> Human

<400> 42
auucauuuuu aggcuggucc gaugguagug gguuaucaga aauuaacauc aguguuacua 60

aagucgguau acaacccuac acug 84

<210> 43
<211> 74
<212> RNA
<213> Human

<400> 43
aaguucagag acacccugcu gucaaacaac aguguaacuc cggcuggucc gaugguagug 60

gguuaucaga acuu 74

<210> 44
<211> 52
<212> RNA
<213> Human

<400> 44
gggacugguc cgaugguagu ggguuaucag aacuuauuaa cauucauguc cc 52

<210> 45
<211> 82
<212> RNA
<213> Human

<400> 45
ggcgugaggg uaugugccuu uggacuacau cguggaagcc agcaccaugc aguccauggg 60

cauauacacu ugccucaagg cc 82

<210> 46
<211> 92
<212> RNA
<213> Human

<400> 46
gacuugcuuu cucuccucca ugccuugagu guaggaccgu uggcaucuua auuacccucc 60

cacacccaag gcuugcaaaa aagcgagccu uc 92

<210> 47
<211> 147
<212> RNA
<213> Human

<400> 47
gcagcgaugg ccgagugguu aaggcguugg acuugaaauc caaugggguc uccccgcgca 60

gguucgaacc cugcucgcug cggaagcggg ugcucuuauu uuuucuauuu uuuuaaauaa 120

acaccuucaa augaaaacuu uuacucu 147

<210> 48
<211> 79
<212> RNA
<213> Human

<400> 48
gcccucaagg agcuuacaau cuagcugggg guaaaugacu ugcacaugaa cacaacuaga 60

cugugagcuu cuagagggc 79

<210> 49
<211> 71
<212> RNA
<213> Human

<400> 49
aguugguccg aguuguuggg guuauuguua aguugauuua acauugucuc cccccacaac 60

cgcgcuugac u 71

<210> 50

<211> 74
<212> RNA
<213> Human

<400> 50
ggacagaaua gcaagcgaaa gggguggugc ucacugcuug gcaauagucc caucuggguc 60

gccaaaaugu gucu 74

<210> 51
<211> 60
<212> RNA
<213> Human

<400> 51
ucuauugccu ucccggguuu cggcaccaga auauaagguc agccgagaga aaggaugaga 60

<210> 52
<211> 70
<212> RNA
<213> Human

<400> 52
auaauucaau gugacaucuc uuugaaugac caucaccugc uugcuccuua gaagggaagu 60

acauuuuuau 70

<210> 53
<211> 63
<212> RNA
<213> Human

<400> 53
ggaggacagg ggauugaucu cggauuguga cuccccagau ccgagucacg gcaccaaaau 60

uuc 63

<210> 54
<211> 60
<212> RNA
<213> Human

<400> 54
uggcugcugg gccuggcaau gccccuagga cauaucagag acaccccacu ccugguacca 60

<210> 55
<211> 81
<212> RNA
<213> Human

<400> 55
uucuauuagg uggcagcccu gguacaaaga cuggagucaa aauuaaaccc cacuccuggu 60

accaguuucu auuaguuaga g 81

<210> 56
<211> 71
<212> RNA
<213> Human

<400> 56
caguuccaaa guuguuucca cuuuuugggg uauuuuauca gcaauacccc acuccuggua 60

ccaacuuacu g 71

<210> 57
<211> 51
<212> RNA
<213> Human

<400> 57
uggcacccca cuccugguac caaagucuga auuuguuugc uuggguugcc a 51

<210> 58

<211> 64
<212> RNA
<213> Human

<400> 58
uauaugugug ugugcuugua uauauauaua uauauauaua uauauacaag cacacacaca 60

aaug 64.

<210> 59
<211> 74
<212> RNA
<213> Human

<400> 59
ucuugcauuu gugugugugc uuguauauau auauauauau auauauauau acaagcacac 60

acacauauau agua 74

<210> 60
<211> 73
<212> RNA
<213> Human

<400> 60
cuagaacccu aucaauauug ucucugcugu guaaauaguu cugaguagug caauauugcu 60

uauaggguuu ugg 73

<210> 61
<211> 81
<212> RNA
<213> Human

<400> 61
ggguaagugg aaagauggug ggccgcagaa caugugcuga guucgugcca uaugucugcu 60

gaccaucacc uuuagaagcc c 81

<210> 62
<211> 93
<212> RNA
<213> Human

<400> 62
gacgugcggu acuuaaugag aaguugcccg uguuuuuuuc gcuuuauuug ugacgaaaca 60

uucgcggugc acuucuuuuu caguauccua uuc 93

<210> 63
<211> 68
<212> RNA
<213> Human

<400> 63
gccugggcug cagcgggagc gacgcgccgg ccgccgggug gggguggagc cccgccgcgg 60

ccccaggu 68

<210> 64
<211> 54
<212> RNA
<213> Human

<400> 64
guccugggac aggucgcguu cagcgugggc gucggccccg ccgcggcccc aggc 54

<210> 65
<211> 61
<212> RNA
<213> Human

<400> 65
uuggcgauag gcgauggucu caccgcucag cgacaggcaa aagucccuuc guggucgcca 60

a                                                                    61

<210> 66
<211> 71
<212> RNA
<213> Human

<400> 66
ugggauugag aagggagcag ugaagggacc gggagguuau acuuuccucu gaugacuucc 60

uguuagugcc a                                                         71

<210> 67
<211> 71
<212> RNA
<213> Human

<400> 67
agcucagugg aauggcucau gcuuuuaauc ccagcacuuu gggaggcuga ggcaggagga 60

uuacuugagc u                                                         71

<210> 68
<211> 70
<212> RNA
<213> Human

<400> 68
caggcaacau ggcgaaaacc cgucucuaau gaaaauacaa aaauuagccg ggcguggugg 60

ugggggccug                                                           70

<210> 69
<211> 105
<212> RNA
<213> Human

<400> 69
cauggugaaa cccugucccu acuaaagaua caaaaauuag ccgggcgugg uggugggggc 60

cuauaauccc agcuacucgg gaggcugagg uaggagaauc gcuug                   105

<210> 70
<211> 52
<212> RNA
<213> Human

<400> 70
uuagccgggc gugguggugg gggccuguag ucccagcuac ucgggaggcu ga           52

<210> 71
<211> 52
<212> RNA
<213> Human

<400> 71
uuagccgggc gugguggugg gggccuauaa ucccagcuac ugacgaggcu ga           52

<210> 72
<211> 52
<212> RNA
<213> Human

<400> 72
uuagccgggc gugguggugg gggccuguaa ucccagcuac ucgggaggcu ga           52

<210> 73
<211> 55
<212> RNA
<213> Human

<400> 73
gugucauggg acagugggcc cuggugccac aaugcccggc aucuccacca gauac       55

<210> 74
<211> 19
<212> DNA
<213> Artificial Sequence

<220>
<223>

<400> 74
cctttggact acatcgtgg                                                    19

<210> 75
<211> 20
<212> DNA
<213> Artificial Sequence

<220>
<223>

<400> 75
caagtgtata tgcccatgga                                                   20

<210> 76
<211> 17
<212> DNA
<213> Artificial Sequence

<220>
<223>

<400> 76
ctcgcttcgg cagcaca                                                      17

<210> 77
<211> 20
<212> DNA
<213> Artificial Sequence

<220>
<223>

<400> 77
aacgcttcac gaatttgcgt                                                   20

<210> 78
<211> 24
<212> RNA
<213> Human

<220>
<223>

<400> 78
uggaauaccg ggugcuguag gcuu                                              24

<210> 79
<211> 18
<212> RNA
<213> Human

<220>
<223>

<400> 79
aucccuucgu gguugcca                                                     18

<210> 80
<211> 18
<212> RNA
<213> Human

<220>
<223>

<400> 80
aucccaccac ugccacca                                                18

<210> 81
<211> 67
<212> RNA
<213> Human

<220>
<223>

<400> 81
aggucggguc aguuaguacu uggaugggag acugccugga auaccgggug cuguaggcuu 60

uuggccu                                                            67

<210> 82
<211> 81
<212> RNA
<213> Human

<220>
<223>

<400> 82
uagaacagaa uugcaaguga aaggggucog augguacuca cuacuuggug auuaucccuu 60

cgugguugcc aaaugugucu g                                            81

<210> 83
<211> 124
<212> RNA
<213> Human

<220>
<223>

<400> 83
cgggcggcug cugcucuccg uuuaucccac cacugccacc auuauugcua cuguucagca 60

ggugcugcug guggugaugg ugauagucug gugggggcgg uggggcugcu guugcugcug 120

cuug                                                               124

<210> 84
<211> 20
<212> RNA
<213> Human

<220>
<223>

<400> 84
aucccacucc ugacaccauu                                              20

SEQUENCE LISTING

<110> KYOWA HAKKO KOGYO CO., LTD.

<120> Novel nucleic acids

<130> P1668 EP

<150> JP2006-238459
<151> 2006-09-04

<160> 84

<170> PatentIn Ver.2.1

<210> 1
<211> 22
<212> RNA
<213> Homo sapiens

<400> 1
cuuggcaccu agcaagcacu ca                                                22

<210> 2
<211> 18
<212> RNA
<213> Homo sapiens

<400> 2
uccuggccuc ucuucuag                                                     18

<210> 3
<211> 18
<212> RNA
<213> Homo sapiens

<400> 3
gcuccaacgg uaggugca                                                     18

<210> 4
<211> 18
<212> RNA
<213> Homo sapiens

<400> 4
aucccacucc ugacacca                                                     18

<210> 5
<211> 22
<212> RNA
<213> Homo sapiens

<400> 5
cccacugcuu cacuugacua gc                                                22

<210> 6
<211> 20
<212> RNA
<213> Homo sapiens

<400> 6
ugguccgaug guaguggguu                                                   20

<210> 7

<211> 22
<212> RNA
<213> Homo sapiens

<400> 7
gcaguccaug ggcauauaca cu                                                22

<210> 8
<211> 22
<212> RNA
<213> Homo sapiens

<400> 8
ccucccacac ccaaggcuug ca                                                22

<210> 9
<211> 20
<212> RNA
<213> Homo sapiens

<400> 9
gaagcgggug cucguauuuu                                                   20

<210> 10
<211> 23
<212> RNA
<213> Homo sapiens

<400> 10
aaggagcuua caaucuagcu ggg                                               23

<210> 11
<211> 15
<212> RNA
<213> Homo sapiens

<400> 11
ccccccacaa ccgcg                                                        15

<210> 12
<211> 19
<212> RNA
<213> Homo sapiens

<400> 12
agucccaucu gggucgcca                                                    19

<210> 13
<211> 18
<212> RNA
<213> Homo sapiens

<400> 13
ucccggguuu cggcacca                                                     18

<210> 14
<211> 15
<212> RNA
<213> Homo sapiens

<400> 14
agaagggaag uacau                                                        15

<210> 15

<211> 18
<212> RNA
<213> Homo sapiens

<400> 15
auccgaguca cggcacca                                                    18

<210> 16
<211> 18
<212> RNA
<213> Homo sapiens

<400> 16
accccacucc ugguacca                                                    18

<210> 17
<211> 22
<212> RNA
<213> Homo sapiens

<400> 17
ugugugugug cuuguauaua ua                                               22

<210> 18
<211> 23
<212> RNA
<213> Homo sapiens

<400> 18
uagugcaaua uugcuuauag ggu                                              23

<210> 19
<211> 20
<212> RNA
<213> Homo sapiens

<400> 19
uaugucugcu gaccaucacc                                                  20

<210> 20
<211> 23
<212> RNA
<213> Homo sapiens

<400> 20
aaacauucgc ggugcacuuc uuu                                              23

<210> 21
<211> 17
<212> RNA
<213> Homo sapiens

<400> 21
cccgccgcgg ccccagg                                                     17

<210> 22
<211> 19
<212> RNA
<213> Homo sapiens

<400> 22
agucccuucg uggucgcca                                                   19

<210> 23

<211> 16
<212> RNA
<213> Homo sapiens

<400> 23
uuccucugau gacuuc                                                    16

<210> 24
<211> 25
<212> RNA
<213> Homo sapiens

<400> 24
agcucagugg aauggcucau gcuuu                                          25

<210> 25
<211> 18
<212> RNA
<213> Homo sapiens

<400> 25
cgggcguggu gguggggg                                                  18

<210> 26
<211> 16
<212> RNA
<213> Homo sapiens

<400> 26
cccggcaucu ccacca                                                    16

<210> 27
<211> 21
<212> RNA
<213> Homo sapiens

<400> 27
cuauacaauc uauugccuuc c                                              21

<210> 28
<211> 21
<212> RNA
<213> Homo sapiens

<400> 28
cuauacaacc uacugccuuc c                                              21

<210> 29
<211> 22
<212> RNA
<213> Homo sapiens

<400> 29
gcaguccaug ggcauauaca cu                                             22

<210> 30
<211> 76
<212> RNA
<213> Homo sapiens

<400> 30
cagaucagug cuuggcaccu agcaagcacu caguaaauau uuguugagug ccugcuaugu 60

gccaggcauu gugcug                                                    76

<210> 31
<211> 57
<212> RNA
<213> Homo sapiens

<400> 31
uuccuggccu cucuucuaga gauuuugauu cagcaggaug ggggguuucc ugaaaau    57

<210> 32
<211> 96
<212> RNA
<213> Homo sapiens

<400> 32
cugcuggcag acccuguguu cggcccgauc cuggccucuc uucuagguga gccugagagc 60

acaggcacag uggguacggg gcaggugcug agggag                           96

<210> 33
<211> 59
<212> RNA
<213> Homo sapiens

<400> 33
ggcuccaacg guaggugcag ggcgcuccgc ugcaggggcc cggcgcggcc gggagagcc  59

<210> 34
<211> 61
<212> RNA
<213> Homo sapiens

<400> 34
uuggucgcuc agaacugggg guuguuagaa gcucuacuuc agaucccacu ccugacacca 60

u                                                                 61

<210> 35
<211> 104
<212> RNA
<213> Homo sapiens

<400> 35
guguguuagu uauagaagca cuacacuaag gauagaauua ugaacguauc ccacugcuuc 60

acuugacuag ccuaaaaaau aaaaauauau gugaacauua caca                  104

<210> 36
<211> 83
<212> RNA
<213> Homo sapiens

<400> 36
ccuucuccac ucccacugcu ucacuugacu agccuuaaaa acaaaaaaga aaagagagag 60

aaagcaggcc gggugugggg gcu                                         83

<210> 37
<211> 78
<212> RNA
<213> Homo sapiens

<400> 37
uccacuccca cugcuucacu ugacuagccu aaaaaauuaa uuaauuaaau aaaacagguu 60

auaggagaag ggagaaga 78

<210> 38
<211> 85
<212> RNA
<213> Homo sapiens

<400> 38
uuccuucuuu acucccacug cuucacuuga cuagccuuua aaaaagaaag gcuugguuug 60

augaaugggu gagagaaaag gugag 85

<210> 39
<211> 80
<212> RNA
<213> Homo sapiens

<400> 39
ucuacacgcc cacugcuuca cuugacuagc uuuaaaaaac aauaaaaaua aauugcaugg 60

ccgggugcgg uggcucacgc 80

<210> 40
<211> 66
<212> RNA
<213> Homo sapiens

<400> 40
cauagcuaau caucaguuac agauuccuug guuccuuccc cacucccacu gcuucacuug 60

acuagc 66

<210> 41
<211> 66
<212> RNA
<213> Homo sapiens

<400> 41
agcuaguuga ucacaaucag agauuuuuau guuccuucuc caaucccacu gcuucacuug 60

acuagc 66

<210> 42
<211> 84
<212> RNA
<213> Homo sapiens

<400> 42
auucauuuuu aggcuggucc gaugguagug gguuaucaga aauuaacauc aguguuacua 60

aagucgguau acaacccuac acug 84

<210> 43
<211> 74
<212> RNA
<213> Homo sapiens

<400> 43
aaguucagag acacccugcu gucaaacaac aguguaacuc cggcuggucc gaugguagug 60

gguuaucaga acuu 74

<210> 44

<211> 52
<212> RNA
<213> Homo sapiens

<400> 44
gggacugguc cgaugguagu ggguuaucag aacuuauuaa cauucauguc cc          52

<210> 45
<211> 82
<212> RNA
<213> Homo sapiens

<400> 45
ggcgugaggg uaugugccuu uggacuacau cguggaagcc agcaccaugc aguccauggg 60

cauauacacu ugccucaagg cc                                          82

<210> 46
<211> 92
<212> RNA
<213> Homo sapiens

<400> 46
gacuugcuuu cucuccucca ugccuugagu guaggaccgu uggcaucuua auuacccucc 60

cacacccaag gcuugcaaaa aagcgagccu uc                               92

<210> 47
<211> 147
<212> RNA
<213> Homo sapiens

<400> 47
gcagcgaugg ccgagugguu aaggcguugg acuugaaauc caaugggguc uccccgcgca 60

gguucgaacc cugcucgcug cggaagcggg ugcucuuauu uuuucuauuu uuuuaaauaa 120

acaccuucaa augaaaacuu uuacucu                                     147

<210> 48
<211> 79
<212> RNA
<213> Homo sapiens

<400> 48
gcccucaagg agcuuacaau cuagcugggg guaaaugacu ugcacaugaa cacaacuaga 60

cugugagcuu cuagagggc                                              79

<210> 49
<211> 71
<212> RNA
<213> Homo sapiens

<400> 49
aguugguccg aguguugugg guuauuguua aguugauuua acauugucuc cccccacaac 60

cgcgcuugac u                                                      71

<210> 50
<211> 74
<212> RNA
<213> Homo sapiens

<400> 50
ggacagaaua gcaagcgaaa gggguggugc ucacugcuug gcaauagucc caucuggguc 60

gccaaaaugu gucu 74

<210> 51
<211> 60
<212> RNA
<213> Homo sapiens

<400> 51
ucuauugccu ucccggguuu cggcaccaga auauaagguc agccgagaga aaggaugaga 60

<210> 52
<211> 70
<212> RNA
<213> Homo sapiens

<400> 52
auaauucaau gugacaucuc uuugaaugac caucaccugc uugcuccuua gaagggaagu 60

acauuuuuau 70

<210> 53
<211> 63
<212> RNA
<213> Homo sapiens

<400> 53
ggaggacagg ggauugaucu cggauuguga cuccccagau ccgagucacg gcaccaaaau 60

uuc 63

<210> 54
<211> 60
<212> RNA
<213> Homo sapiens

<400> 54
uggcugcugg gccuggcaau gccccuagga cauaucagag acaccccacu ccugguacca 60

<210> 55
<211> 81
<212> RNA
<213> Homo sapiens

<400> 55
uucuauuagg uggcagcccu gguacaaaga cuggagucaa aauuaaaccc cacuccuggu 60

accaguuucu auuaguuaga g 81

<210> 56
<211> 71
<212> RNA
<213> Homo sapiens

<400> 56
caguuccaaa guuguuucca cuuuuugggg uauuuuauca gcaauacccc acuccuggua 60

ccaacuuacu g 71

<210> 57
<211> 51
<212> RNA

<213> Homo sapiens

<400> 57
uggcacccca cuccugguac caaagucuga auuuguuugc uuggguugcc a          51

<210> 58
<211> 64
<212> RNA
<213> Homo sapiens

<400> 58
uauaugugug ugugcuugua uauauauaua uauauauaua uauauacaag cacacacaca 60

aaug                                                              64

<210> 59
<211> 74
<212> RNA
<213> Homo sapiens

<400> 59
ucuugcauuu gugugugugc uuguauauau auauauauau auauauauau acaagcacac 60

acacauauau agua                                                   74

<210> 60
<211> 73
<212> RNA
<213> Homo sapiens

<400> 60
cuagaacccu aucaauauug ucucugcugu guaaauaguu cugaguagug caauauugcu 60

uauaggguuu ugg                                                    73

<210> 61
<211> 81
<212> RNA
<213> Homo sapiens

<400> 61
ggguaagugg aaagauggug ggccgcagaa caugugcuga guucgugcca uaugucugcu 60

gaccaucacc uuuagaagcc c                                           81

<210> 62
<211> 93
<212> RNA
<213> Homo sapiens

<400> 62
gacgugcggu acuuaaugag aaguugcccg uguuuuuuuc gcuuuauuug ugacgaaaca 60

uucgcggugc acuucuuuuu caguauccua uuc                              93

<210> 63
<211> 68
<212> RNA
<213> Homo sapiens

<400> 63
gccugggcug cagcgggagc gacgcgccgg ccgccgggug gggguggagc cccgccgcgg 60

ccccaggu                                                          68

<210> 64
<211> 54
<212> RNA
<213> Homo sapiens

<400> 64
guccugggac aggucgcguu cagcgugggc gucggccccg ccgcggcccc aggc       54

<210> 65
<211> 61
<212> RNA
<213> Homo sapiens

<400> 65
uuggcgauag gcgauggucu caccgcucag cgacaggcaa aagucccuuc guggucgcca 60

a                                                                 61

<210> 66
<211> 71
<212> RNA
<213> Homo sapiens

<400> 66
ugggauugag aagggagcag ugaagggacc gggagguuau acuuuccucu gaugacuucc 60

uguuagugcc a                                                      71

<210> 67
<211> 71
<212> RNA
<213> Homo sapiens

<400> 67
agcucagugg aauggcucau gcuuuuaauc ccagcacuuu gggaggcuga ggcaggagga 60

uuacuugagc u                                                      71

<210> 68
<211> 70
<212> RNA
<213> Homo sapiens

<400> 68
   caggcaacau ggcgaaaacc cgucucuaau gaaaauacaa aaauuagccg ggcguggugg 60
   ugggggccug                                                        70

<210> 69
<211> 105
<212> RNA
<213> Homo sapiens

<400> 69
   cauggugaaa cccugucccu acuaaagaua caaaaauuag ccgggcgugg uggugggggc 60
   cuauaauccc agcuacucgg gaggcugagg uaggagaauc gcuug                 105

<210> 70
<211> 52
<212> RNA
<213> Homo sapiens

<400> 70
uuagccgggc gugguggugg gggccuguag ucccagcuac ucgggaggcu ga          52

<210> 71
<211> 52
<212> RNA
<213> Homo sapiens

<400> 71
uuagccgggc gugguggugg gggccuauaa ucccagcuac ugacgaggcu ga          52

<210> 72
<211> 52
<212> RNA
<213> Homo sapiens

<400> 72
uuagccgggc gugguggugg gggccuguaa ucccagcuac ucgggaggcu ga          52

<210> 73
<211> 55
<212> RNA
<213> Homo sapiens

<400> 73
gugucauggg acagugggcc cuggugccac aaugcccggc aucuccacca gauac        55

<210> 74
<211> 19
<212> DNA
<213> Artificial Sequence

<220>
<223>

<400> 74
cctttggact acatcgtgg                                                19

<210> 75
<211> 20
<212> DNA
<213> Artificial Sequence

<220>
<223>

<400> 75
caagtgtata tgcccatgga                                               20

<210> 76
<211> 17
<212> DNA
<213> Artificial Sequence

<220>
<223>

<400> 76
ctcgcttcgg cagcaca                                                  17

<210> 77
<211> 20
<212> DNA
<213> Artificial Sequence

<220>
<223>

<400> 77
aacgcttcac gaatttgcgt                                                    20

<210> 78
<211> 24
<212> RNA
<213> Homo sapiens

<220>
<223>

<400> 78
uggaauaccg ggugcuguag gcuu                                               24

<210> 79
<211> 18
<212> RNA
<213> Homo sapiens

<220>
<223>

<400> 79
aucccuucgu gguugcca                                                      18

<210> 80
<211> 18
<212> RNA
<213> Homo sapiens

<220>
<223>

<400> 80
aucccaccac ugccacca                                                      18

<210> 81
<211> 67
<212> RNA
<213> Homo sapiens

<220>
<223>

<400> 81
aggucggguc aguuaguacu uggaugggag acugccugga auaccgggug cuguaggcuu 60

uuggccu                                                                  67

<210> 82
<211> 81
<212> RNA
<213> Homo sapiens

<220>
<223>

<400> 82
uagaacagaa uugcaaguga aagggguccg augguacuca cuacuuggug auuaucccuu 60

cgugguugcc aaauguguc u g                                                81

<210> 83
<211> 124
<212> RNA
<213> Homo sapiens

<220>
<223>

<400> 83
cgggcggcug cugcucuccg uuuaucccac cacugccacc auuauugcua cuguucagca 60

ggugcugcug guggugaugg ugauagucug gugggggcgg uggggcugcu guugcugcug 120

cuug                                                                    124

<210> 84
<211> 20
<212> RNA
<213> Homo sapiens

<220>
<223>

<400> 84
aucccacucc ugacaccauu                                                   20